# EUROPÄISCHE PATENTANMELDUNG

(11) **EP 1 813 310 A2**
(43) Veröffentlichungstag der Anmeldung: **01.08.2007**
(21) Anmeldenummer: 07000974.1
(22) Anmeldetag: 18.01.2007
(51) Int. Cl.: A61Q 15/00, A61K 8/06, A61K 8/33, A61K 8/34, A61K 8/92, A61K 8/37, A61K 8/26, A61K 8/28, A61K 8/39

(54) **Kosmetischer Stift aus Basis einer Öl-in-Wasser-Dispersion/Emulsion**

(30) Priorität: 25.01.2006 DE 102006003789; 01.02.2006 DE 102006004955
(71) Anmelder: Henkel Kommanditgesellschaft auf Aktien, 40589 Düsseldorf (DE)
(72) Erfinder: Banowski, Bernhard, 40597 Düsseldorf (DE); Wadle, Armin, 40699 Erkrath (DE); Claas, Marcus, 40724 Hilden (DE)

(57) **Zusammenfassung**

Die Anmeldung betrifft kosmetische Stifte auf Basis einer Öl-in-Wasser-Dispersion/ Emulsion zur Applikation auf die Haut.

## Beschreibung

Die Erfindung betrifft kosmetische Stifte auf Basis einer Öl-in-Wasser-Dispersion/Emulsion zur Applikation kosmetischer Wirkstoffe auf die Haut.

Handelsübliche kosmetische Zusammensetzungen werden meistens als Cremes oder Lotionen konfektioniert, die einem Tiegel, einer Tube, einer Flasche oder einem Pumpspender entnommen werden; daneben gibt es Roll-on-Präparate, Sprays und (Kompakt-) Puder im Markt. Bei den Verbrauchern erfreuen sich Stiftpräparate zur Anwendung sowohl dekorativer als auch pflegender Kosmetik hoher Beliebtheit. Sie sind handlich, transportstabil und bequem aufzutragen. Zunehmend besteht bei den Konsumenten das Bedürfnis nach Zusammensetzungen, die neben der Basisfunktion, also z. B. dem Abdecken von Hautunreinheiten oder dem Mattieren fettglänzender Haut, noch weitere Pflege- und Behandlungseffekte, z. B. durch antibakterielle oder hautberuhigende Wirkstoffe, und gleichzeitig ein angenehmes, frisches Hautgefühl vermitteln.
Viele stiftförmige Präparate werden als wasserfreie Suspensionsstifte formuliert. Derartige Präparate hinterlassen beim Anwender ein angenehm trockenes Hautgefühl nach dem Auftragen. Eine effektive Freisetzung der wasserlöslichen Wirkstoffe aus solchen Präparaten ist jedoch limitiert (dies wurde z. B. für Antitranspirant-Stifte in der Literatur gezeigt, vgl. Chemistry and Technology of the Cosmetics and Toiletries Industry, Hrsg.: D. F. Williams und W. H. Schmitt, London: Blackie, 1996, 2. Auflage, S. 326), und es wird meist nicht das von vielen Verbrauchern geschätzte Frischegefühl erzielt. Wasserfreie Stiftpräparate, insbesondere solche auf Basis von flüchtigen Siliconölen, haben den Nachteil, dass die dispergierten Wirkstoffe leicht zu sichtbaren Produktrückständen auf Haut und Kleidung führen. Außerdem sind solche Zubereitungen relativ kostspielig, da die Ölkomponenten als Wirkstoffträger teurer sind als Wasser. Unter der Druckbelastung bei der Applikation kommt es häufig zu einem Ausölen, was die kosmetische Akzeptanz dieser Präparate beim Anwender herabsetzt.

Gegenüber wasserfreien Stiften, wie sie zum Beispiel aus US 5733534 und WO 00/67713 A1 bekannt sind, weisen Emulsionsstifte, wie sie zum Beispiel in WO 98/17238 A1, US 4814165, DE 2 335 549, US 4,725,431, US 5466457 und US 4948578 offenbart sind, mehrere Vorteile auf. Der Ersatz der Wachs- und Ölzusätze durch Wasser macht die Emulsionsstifte kostengünstiger in der Herstellung. Die emulgierten Wachse vermitteln ein weiches, leichtes Hautgefühl, und schließlich können wasserlösliche kosmetische Wirkstoffe leichter an die Haut abgegeben werden, da sie in der wässrigen Phase der Emulsion bereits in gelöster Form vorliegen.
US 20020051758 A1 offenbart wasserhaltige Antitranspirant-Stifte ohne W/O-Emulgator und hochschmelzendes Wachs, die ein siliconiertes Polyamid als Konsistenzgeber bzw. Structurant enthalten. Gemäß Patentanspruch 1 der WO 02/017870 A2 bildet die wässrige Phase die interne, also dispergierte Phase, so dass es sich bei den offenbarten Gelen um Wasser-in-Öl-Emulsionen handelt.
US 20020072506 A1 offenbart in einigen Ausführungsbeispielen wasserhaltige Antitranspirant-Stifte auf Basis einer Wasser-in-Öl-Emulsion, die acylierte Cellobiose als Konsistenzgeber bzw. Structurant sowie einen hohen Anteil an erfindungsgemäß ungünstigen Silicon- und Kohlenwasserstoffölen, weiterhin weder Öl-in-Wasser-Emulgatoren noch ein hochschmelzendes Wachs enthalten.
Die Emulsionsstifte des zitierten Standes der Technik sind auf Basis einer Wasser-in-Öl- Dispersion/Emulsion formuliert, die wasserlöslichen Wirkstoffe liegen also in der inneren, dispergierten Phase vor und müssen nach der Applikation erst durch die äußere, lipophile Schicht wandern, um ihren Wirkungsort auf der Haut zu erreichen. Damit weisen die bekannten Wasser-in-Öl-Emulsionsstifte hinsichtlich der Wirkstoffverfügbarkeit ähnliche Nachteile auf wie wasserfreie Suspensionsstifte.
US 6428776 offenbart wasser- und ölhaltige, wachsfreie Antitranspirant-Stifte auf Basis einer Öl-in-Wasser-Emulsion. Derartige Stifte weisen unzureichende kosmetische Eigenschaften auf, hinterlassen unangenehme klebrige und sichtbare Rückstände und zeigen eine für den längeren Gebrauch nicht ausreichende Stabilität. Ein Beispiel mit Glycerinmonostearat als W/O-Emulgator und Octyldodecanol als Ölkomponente weist eine mittelfeste Konsistenz und ein fettiges Hautgefühl auf und beginnt bereits bei 50 °C zu erweichen.
In WO 99/59537 A1 sind wasserhaltige kosmetische Stifte offenbart, die Wachskomponenten mit einem Schmelzpunkt > 50°C, nichtionische Wasser-in-ÖI-Emulgatoren, einen nichtionischen ÖI-in-Wasser-Emulgator mit einem HLB-Wert von mehr als 7 und ein Polyol enthalten. Einige der Stifte enthalten bei 25°C flüssige Ölkomponenten, die aber im Unterschied zu den Stiften der vorliegenden Anmeldung nicht zu Beginn des Emulsionsprozesses eingearbeitet werden können, sondern der eigentlichen Stiftmasse als voremulgiertes Konzentrat, beispielsweise als Mikroemulsion oder PIT-Emulsion, während der Abkühlphase der Stiftmasse bei einer Temperatur von ca. 55°C unter Rühren zugesetzt werden. Ein derartiges Herstellverfahren ist notwendig, um die Stabilität des Systems, einer Dispersion von Lipid- und Wachskristallen, nicht zu gefährden oder gar zu zerstören. Derartige Stifte weisen ebenfalls unzureichende kosmetische Eigenschaften auf, können unangenehme klebrige und sichtbare Rückstände hinterlassen und zeigen eine für den längeren Gebrauch nicht ausreichende Stabilität.
US 20030103921 A1 offenbart strukturierte Antitranspirant-Zusammensetzungen in Form einer Mikroemulsion, die je nach Art und Menge der Tenside eine Öl-in-Wasser-Mikroemulsion oder eine Wasser-in-Öl-Mikroemulsion oder eine bikontinuierliche Phase darstellt, wobei aber insgesamt die bikontinuierliche Phase vorherrscht. Die (transparenten) Mikroemulsionen werden durch ein öllösliches oder öldispergierbares "Structurant" verdickt. Das öllösliche oder öldispergierbare "Structurant" ist ausgewählt aus Estern und Amiden der 12-Hydroxystearinsäure, Estern und Amiden von N-Acylaminosäuren, Estern und Amidenvon Di- und Tricarbonsäuren, Sterolen, Sterolestern wie Oryzanol, Cellobiosefettsäureestern, Zuckerestern wie acylierter Maltose und nicht-vernetzten öllöslichen oder öldispergierbaren polymeren Ölphasen-Verdickungsmitteln wie z.B. das Handelsprodukt Kraton G. Weiterhin sind nichtionische Emulgatoren mit einem HLB-Wert von 2 - 15, bevorzugt mit einem HLB-Wert unter 12, enthalten. Polyole sind lediglich als optional offenbart. An keiner Stelle ist eine mögliche Bedeutsamkeit der Abstimmung der Löslichkeitsparameter von W/O-Emulgatoren und Ölkomponenten aufeinander offenbart. Der strukturelle Unterschied zwischen diesen Zusammensetzungen und den Öl-in-Wasser-Dispersions-/Emulsionsstiften der vorliegenden Erfindung, die keine Mikroemulsionen darstellen, wird besonders deutlich durch den hohen Anteil von 19-66 Gew.-% der Gesamtzusammensetzung an erfindungsgemäß ungünstigen Silicon- und (paraffinischen) Kohlenwasserstoffölen, den alle Ausführungsbeispiele offenbaren.
In den Offenlegungsschriften DE 199 62 878 A1 und DE 199 62 881 A1 sind Deodorant- oder Antitranspirant-Cremes auf Basis einer Öl-in-Wasser-Emulsion offenbart, die bei 21 °C eine Viskosität von wenigstens 50000 mPa·s, bevorzugt im Bereich von 200000 - 1500000 mPa·s, aufweisen, das heißt, sie liegen in viskoser bis hochviskoser pastöser Form vor. Diese Cremes enthalten Wachskomponenten mit einem Schmelzpunkt > 50°C, nichtionische Wasser-in-ÖI-Emulgatoren, nichtionische ÖI-in-Wasser-Emulgator mit einem HLB-Wert von mehr als 7 und ein Polyol. Als weiche Cremes lassen sie sich entweder nur mit den Fingern applizieren, was von vielen Verbrauchern als unpraktisch abgelehnt wird, oder die Cremes müssen in spezielle Applikatoren abgefüllt werden, die deutlich kostspieliger sind als die Stifthülsen für die erfindungsgemäßen kosmetischen Stifte. Würde man die in DE 199 62 878 A1 und DE 199 62 881 A1 offenbarten Zusammensetzungen nach dem Erhitzen und Mischen statisch, das heißt ohne Rühren, abkühlen lassen, so würde man stiftähnliche Zusammensetzungen mit insgesamt ungünstigen Anwendungseigenschaften wie schlechter Haptik und/oder mangelnder Stabilität, zum Beispiel durch Phasenseparation oder Bildung von Kondenswasser, erhalten, da die Emulgatoren und die Öle nicht wie in der vorliegenden Erfindung in Bezug auf ihre Löslichkeitsparameter aufeinander abgestimmt sind.
US 20060029624 A1 offenbart Deodorant- oder Antitranspirant-Stifte in Form einer Öl-in-Wasser-Dispersion, die mindestens eine Lipid- oder Wachskomponente mit einem Schmelzpunkt > 50°C, mindestens einen nichtionischen Öl-in-Wasser-Emulgator mit einem HLB-Wert von mehr als 7 innerhalb eines Öl-in-Wasser-Emulgatorsystems mit einem mittleren HLB-Wert im Bereich von 10 - 19, mindestens einen nichtionischen Wasser-in-Öl-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder, mindestens ein bei 20 ° C flüssiges Öl, das keine Duftstoffkomponente und kein etherisches ÖI darstellt, wobei der (mittlere) Löslichkeitsparameter der Gesamtheit der enthaltenen Öle in Gegenwart von linearen gesättigten Fettalkoholen als Wasser-in-ÖI-Emulgator oder als Teil eines Wasser-in-ÖI-Emulgator-Gemisches um maximal -0,7 (cal/cm³)^{0,5} bzw. maximal +0,7 (cal/cm³)^{0,5} und in Gegenwart von Wasser-in-ÖI-Emulgatoren, die von linearen gesättigten Fettalkoholen verschieden sind, in Abwesenheit von linearen gesättigten Fettalkoholen als Wasser-in-Öl-Emulgator um maximal -0,4 (cal/cm³)^{0,5} bzw. maximal +0,7 (cal/cm³)^{0,5} vom (mittleren) Löslichkeitsparameter des Wasser-in-ÖI-Emulgators/der Wasser-in-ÖI-Emulgatoren abweicht, mindestens ein wasserlösliches mehrwertiges C₂ - C₉-Alkanol mit 2 - 6 Hydroxylgruppen und/oder mindestens ein wasserlösliches Polyethylenglycol mit 3-20 Ethylenoxid-Einheiten, 5 bis weniger als 50 Gew.-% Wasser, bezogen auf die Gesamtzusammensetzung und mindestens einen Deodorant- oder Antitranspirant-Wirkstoff enthalten, wobei der Stift einen Penetrationskraftwert im Bereich von 200 - 600 Gramm-Kraft (g-force) bei einer Penetrationstiefe von 5,000 mm und einen elektrischen Widerstand von maximal 300 kΩ (Kiloohm) aufweist. Es wurde jedoch festgestellt, dass das Applikationsverhalten der in US 20060029624 A1 offenbarten Stifte, insbesondere hinsichtlich der Gleitfähigkeit auf der Haut, verbesserungswürdig war. Außerdem wurde festgestellt, dass die in US 20060029624 A1 offenbarten Stifte hinsichtlich ihrer Haptik und Klebrigkeit verbessert werden sollten. Es zeigte sich, dass der Bereich der geeigneten Ölkomponenten insbesondere bei solchen Stiften, die keinen linearen Fettalkohol mit mindestens 8 Kohlenstoffatomen als W/O-Emulgator enthielten, zu eng gefasst war und einige geringer klebrige und besser spreitende Ölkomponenten bisher aus Stabilitätsgründen nicht berücksichtigt worden waren.

Es bestand daher die Aufgabe, eine kosmetische Stiftzusammensetzung zu entwickeln, die sich als effektiver Träger für kosmetische Wirkstoffe eignet und eine schnelle Freisetzung des Wirkstoffes auf der Haut ermöglicht.
Eine weitere Aufgabe bestand darin, eine kosmetische Stiftzusammensetzung mit hervorragenden kosmetischen Pflegeeigenschaften zu entwickeln.
Eine weitere Aufgabe bestand darin, eine kosmetische Stiftzusammensetzung zu entwickeln, die einerseits eine hohe Stabilität, das heißt Festigkeit, andererseits aber ein angenehmes Abgabeverhalten aufweist, also nicht zu fest ist, sondern sich leicht über die Haut streichen lässt und dabei eine ausreichende Produktmenge abgibt.
Eine weitere Aufgabe bestand darin, eine kosmetische Stiftzusammensetzung zu entwickeln, die beim Auftragen auf die Haut möglichst wenig klebrige oder sichtbare Rückstände hinterlässt.
Eine weitere Aufgabe bestand darin, eine kosmetische Stiftzusammensetzung zu entwickeln, die möglichst wenig sichtbare Rückstände auf der Kleidung, die mit der behandelten Haut in Kontakt kommt, hinterlässt.
Eine weitere Aufgabe bestand darin, eine kosmetische Stiftzusammensetzung zu entwickeln, die sich leicht von der Haut abwaschen lässt.

Eine weitere Aufgabe bestand darin, eine kosmetische Stiftzusammensetzung mit einem wirtschaftlich und anwendungstechnisch günstigen Kosten-Leistungs-Verhältnis zu entwickeln.
Eine weitere Aufgabe bestand darin, eine Stiftzusammensetzung, insbesondere eine Deodorant- oder Antitranspirant-Zusammensetzung in Stiftform, zu entwickeln, die eine großtechnische Fertigung stabiler Stifte mit geeigneter Konsistenz erlaubt.

Überraschend und für den Fachmann nicht vorhersehbar wurden diese Aufgaben gelöst durch den Gegenstand der vorliegenden Anmeldung, nämlich eine kosmetische Stiftzusammensetzung in Form einer Öl-in-Wasser-Dispersion/Emulsion, enthaltend
a) mindestens eine Lipid- oder Wachskomponente mit einem Schmelzpunkt > 50°C, die nicht den Komponenten b) oder c) zuzurechnen ist,
b) mindestens einen nichtionischen ÖI-in-Wasser-Emulgator mit einem HLB-Wert von mehr als 7 innerhalb eines Öl-in-Wasser-Emulgatorgemisches mit einem mittleren HLB-Wert im Bereich von 10 - 19,
c) mindestens einen nichtionischen Wasser-in-Öl-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder,
d) mindestens ein unter Normalbedingungen flüssiges Öl, das keine Duftstoffkomponente und kein etherisches Öl darstellt, wobei der (mittlere) Löslichkeitsparameter der Gesamtheit der enthaltenen Öle d) in Gegenwart von linearen gesättigten Fettalkoholen mit mindestens 8 Kohlenstoffatomen um maximal -0,7 (cal/cm³)^{0,5} bzw. maximal +0,7 (cal/cm³)^{0,5} und in Gegenwart von Wasser-in-Öl-Emulgatoren, die von linearen gesättigten Fettalkoholen mit mindestens 8 Kohlenstoffatomen verschieden sind, in Abwesenheit von linearen gesättigten Fettalkoholen mit mindestens 8 Kohlenstoffatomen um maximal -0,4 (cal/cm³)^{0,5} bzw. maximal +0,7 (cal/cm³)^{0,5} vom (mittleren) Löslichkeitsparameter des Wasser-in-Öl-Emulgators/der Wasser-in-Öl-Emulgatoren c) abweicht,
e) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten,
f) 5 bis weniger als 50 Gew.-% Wasser, bezogen auf die Gesamtzusammensetzung,
g) mindestens einen kosmetischen Wirkstoff, ausgewählt aus
   - Monomeren, Oligomeren und Polymeren von Aminosäuren, N-C₂-C₂₄-Acylaminosäuren, den Estern und/oder den physiologisch verträglichen Metallsalzen dieser Substanzen,
   - DNA- oder RNA-Oligonucleotiden,
   - feuchtigkeitsspendenden Wirkstoffen,
   - Vitaminen, Provitaminen und Vitaminvorstufen der Gruppen A, B, C, E und H und den Estern der vorgenannten Substanzen,
   - α-Hydroxycarbonsäuren, α-Ketocarbonsäuren, β-Hydroxycarbonsäuren und deren Ester-, Lacton- oder Salzform,
   - Flavonoiden und Flavonoid-reichen Pflanzenextrakten,
   - Isoflavonoiden und Isoflavonoid-reichen Pflanzenextrakten,
   - Polyphenolen und Polyphenol-reichen Pflanzenextrakten,
   - Ubichinon und Ubichinol sowie deren Derivaten,
   - Silymarin,
   - Purin und Purin-Derivaten, insbesondere den natürlich vorkommenden Xanthin-Derivaten, ausgewählt aus Coffein, Theophyllin, Theobromin und Aminophyllin,
   - Aporphin-Alkaloiden,
   - Ectoin,
   - anorganischen und organischen UV-Filtersubstanzen,
   - selbstbräunenden Wirkstoffen,
   - hautaufhellenden Wirkstoffen,
   - hautberuhigenden Wirkstoffen,
   - sebumregulierenden Wirkstoffen,
   - hyperämisierenden Wirkstoffen,
   - antimikrobiellen Wirkstoffen,
   - präbiotischen Wirkstoffen,
   - färbenden, farbgebenden, mattierenden oder glanzgebenden Pigmenten,
h) einen Gesamtgehalt an nichtionischen und ionischen Emulgatoren und/oder Tensiden mit einem HLB-Wert über 8 von maximal 20 Gew.-%, bezogen auf die gesamte Stiftzusammensetzung.

Alle Angaben über die Aggregatzustände der verwendeten Ausgangsstoffe (fest, flüssig...) in dieser Anmeldung beziehen sich auf Normalbedingungen. "Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die Lipid- oder Wachskomponente mit einem Schmelzpunkt > 50°C formt mit dem/den ÖI/en und ggf. weiteren höherschmelzenden Lipid- oder Wachskomponenten eine Gelmatrix, die größere Mengen an Wasser und Polyol aufnehmen kann. Diese Strukturen, die durch gewisse Mengen an Wasser-in-Öl-Emulgatoren und Öl-in-Wasser-Emulgatoren stabilisiert sind, hinterlassen aufgrund ihres Wassergehaltes bei der Anwendung einen frischen, kühlenden Eindruck. Dabei werden die Emulgatoren so aufeinander abgestimmt, dass die erfindungsgemäßen Stiftzusammensetzungen in Form einer Öl-in-Wasser-Dispersion/Emulsion vorliegen. Die erfindungsgemäßen Stiftzusammensetzungen liegen nicht als Mikroemulsion vor. Zur Herstellung der erfindungsgemäßen Stiftzusammensetzungen müssen die Wasserphase und die Ölphase auf eine Temperatur von mindestens 70°C erhitzt und heiß, also bei mindestens 70°C, miteinander verrührt oder homogenisiert werden, um die erfindungsgemäße Emulsionsstruktur zu erzielen. Ein Herstellverfahren, wie es beispielsweise in US 4,205,062 offenbart ist (Kneten von Fett- und Wasserphase bei 65°C) reicht nicht aus, um eine Stiftzusammensetzung, insbesondere eine homogene Stiftzusammensetzung, auf Basis einer Öl-in-Wasser-Dispersion/Emulsion zu erhalten. Ohne an diese Theorie gebunden sein zu wollen, wird vermutet, dass die Öl-in-Wasser-Emulgatoren zusammen mit einem Teil der Wasser-in-Öl-Emulgatoren lamellare Flüssigkristallphasen bilden, die mit einem Teil des Wassers zu einer hydrophilen Gelphase aufgebaut werden. Diese hydrophile Gelphase umgibt die wässrige Bulkphase. In dieser wässrigen Bulkphase wiederum sind die lipophilen Komponenten, umgeben von einer lipophilen Gelphase, die von den Wasser-in-Öl-Emulgatoren mit einem Teil der ÖI-in-Wasser-Emulgatoren und etwas Wasser gebildet wird, dispergiert.
Der mindestens eine kosmetische Wirkstoff g) ist bevorzugt ausgewählt aus wasserlöslichen und öllöslichen Wirkstoffen der genannten Wirkstoffgruppen. Besonders bevozugt sind die wasserlöslichen Wirkstoffe.

Bevorzugte erfindungsgemäße Stiftzusammensetzungen sind dadurch gekennzeichnet, dass mindestens ein kosmetischer Wirkstoff g) in der äußeren, kontinuierlichen wässrigen Phase gelöst ist. Die O/W-Emulsionsbasis der erfindungsgemäßen Stiftzusammensetzungen bewirkt eine deutlich verbesserte und effizientere Wirkstoffabgabe im Vergleich zu den bekannten wasserfreien Suspensionsstiften und Wasser-in-Öl-Emulsionsstiften. Diese Wirkstofffreisetzung lässt sich indirekt sehr gut bestimmen über die Messung des elektrischen Widerstandes des jeweiligen Produktes. Die Messung des elektrischen Widerstandes derartiger Zusammensetzungen ist auch ein geeignetes Verfahren, um schnell und einfach die Unterscheidung zwischen einem Öl-in-Wasser- und einem Wasser-in-Öl-System treffen zu können. Ein Öl-in-Wasser-System weist wegen der kontinuierlichen Wasserphase eine hohe elektrische Leitfähigkeit und dementsprechend einen niedrigen elektrischen Widerstand auf. Die genaue Messanordnung und die Durchführung der Messung sind im folgenden beschrieben (siehe unten). Die erfindungsgemäßen Stifte weisen demnach einen elektrischen Widerstand von bevorzugt maximal 300 kΩ, besonders bevorzugt von maximal 100 kΩ und außerordentlich bevorzugt von maximal 80 kΩ auf. Die in WO 98/17238 A1 offenbarten Stifte hingegen weisen einen elektrischen Widerstand von mehr als 3000 kΩ auf; es handelt sich hierbei also offensichtlich um ein Wasser-in-Öl-System.
Die Verfestigung der erfindungsgemäßen kosmetischen Stifte erfolgt nicht auf der Basis von Seifengelen beziehungsweise Fettsäuresalz-Gelen, wobei unter Fettsäuren Alkan-, Alken- und Alkinsäuren mit mindestens 4 Kohlenstoffatomen, die substituiert, beispielsweise mit Hydroxylgruppen, verstanden werden. In einer besonders bevorzugten Ausführungsform sind die erfindungsgemäßen kosmetischen Stifte frei von Seifengelen beziehungsweise Fettsäuresalz-Gelen, insbesondere frei von Lithium-, Natrium-, Kalium-, Ammonium-, Diethanolamin- und Triethanolamin-Salzen von Fettsäuren.

Bevorzugte erfindungsgemäße kosmetische Stifte sind dadurch gekennzeichnet, dass sie mindestens einen anorganischen und/oder organischen polymeren Hydrogelbildner enthalten. Durch den Gehalt an einem derartigen Hydrogelbildner oder wasserquellenden Verdickungsmittel kann die Stabilität der erfindungsgemäßen Zusammensetzungen insbesondere während des Herstell- und Abfüllvorganges merklich verbessert werden. Der Hydrogelbildner verhindert die Sedimentation einzelner Bestandteile, beispielsweise gegebenenfalls vorhandener unlöslicher Füllstoffe, während des statischen Abkühlens. Besonders bevorzugte erfindungsgemäße kosmetische Stifte sind dadurch gekennzeichnet, dass sie mindestens einen anorganischen und/oder organischen polymeren Hydrogelbildner enthalten, der ausgewählt ist aus wasserlöslichen Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose, Methylhydroxyethylcellulose, weiterhin Xanthan-Gum, Sclerotium Gum, Succinoglucanen, Polygalactomannanen, insbesondere Guar-Gums und Johannisbrotkernmehl (Locust Bean Gum), insbesondere Guar-Gum und Locust Bean Gum selbst und den nichtionischen Hydroxyalkylguarderivaten und Johannisbrotkernmehl-Derivaten, wie Hydroxypropylguar, Carboxymethylhydroxypropylguar, Hydroxypropylmethylguar, Hydroxyethylguar und Carboxymethylguar, weiterhin Pectinen, Agar, Carragheen, Traganth, Gummi arabicum, Karayagummi, Taragummi, Gellan, Gelatine, Casein, Propylenglycolalginat, Alginsäuren und deren Salze, insbesondere Natriumalginat, Kaliumalginat und Calciumalginat, weiterhin Polyvinylpyrrolidonen, Polyvinylalkoholen, Polyacrylamiden, weiterhin Veegum, Laponiten und Bentonen, weiterhin - wenn auch weniger bevorzugt - physikalisch (z. B. durch Vorverkleisterung) und/oder chemisch modifizierten Stärken, insbesondere hydroxypropylierten Stärkephosphaten und Octenylstärkesuccinaten und deren Aluminium-, Calcium- oder Natriumsalzen, weiterhin - ebenfalls weniger bevorzugt - wasserlöslichen Acrylsäure-Acrylat-Copolymeren, Acrylsäure-Acrylamid-Copolymeren, Acrylsäure-Vinylpyrrolidon-Copolymeren, Acrylsäure-Vinylformamid-Copolymeren und Polyacrylaten.

Weitere besonders bevorzugte erfindungsgemäße kosmetische Stifte sind dadurch gekennzeichnet, dass sie mindestens einen anorganischen und/oder organischen polymeren Hydrogelbildner in einer Gesamtmenge von 0,1 - 3,0 Gew.-%, bevorzugt 0,3 - 2, 0 Gew.-%, besonders bevorzugt 0,5 -1,5 Gew.-% und außerordentlich bevorzugt 0,7 - 1,0 Gew.-%, enthalten, jeweils bezogen auf das Gewicht der gesamten erfindungsgemäßen Zusammensetzung.
Weitere besonders bevorzugte erfindungsgemäße kosmetische Stifte sind dadurch gekennzeichnet, dass sie mindestens einen anorganischen und/oder organischen polymeren Hydrogelbildner, ausgewählt aus Guar-Gum, Locust Bean Gum, Pectin(en), wasserlöslichen Celluloseethern, vor allem Hydroxyalkylcellulosen, insbesondere Hydroxypropylcellulose, Hydroxypropylmethylcellulose, Hydroxyethylcellulose, Carboxymethylcellulose, Cetylhydroxyethylcellulose, Hydroxybutylmethylcellulose und Methylhydroxyethylcellulose, sowie Mischungen hiervon, insbesondere Mischungen aus Guar-Gum, Locust Bean Gum und Pectin, in einer Gesamtmenge von 0,1- 3,0 Gew.-%, bevorzugt 0,3- 2, 0 Gew.-%, besonders bevorzugt 0,5- 1,5 Gew.-% und außerordentlich bevorzugt 0,7- 1,0 Gew.-%, enthalten, jeweils bezogen auf das Gewicht der gesamten erfindungsgemäßen Zusammensetzung.

Neben der günstigen Wirkstofffreisetzung bringt die Konfektionierung als Öl-in-Wasser-Dispersion/Emulsion weitere Vorteile mit sich. Zum einen kann die Zusammensetzung leicht von der Haut abgewaschen werden. Zum anderen kann sich bei oder nach dem Auftragen auf die Haut zusammen mit der Hautfeuchtigkeit eine pflegende Öl-in-Wasser-Creme ausbilden.
Überraschend und für den Fachmann nicht vorhersehbar wurde gefunden, dass die Ölkomponenten und der Wasser-in-Öl-Emulgator beziehungsweise das Wasser-in-Öl-Emulgatoren-Gemisch in Bezug auf ihre Löslichkeitsparameter aufeinander abgestimmt sein müssen, um Stiftzusammensetzungen mit anwendungstechnisch zufriedenstellenden Härten zu bilden. Bei der Definition des Löslichkeitsparameters im Sinne der vorliegenden Erfindung wird Bezug genommen auf die Veröffentlichung "Solubility - Effects in Product, Package, Penetration and Preservation" von Chr. D. Vaughan in Cosmetics & Toiletries, Vol. 103, October 1988, Seiten 47 - 69. Die dort veröffentlichten Werte der Löslichkeitsparameter sind in der Nicht-SI-Einheit (cal/cm³)^{0,5} notiert. Der Einfachheit halber soll in dieser Schrift diese Nicht-SI-Einheit beibehalten werden. Über die Beziehung 1 cal = 4,1860 Joule können die Werte leicht umgerechnet werden.

Zahlreiche der von Vaughan in Cosmetics & Toiletries, Vol. 103, October 1988, Seiten 47 - 69, tabellierten Löslichkeitsparameter sind nach der Hildebrand-Gleichung berechnet worden (siehe C.D. Vaughan: J. Soc. Cosmet. Chem., Vol. 36, Seiten 319 - 333 (Sept./Oct. 1985) und die darin zitierte Hildebrand-Gleichung sowie J. Am. Chem. Soc., Vol. 38, Seiten 1442 - 1473 (1916) und J. Hildebrand and R. Scott: The Solubility of Nonelectrolytes, 3rd Edition, Reinhold Publ. Corp., New York, 1949) und nachfolgend zusammengestellt. Vaughan verweist darauf, dass die Löslichkeitsparameter nicht nur nach der Hildebrand-Gleichung berechnet werden können, sondern beispielsweise auch mit Hilfe der Verdampfungsenthalpie (nach Scatchard, J. Am. Chem. Soc., Vol. 38, Seite 321 (1916)). Alle Bestimmungsmethoden können zu abweichenden Werten des Löslichkeitsparameters führen, insbesondere, wenn das chemische Material eine Säure- oder Base-Funktion hat.
Im Sinne der vorliegenden Erfindung ist es bevorzugt, dass der Abgleich der Löslichkeitsparameter der Ölkomponenten und des Wasser-in-Öl-Emulgators beziehungsweise des Wasser-in-Öl-Emulgatoren-Gemisches nur für Löslichkeitsparameter-Werte vorgenommen wird, die jeweils nach derselben Methode bestimmt wurden. Besonders bevorzugt werden die Löslichkeitsparameter-Werte, die nach der Hildebrand-Gleichung (siehe C.D. Vaughan: J. Soc. Cosmet. Chem., Vol. 36, Seiten 319 - 333 (Sept./Oct. 1985)) erhalten wurden, für den erfindungsgemäßen Abgleich herangezogen. Falls für eine bestimmte Kombination von Ölkomponente und Wasser-in-Öl-Emulgator kein nach derselben Methode bestimmtes Löslichkeitsparameter-Wertepaar erhältlich ist, können auch Werte, die nach verschiedenen, auch experimentellen, Methoden bestimmt wurden, verwendet werden. Letzteres ist erfindungsgemäß jedoch weniger bevorzugt.

**Tabelle 1: Löslichkeitsparameter verschiedener chemischer Komponenten (aus Cosmetics & Toiletries, Vol. 103, October 1988, Seiten 47 - 69)**

| MATERIAL NAME (CTFA) | Solubility | Ref. | MATERIAL NAME (CTFA) | Solubility Parameter (cal/c m³)^{0,5} | Ref. |
|---|---|---|---|---|---|
| with Dielectric Constant | Parameter (cal/cm³)^{0,5} | | with Dielectric Constant | | |
| | | | Cyclohexane (2.02) | 7.30 | E |
| | | | Dioctyl Ether | 7,30 | A |
| | | | Eicosane (020) | 7.32 | C |
| Helium (1.06) | 0.50 | *N | Lanolin Oil | 7.33 | LI |
| Hydrogen (1.23) | 2.50 | *N | Petrolatum | 7.33 | *0 |
| Propellant 13 | 2.59 | 0* | Behenic Acid | 7.35 | ID |
| Methane (1.70) | 4.70 | *0 | Diethyl Ether (4.34) | 7.37 | CO |
| Neon | 4.90 | *N | Corn Oil-Refined | 7.40 | LI |
| perfluorohexane | 5.68 | A | Cetane (016) | 7.41 | |
| Perfluoroctane | 5.72 | A | Heptane (1.92) | 7.41 | CO |
| Cyclomethicone D5 (2.50) | 5.77 | MO | Isostearyl Neopentanoate | 7.43 | M |
| Nitrogen (1.45) | 5.90 | *N | Octyl Palmitate | 7.44 | 0 |
| Dimethicone | 5.92 | *0 | Propyl Fluoride | 7.48 | C |
| Cyclomethicone D4 (2.39) | 5.99 | MO | Rice Oil - SO | 7.48 | LI |
| Squalane | 6.03 | MO | Tridecane (C13) | 7.48 | CO |
| Propellant 12 (2.13) | 6.11 | *0 | Propellant 11 (2.28) | 7.49 | 0 |
| Hexamethyldisiloxane (2.17) | 6.15 | MO | Cottonseed Oil | 7.52 | LI |
| Isocetyl Stearate | 6.19 | | Carbon Dioxide (1.60) | 7,53 | H |
| Squalene | 6,19 | MO | Isopropyl Linoleate | 7.55 | M |
| Polytetrafluoroethylene | 6.20 | | C o d Liver Oil | 7.56 | L1 |
| Propane | 6.21 | *0 | Erucic Acid | 7.57 | CD |
| Propellant 22(6.11) | 6.23 | MO | octane (1.95) | 7.58 | MO |
| Perfluorodecalin | 6.34 | A | Cetyl Octanoate | 7.59 | M |
| Neopentane | 6.38 | CO | Decene-1 | 7.59 | C |
| Safflower Oil | 6.42 | LI | Dodecene (2,01)(7.65-l) | 7.59 | C |
| Melene (C30) | 6.58 | C | Diethylhexyl Adipate | 7.60 | M |
| Docosane (C22) | 6.60 | I | Decane (1.99) | 7.62 | CD |
| Almond Oil | 6,81 | LI | C12-15 Alcohols Benzoate | 7,63 | MD |
| Isopentane | 6.82 | CO | Isobutyl Stearate | 7.65 | 0 |
| Avocado Oil | 6.83 | LI | Butyl Myristate | 7,68 | D |
| Nonacosane (D29) | 6.83 | C | Butyl Stearate(3.11) | 7.68 | CO |
| Arachidic Acid | 6.85 | H | Stearic Acid (C18)(2,30) | 7.74 | IO |
| Pristane | 6.85 | MD | Dioctyl Maleate | 7 75 | 0 |
| Decyl Oleate | 6.92 | | Octyl Fluoride | 7.76 | AG |
| C8-Isoparaffin (1.94) | 6.93 | MO | Isopropyl Palmitate | 7.78 | 0 |
| Diisopropyl Ether (3.88) | 6.95 | ICE | Dioctyl Adipate | 7.82 | M |
| Argon (1.53) | 7.00 | *N | Oleth-3 | 7.83 | *0 |
| Sperm Oil | 7.09 | *0 | Diethyl Amine | 7.86 | C |
| White Mineral Oil | 7.09 | *0 | Linolenic Acid | 7.86 | C0 |
| Pentane | 7.10 | *0 | Olive Oil | 7.87 | *0 |
| Tricosane (C23) | 7.13 | C | Palmitic Acid (C16) (22.30) | 7.89 | ID |
| Isodecyl Oleate | 7.17 | M | Oleic Acid(2,46) | 7.91 | 10 |
| Propellant 113 | 7.19 | H | PEG-4 Stearate | 7.92 | 0 |
| Oxygen (1.50) | 7.20 | *N | Tetraethyl Lead | 7.92 | E |
| Cholesteryl Oleate | 7.24 | | Tridecyl Neopentanoate | 7.92 | LI |
| Peanut Oil | 7.74 | L1 | Pentaerythrityl Tetraoleate | 7.98 | LI |
| Hexane (1.88) | 7.28 | C O | Tocopheryl Acetate | 7.98 | M |
| Linseed Oil | 7.29 | *0 | Ethyl Myristate | 8.00 | C |
| Octadecane (C18) | 7.29 | C | | | |
| Isopropyl Myristate | 8.02 | 0 | Stearyl Alcohol (C18) | 8.90 | 10 |
| Terpentine (pinene)(2.70) | 8.03 | CO | Methyl Hexyl Ketone | 6.91 | A |
| Human Erythrocyte | 8.05 | | Octyl Dodecanol | 8.92 | OM |
| Methyl Oleate (3.21) | 8.05 | CO | Butyl Acetate (5.01) | 8.93 | CO |
| Cetyl Acetate | 8.06 | 0 | Cetyl Alcohol (CIG) | 8.94 | 10 |
| Methyl Linoleate | 8.08 | C | alpha-Thujone | 8.94 | A |
| Isostearic Acid | 8.09 | 0 | Toluene (2.38) | 8.94 | C |
| Coconut Oil | 8.10 | L1 | Oleyl Alcohol | 8.95 | CO |
| Myristic Acid (C14) | 8.10 | 10 | Propylene Oxide | 8.99 | A |
| Dibutylamine | 8.15 | * | Aspergillus Niger | 9.00 | P |
| Eucalyptol (Cineole) | 8.17 | L1 | G Octyl Dimethyl PABA 9.34 | 9.01 | OM |
| Natural Rubber | 8.20 | H | Propyl Acetate | 9.02 | CO |
| Octylamine | 8,21 | A | Chloroform | 9.05 | A |
| Propylene Glycol Dipelargonate | | LI | Benzene (2,28) | 9.08 | 8 |
| Titanium Isopropoxide | 8.21 | M | PEG-20 Stearate | 9.08 | 33 |
| Melissyl Alcohol (C 30) | 8.22 | CO | Ceteth-20 | 9.10 | H |
| Glycol Distearate | 8.24 | J3 | Methyl Butyl Methacrylate CO | 8.10 | M |
| Glycol Stearate | 8,28 | J3 | Octyl Methoxycinnamate | 9.10 | M |
| Capric/Caprylic Triglycerid | 8.29 | Li | Methyl Butyl Ketone | 9.11 | E |
| Isosteareth-2 | 8.29 | LI | Myristyl Alcohol (C14) | 9.16 | IO |
| PPG-2 Myristyl Ether | 8.29 | LI | Polysorbate-20 | 9.16 | 33 |
| Ricinoleic Acid | 8.30 | C | THE (7.58) | 9.16 | |
| Staphylococcus Aureus | 8.30 | P | BHT | 9.17 | 0 |
| Glyceryl Isostearate | 8.31 | J3 | Tocopherol | 9.17 | H |
| Glyceryl Stearate (mono) | 8.31 | *0 | Lauryl Lactate | 9.18 | M |
| Laureth-4 | 8.31 | 33 | PEG-40 Stearate | 9.18 | J3 |
| Limonene (2,30) | 8.33 | C | Ethyl Acetate (6.02) | 9.19 | CO |
| Propylene Glycol Laurate | 8.33 | LI | Tributyl Citrate | 9.20 | M |
| Octyl Mercaptan | 8.35 | K | Ethyl Acrylate | 9.22 | A |
| PEG-2 Stearate | 8.36 | J3 | Propionaldehyde | 9.22 | A |
| Ethyl Caprate (C10) | 8.39 | A | Methyl Propyl Ketone | 9.27 | C |
| Radon | 8.40 | *N | Dipropyl Nitrosamine | 9.29 | |
| Amyl Acetate | 8.43 | C | alpha-Bisabolol | 9.30 | M |
| Glyceryl Stearate SE | 8.43 | J3 | Pseudomonas Aeroginosa | 9.30 | P |
| Diisopropyl Adipate | 8.46 | E0 | Trichomonas Ment. | 9,30 | P |
| Lauric Acid (C12) | 8.46 | 10 | Caprylic Acid (C8)(2.45) | 9.32 | 60 |
| Polyethylene (2,35) | 8.50 | "0 | Cetyl Lactate | 9.32 | Pi |
| Diisopropyl Amine | 8.51 | *0 | PEG-100 Stearate | 9.35 | 33 |
| Polyglycery1-3 Oleate | 8.52 | J3 | Trimethyl Citrate | 9.39 | H |
| tate(AC400) Ace- | 8.55 | *0 | Klebsiella Pneumoniae | 9.40 | P |
| Ethyl Caprylate (C8) | 8.57 | A | Methyl Methacrylate Copolymer | 9.40 | H |
| Octyl Acetate | 8.58 | A | Nicotine | 9.40 | C |
| Octyllodide | 8.58 | A | Camphor | 9.45 | C |
| Ethyl Oleate (3.17) | 8.60 | | Oxidized Polyethylene (AC392) | 9.50 | *0 |
| Isopropylbenzene (12.38) | 8.60 | * | Lauryl Alcohol (C12) | 9.51 | CO |
| Sorbitan Laurate | 8.61 | 0 | Pulegone | 9.51 | A |
| Behenyl Alcohol (C22) | 8.63 | ID | Cholesterol | 9.55 | 0 |
| Carbon Tetrachloride (2.23) | 8.64 | C | Ethylene/Vinyl Acetate (AC430) | 9,55 | *0 |
| Butyl Mercaptan | 8.6$ | KA | Methylene Chloride (9.08) | 9.55 | E |
| Isostearyl Alcohol | 8.67 | 0 | Dimethyl Isosorbide | 9.58 | M |
| Lauraldehyde | 8.66 | A | PPG-2 Methyl Ether | 9.60 | |
| Ethyl Caproate (C6) | 8.69 | A | Acetaldehyde (21.8) | 9.61 | A |
| Cholesteryl Propionate | 8.70 | * | Undecyl Alcohol | 9.51 | CO |
| Isocetyl Alcohol | 8.71 | M | Linalool | 9.62 | C |
| Bornyl Acetate | 8.74 | CA | MEK(18.50) 9.53A | 9.63 | CO |
| Ethyl Mercaptan | 8.75 | K | Acetylacetone | 9.88 | M |
| Decanone-2 | 8.76 | A | Amyl Dimethyl PABA | 9.72 | C |
| Octanal | 8.77 | C | Methyl Iodide | 9.75 | CO |
| Trifluoroactylacetone | 8.77 | A | Decyl Alcohol (C10) (8.10) | 9.78 | |
| Cholesteryl Myristate | 8.80 | * | Chlorine | 9.80 | |
| Zinc Stearate | 8.80 | 0 | Ethylhexanol | 9.80 | A |
| Citronella | 8.83 | CD | Stratum Corneum-Porcine | 9.80 | |
| Diethyl Ketone (17.00) | 8.85 | E | Acetone (20.70) | 9.87 | C |
| Methyl Isobutyl Ketone (14.70) | 8.85 | EO | Citronellol | 9.88 | A |
| Oxidized Polyethylene (AC629) | 8.85 | *0 | Dibutyl Phthalate (6.44) | 9.88 | M |
| Methyl Heptyl Ketone | 8.86 | A | Menthyl Anthranilate | 9.89 | M |
| Myristyl Lactate | 8.87 | M | PPG-4 | 9.89 | M |
| Capric Acid (C10) | 8.88 | 10 | Ethoxyethanol (29.60) | 9.90 | *M |
| Methyl Caproate (CB) | 8.88 | B | Ethylene Oxide (13.90) | 9.93 | A |
| Arachidyl Alcohol (C20) | 8.89 | CO | Menthol | 9.94 | CO |
| Dipropyl Ketone | 8.89 | C | Tributyrin | 9.97 | 0 |
| Muscone | 8.89 | CO | Butoxydiglyool-Bucarbitol | 9.98 | |
| Candida Albicans | 8.90 | P | Nitrous Oxide (1.60) | 10.00 | 'H |
| Castor Oil | 8.90 | H | Dioxane (2.21) | 10.01 | * |
| Elaidyl Alcohol | 8.90 | CO | Ethyl Benzoate (6.02) | 10,01 | C |
| beta-lonone | 8.90 | CO | Caproic Acid (C8) (2.53) | 10.05 | 80 |
| Polystyrene | 8.90 | M | Salicylic Acid | 10.06 | C |
| Nicoteine | 10.08 | C | Copper Acetylacetonide | 11.60 | |
| Octanol/Caprylic (C8) Alcohol (10.34) | 10.09 | CO | | | |
| Acetic Anhydride (22.40) | 10,12 | C | Sulfamethoxazole | 11.60 | Jr |
| Nerol | 10.13 | C | PEG-4 (20,44) | 11.61 | DO |
| Ethyl Cinnamate | 10.14 | A | Acetohexamide | 11,64 | |
| Diethyl Nitrosamine | 10.16 | C | N-Methylpyrrolidone | 11.71 | A |
| Octyl Salicylate | 10.17 | m | Propyl Alcohol (20.10) | 11.73 | CO |
| Griseofulvin | 10.20 | M | Dimethyl Nitrosamine | 11.74 | C |
| Dioctyl Malate | 10.21 | M | Pentobarbital | 11.75 | 31 |
| Geraniol | 10.21 | CO | Butadiene Diepoxide | 11.78 | A |
| Butyl Lactate | 10.27 | AO | Dipropylene Glycol (PPG-2) | 11.78 | M |
| t-Butyl Alcohol (10.90) | 10.28 | CO | Phthalide | 11.78 | C |
| Morpholine (7.33) | 10.28 | C | Lysine | 11.79 | 31 |
| Homosalate | 10.29 | GM | Phenethyl Alcohol | 11,70 | CO |
| Valeric Acid (C5) | 10.29 | A | Acetonitrile (37.5) | 11.81 | AO |
| Polyethylene Terephthalate (PET) | 10.30 | * | Cinnamic Acid | 11.83 | C |
| Pyridine (12.3) | 10,30 | A | p-Nitrotoluene (24.20) | 11.83 | |
| Phenyl Acetate (5.23) | 10.33 | | Phenoxyethanol | 11.87 | CO |
| Thiolacetic Acid | 10.38 | A | Butobarbital | 11.90 | 31 |
| Methoxypropanol | 10.40 | | Sulfadiazine | 11.90 | |
| Diethyl Toluamide | 10.46 | M | Butalbital | 11,05 | 31 |
| Nonoxynol-1 | 10.47 | * | Cinnamyl Alcohol | 11.96 | C |
| Borneol | 10.48 | C | Sorbic Acid | 11.97 | MO |
| Methyl Benzoate (6.59) | 10.48 | E | Methylparaben | 11.98 | 0 |
| Hexyl Alcohol (13.30) | 10.50 | ICI | Hydroxyanisole | 12.00 | C |
| SAN (85/15) | 10,50 | * | Benzocaine | 12.05 | |
| Butoxyethanol (9.30) | 10.53 | E | Triethylene Glycol (23.69) | 12.21 | MO |
| Formaldehyde | 10.54 | C | Alanine | 12.23 | 31 |
| o-Nitrotoluene (27.40) | 10.55 | | Nitromethane | 12.27 | C |
| Butylparaben | 10.57 | * | Benzyl Alcohol (13.10) | 12.31 | 0 |
| Propionitrile | 10.52 | A | Hexylene Glycol | 12.32 | * |
| Tripropylene Glycol (PPG-3) | 10.60 | M | Butyramide | 12.33 | A |
| Methyl Salicylate (9,41) | 10.62 | Co | Human Serum Albumin A | 12.33 | J1 |
| Acetophenone (17.39) | 10.64 | C | Vanillin | 12.34 | D |
| Diacetone Alcohol (18.20) | 10.67 | CO | BHA | 12.37 | 0 |
| Ethyl Anthranilate | 10.67 | C | Acetic Acid (6.15) | 12.40 | CO |
| Naphthylene | 11.74 | 8 | Cyclobarbital | 12.40 | J1 |
| Phenylpentanol | 10.74 | A | Diisopropanolamine | 12.40 | A |
| Butyric Acid (2.97) | 10.76 | E | Ethyl Dihydroxypropyl PABA | 12,42 | M |
| Cyclopentanone | 10.77 | | o-Propylene Diamine | 12.43 | 0 |
| Thymol | 10.77 | C | p-Oinitrobenzene | 12.49 | B |
| Triacetin | 10.77 | 0 | Ethyl Alcohol (24.30) | 12.55 | CO |
| Methoxyethanol (16.90) | 10.80 | * | Rat Gut Membrane | 12.60 | |
| Amyl Alcohol (13.90) | 10.84 | CE | Sulfamethazine | 12.60 | J1 |
| Ethanedithiol | 10.87 | A | Sulfisomidinc | 12.70 | 31. |
| Ethyl Hexanediol | 10.89 | A | Sulfur (3.55) | 12.70 | |
| Trichloroacetic Acid | 10.89 | E | Phenol (9.78) | 12.79 | CE |
| Benzalphthalide | 10.90 | 0* | Sulfisomidine | 12.60 | |
| Testosterone | 10.90 | | Allobarbital | 12.85 | 31 |
| Cinnamaldehyde | 10,92 | C | o-Nitroaniline (34.50) | 12,80 | 0 |
| Propylparaben | 10.94 | GM | Pyruvic Acid | 12.94 | * |
| Valine | 10.94 | J1 | Phenobarbital | 13.00 | 31. |
| Tolbutamide | 10.98 | | Isopropanolamine | 13.02 | A |
| Benzaldehyde (17-80) | 11.00 | CO | Adipic Acid | 13.04 | 0 |
| Trüsopropanolamine | 11.02 | M | BAL (2,3-Dimercapto-1-propanol) | 13,10 | |
| Phenylbutanol | 11.04 | A | Sulfathiazole | 13.10 | |
| D&C Red 22 (Eosin) | 11.15 | L. | Glutathione | 13.18 | G |
| Butyl Alcohol (17.51) | 11.18 | CR | Butylene Glycol | 13,20 | CO |
| Cellulose Acetate | 11.20 | m | m-Nitroaniline | 13.23 | C |
| Methyl Anthranilate | 11.22 | C | Triethanolamine (29.36) | 13,28 | MO |
| Caproamide (C6) | 11.24 | M | Propylene Carbonate (65,00) | 13.35 | |
| Isopropyl Alcohol (18,30) | 11.24 | CO | Benzamide | 13.38 | B |
| Nitrocellulose | 11.25 | MO | Dimethyl Sulfoxide (46.68) | 13,40 | H |
| Hexobarbital | 11.30 | 31 | Sulfamerazine | 13.40 | 31 |
| Secobarbital | 11.30 | 31 | Propionamide | 13,46 | AC |
| p-Anisaldehyde | 11.32 | A | Barbital | 13.50 | 31 |
| PEG-8 | 11.34 | MO | Mercaptoethanol | 13.55 | A |
| Panthenol | 11.39 | MO | Propiolactone | 13,56 | A |
| Propionic Acid (3.35) | 11.40 | EA | Diethylene Glycol (31.70) | 13.61 | ED |
| Glyoxal | 11.46 | C | Propargyl Alcohol | 13,61 | A |
| Phenylpropanol | 11.46 | A | p-Nitroaniline (56,30) | 13.67 | A |
| Methyl Lactate | 11.47 | CO | Caffeine | 13.80 | |
| PEG-6 (16.00) | 11,47 | 00 | Thiodiglycol | 13,80 | H |
| | | | Thioglycolic Acid | 13.86 | A |
| PEG-5 (18.16) | 11.54 | 00 | Sulfameter | 13.90 | J1 |
| Phenylalanine | 11.57 | G | Diethanolamine | 13.95 | M |
| Propylene Glycol (32.00) | 14.00 | CO | Pyrrolidone | 14.00 | * |
| Theophyllin | 14.00 | | Hexyl Resorcinol | 14.06 | * |
| Aspartic Acid | 14.11 | J1 | Sodium Lauryl Sulfate | 14.18 | * |
| Pyrrolidinone-2 | 14.22 | | Methyl Alcohol (32.70) | 14.33 | CO |
| Ethylene Glycol(37.00) | 14.50 | CO | Urea | 14.50 | G |
| Hydroquinone | 14.62 | | Formic Acid (58.5 | 14.72 | E |
| Lactic Acid (22.00) | 14.81 | | PABA 14.56G | 14.82 | DO |
| Resorcinol | 14.96 | C | Acetamide MEA | 15.11 | J1 |
| Histidine | 15.25 | J1 | p-Hydroxybenzoic Acid | 15.30 | * |
| Ethanolamine (37.72) | 15.41 | *M | Pyrogallol | 15.41 | A |
| Sodium Capryl Sulfate (14.84) | 15.80 | * | Acetamide (59.00) | 16.03 | C |
| Erythritol | 16.06 | * | Glycerin (42.50) | 16.26 | E0 |
| Formamide (109.0) | 17.82 | E | Ammonia (16.90) | 18.05 | 0 |
| Lactose | 19.50 | * | Water (80.10) | 23.40 | CN |

| | | | | | |
|---|---|---|---|---|---|
| Referenzen NOTE: * = Löslichkeitsparameter-Werte aus der Literatur Zitatangaben für die Herkunft der Löslichkeitsparameter: A. Aldrich Chemical Co, Catalog 1986 gram B. Beilstein's Index C. Chemical Rubber Handbook of Chemistry & Physics, 42d Ed., (1961-1962) D. Dictionary of Organic Compounds E. Eastman Organic Chemical Bulletin 47, No.1, 1975 F. Fisher Scientific Catalog- 1986 G. Group Contribution Method of Hay, Van Krevelen and Feodors. H. HANDBOOK OF SOLUBILITY PARAMETERS, A.F. Barton, Chemical Rubber Publ.1985 I. INDUSTRIAL WAXES, H. Bennett, Chemical Pub. Co. J. Journal Reference by number 0(x). J1. J. Pharm. Sci.75, (7), 639 J2. Pharm. Acta Helv., 48, 549 (1973) J3. Am. Cosmet. Perf., 87, Seite 85 (1972) K. Kolthof & Elving: Treatise on Analytical Chemistry L. Laboratory Determination by L(1)-Consolubilizer Study L(2)-Solubility Study Unpublished M. Manufacturer's Physical Date by Personal Communication N. Hildebrand & Scott: The Solubility of Nonelectrolytes. Dover Press O. Original published values JSCC 36, 319 P. Pharm. Acta Helv. 81, (3), 95 Antimicrobial Activity and Solubility Parameters-C.V./F.W. | | | | | |

In den erfindungsgemäßen Stiftzusammensetzungen weichen der (mittlere) Löslichkeitsparameter der Gesamtheit der enthaltenen Öle in Gegenwart von linearen gesättigten Fettalkoholen mit einer Kettenlänge von mindestens 8 Kohlenstoffatomen um maximal -0,7 (cal/cm³)^{0,5} bzw. maximal +0,7 (cal/cm³)^{0,5}, bevorzugt um maximal -0,6 (cal/cm³)^{0,5} bzw. maximal +0,6 (cal/cm³)^{0,5}, besonders bevorzugt um maximal -0,4 (cal/cm³)^{0,5} bzw. maximal +0,5 (cal/cm³)^{0,5}, und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -0,4 (cal/cm³)^{0,5} bzw. maximal +0,7 (cal/cm³)^{0,5}, bevorzugt um maximal -0,3 (cal/cm³)^{0,5} bzw. maximal +0,6 (cal/cm³)^{0,5}, besonders bevorzugt um maximal -0,2 (cal/cm³)^{0,5} bzw. maximal +0,5 (cal/cm³)^{0,5}, vom (mittleren) Löslichkeitsparameter des Wasser-in-Öl-Emulgators/der Wasser-in-Öl-Emulgatoren ab. Falls Wasser-in-Öl-Emulgatoren-Gemische oder Ölgemische eingesetzt werden, wird jeweils der mittlere Löslichkeitsparameter der Mischung betrachtet, wobei das arithmetische Mittel gemäß dem Gewichtsanteil der Einzelkomponenten betrachtet wird. Im Rahmen der Erfindung ist es weiterhin möglich, dass ein Gewichtsanteil der eingesetzten, unter Normalbedingungen flüssigen Öle von maximal 20 Gew.-% aus Ölen besteht, deren Löslichkeitsparameter um mehr als -0,4 bzw. -0,7 (cal/cm³)^{0,5} oder um mehr als +0,7 (cal/cm³)^{0,5} vom (mittleren) Löslichkeitsparameter des Wasser-in-Öl-Emulgator(gemisch)s abweicht. In einer besonders bevorzugten Ausführungsform der Erfindung sind keine unter Normalbedingungen flüssigen Öle enthalten, deren Löslichkeitsparameter um mehr als ± 1,0 (callcm³)^{0,5}, bevorzugt um ± 0,7 (cal/cm³)^{0,5} und besonders bevorzugt um ± 0,5 (cal/cm³)^{0,5} vom (mittleren) Löslichkeitsparameter des Wasser-in-ÖI-Emulgators/der Wasser-in-Öl-Emulgatoren abweicht.

### Lipid- oder Wachsmatrix

Die Lipid- oder Wachsmatrix der erfindungsgemäßen Stiftzusammensetzungen umfasst mindestens eine Lipid- oder Wachskomponente mit einem Schmelzpunkt > 50°C, die nicht den nichtionischen Öl-in-Wasser-Emulgatoren mit einem HLB-Wert von mehr als 7 oder den nichtionischen Wasser-in-Öl-Emulgatoren mit einem HLB-Wert größer 1,0 und kleiner/gleich 7,0, die mit Wasser allein oder mit Wasser in Gegenwart eines hydrophilen Emulgators flüssigkristalline Strukturen ausbilden können, zuzurechnen ist.

Generell sind Wachse von fester bis brüchig harter Konsistenz, grob bis feinkristallin, durchscheinend bis opak, jedoch nicht glasartig, und schmelzen oberhalb von 50 °C ohne Zersetzung. Sie sind schon wenig oberhalb des Schmelzpunktes niedrigviskos und zeigen eine stark temperaturabhängige Konsistenz und Löslichkeit.

Erfindungsgemäß bevorzugt sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs, Schellackwachs und Walrat. Im Sinne der Erfindung kann es besonders bevorzugt sein, hydrierte oder gehärtete Wachse einzusetzen. Als Wachskomponente sind auch chemisch modifizierte Wachse, insbesondere die Hartwachse, wie z. B. Montanesterwachse, hydrierte Jojobawachse und Sasolwachse, einsetzbar. Zu den synthetischen Wachsen, die ebenfalls erfindungsgemäß bevorzugt sind, zählen beispielsweise Polyalkylenwachse und Polyethylenglycolwachse, C₂₀-C₄₀-Dialkylester von Dimersäuren, C₃₀-₅₀-Alkylbienenwachs sowie Alkyl- und Alkylarylester von Dimerfettsäuren.

Eine besonders bevorzugte Wachskomponente ist ausgewählt aus mindestens einem Ester aus einem gesättigten, einwertigen C₁₆-C₆₀-Alkohol und einer gesättigten C₈-C₃₆-Monocarbonsäure. Erfindungsgemäß zählen hierzu auch Lactide, die cyclischen Doppelester von α-Hydroxycarbonsäuren der entsprechenden Kettenlänge. Ester aus Fettsäuren und langkettigen Alkoholen haben sich für die erfindungsgemäße Zusammensetzung als besonders vorteilhaft erwiesen, weil sie der Antitranspirantzubereitung ausgezeichnete sensorische Eigenschaften und dem Stift insgesamt eine hohe Stabilität verleihen. Die Ester setzen sich aus gesättigten, verzweigten oder unverzweigten Monocarbonsäuren und gesättigten, verzweigten oder unverzweigten einwertigen Alkoholen zusammen. Auch Ester aus aromatischen Carbonsäuren bzw. Hydroxycarbonsäuren (z. B. 12-Hydroxystearinsäure) und gesättigten, verzweigten oder unverzweigten Alkoholen sind erfindungsgemäß einsetzbar, sofern die Wachskomponente einen Schmelzpunkt > 50°C hat. Besonders bevorzugt ist, die Wachskomponenten zu wählen aus der Gruppe der Ester aus gesättigten, verzweigten oder unverzweigten Alkancarbonsäuren einer Kettenlänge von 12 bis 24 C-Atomen und den gesättigten, verzweigten oder unverzweigten Alkoholen einer Kettenlänge von 16 bis 50 C-Atomen, die einen Schmelzpunkt > 50°C haben.
Insbesondere können als Wachskomponente C₁₆₋₃₆-Alkylstearate und C₁₈₋₃₈-Alkylhydroxystearoyl-stearate, C₂₀₋₄₀-Alkylerucate sowie Cetearylbehenat vorteilhaft sein. Das Wachs oder die Wachskomponenten weisen einen Schmelzpunkt > 50°C, bevorzugt > 60°C, auf.

Eine besonders bevorzugte Ausführungsform der Erfindung enthält als Wachskomponente ein C₂₀-C₄₀-Alkylstearat. Dieser Ester ist unter den Namen Kesterwachs^{®} K82H oder Kesterwachs^{®} K80H bekannt und wird von Koster Keunen Inc. vertrieben. Es handelt sich um die synthetische Nachahmung der Monoesterfraktion des Bienenwachses und zeichnet sich durch seine Härte, seine Ölgelierfähigkeit und seine breite Kompatibiltät mit Lipidkomponenten aus. Dieses Wachs kann als Stabilisator und Konsistenzregulator für W/O- und O/W-Emulsionen verwendet werden. Kesterwachs bietet den Vorteil, dass es auch bei geringen Konzentrationen eine exzellente Ölgelierfähigkeit aufweist und so die Stiftmasse nicht zu schwer macht und einen samtigen Abrieb ermöglicht. Eine weitere besonders bevorzugte Ausführungsform der Erfindung enthält als Wachskomponente Cetearylbehenat, d. h. Mischungen aus Cetylbehenat und Stearylbehenat. Dieser Ester ist unter dem Namen Kesterwachs^{®} K62 bekannt und wird von Koster Keunen Inc. vertrieben.

Weitere bevorzugte Lipid- oder Wachskomponenten mit einem Schmelzpunkt > 50°C sind die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2 - 6 Kohlenstoffatomen, solange sie einen Schmelzpunkt oberhalb von 50 °C aufweisen, beispielsweise bevorzugt C₁₈ - C₃₆ Acid Triglyceride (Syncrowax^{®} HGL-C).
Erfindungsgemäß ist als Wachskomponente hydriertes Rizinusöl, erhältlich z. B. als Handelsprodukt Cutina^{®} HR, besonders bevorzugt.
Weitere bevorzugte Lipid- oder Wachskomponenten mit einem Schmelzpunkt > 50°C sind die gesättigten linearen C₁₄ - C₃₆-Carbonsäuren, insbesondere Myristinsäure, Palmitinsäure, Stearinsäure und Behensäure sowie Mischungen dieser Verbindungen, z. B. Syncrowax^{®} AW 1C (C₁₈ - C₃₆-Fettsäuren) oder Cutina® FS 45 (Palmitin- und Stearinsäure).

Bevorzugte erfindungsgemäße kosmetische Stifte sind dadurch gekennzeichnet, dass die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂- C₃₀-Carbonsäuren, die hydroxyliert sein können, Candelillawachs, Carnaubawachs, Bienenwachs, gesättigten linearen C₁₄- C₃₆-Carbonsäuren sowie Mischungen der vorgenannten Substanzen. Besonders bevorzugte Lipid- oder Wachskomponenten-Mischungen a) sind ausgewählt aus Mischungen von Cetylbehenat, Stearylbehenat, gehärtetem Rizinusöl, Palmitinsäure und Stearinsäure. Weitere besonders bevorzugte Lipid- oder Wachskomponenten-Mischungen a) sind ausgewählt aus Mischungen von C₂₀-C₄₀-Alkylstearat, gehärtetem Rizinusöl, Palmitinsäure und Stearinsäure.

Besonders bevorzugte erfindungsgemäße kosmetische Stifte sind dadurch gekennzeichnet, dass die Lipid- oder Wachskomponente a) ausgewählt ist aus Mischungen von Estern aus einem gesättigten, einwertigen C₁₆-C₆₀-Alkanol und einer gesättigten C₈-C₃₆-Monocarbonsäure, insbesondere C₂₀-C₄₀-Alkylstearat, Glycerintriestern von gesättigten linearen C₁₂- C₃₀-Carbonsäuren, die hydroxyliert sein können, insbesondere hydriertem Rizinusöl, und gesättigten linearen C₁₄-C₃₆-Carbonsäuren, insbesondere Palmitinsäure und Stearinsäure.

Weitere bevorzugte erfindungsgemäße kosmetische Stifte sind dadurch gekennzeichnet, dass die Lipid- oder Wachskomponente/n a) insgesamt in Mengen von 4-20 Gew.-%, bevorzugt 8-15 Gew.-%, bezogen auf die Gesamtzusammensetzung, enthalten ist. In einer besonders bevorzugten Ausführungsform ist/sind der/die Ester aus einem gesättigten, einwertigen C₁₆-C₆₀-Alkohol und einer gesättigten C₈-C₃₆-Monocarbonsäure, der/die Lipid- oder Wachskomponente/n a) darstellt/darstellen, in Mengen von 2 - 10 Gew.-%, bevorzugt 2 - 6 Gew.-%, bezogen auf die Gesamtzusammensetzung, enthalten.

### Öl-in-Wasser-Emulgatoren

Die erfindungsgemäßen Stiftzusammensetzungen enthalten mindestens einen nichtionischen Öl-in-Wasser-Emulgator mit einem HLB-Wert von mehr als 7, wobei das gesamte ÖI-in-Wasser-Emulgatorsystem einen gewichtsmittleren (gewichtsgemittelten) HLB-Wert im Bereich von 10 - 19 aufweist. Hierbei handelt es sich um dem Fachmann allgemein bekannte Emulgatoren, wie sie beispielsweise in Kirk-Othmer, "Encyclopedia of Chemical Technology", 3. Aufl., 1979, Band 8, Seiten 913 - 916, aufgelistet sind. Für ethoxylierte Produkte wird der HLB-Wert nach der Formel HLB = (100 - L) : 5 berechnet, wobei L der Gewichtsanteil der lipophilen Gruppen, das heißt der Fettalkyl- oder Fettacylgruppen, in den Ethylenoxidaddukten, ausgedrückt in Gewichtsprozent, ist. Bei der Auswahl erfindungsgemäß geeigneter nichtionischer Öl-in-Wasser-Emulgatoren ist es besonders bevorzugt, ein Gemisch von nichtionischen Öl-in-Wasser-Emulgatoren einzusetzen, um die Stabilität der erfindungsgemäßen Stiftzusammensetzungen optimal einstellen zu können. Die einzelnen Emulgatorkomponenten liefern dabei einen Anteil zum Gesamt-HLB-Wert oder mittleren HLB-Wert des ÖI-in-Wasser-Emulgatorgemisches gemäß ihrem Mengenanteil an der Gesamtmenge der ÖI-in-Wasser-Emulgatoren. Erfindungsgemäß beträgt der mittlere HLB-Wert des ÖI-in-Wasser-Emulgatorgemisches 10-19, bevorzugt 12-18 und besonders bevorzugt 14 - 17. Um derartige mittlere HLB-Werte zu erzielen, werden bevorzugt ÖI-in-Wasser-Emulgatoren aus den HLB-Wertbereichen 10- 14, 14- 16 und gegebenenfalls 16 - 19 miteinander kombiniert. Selbstverständlich können die ÖI-in-Wasser-Emulgatorgemische auch nichtionische Emulgatoren mit HLB-Werten im Bereich von > 7 - 10 und 19 - 20 enthalten; derartige Emulgatorgemische können erfindungsgemäß ebenfalls bevorzugt sein. Die erfindungsgemäßen kosmetischen Stifte können aber in einer anderen bevorzugten Ausführungsform auch nur einen einzigen Öl-in-Wasser-Emulgator mit einem HLB-Wert im Bereich von 10 -19 enthalten.
Bevorzugte erfindungsgemäße kosmetische Stifte sind dadurch gekennzeichnet, dass die nichtionischen Öl-in-Wasser-Emulgatoren b) ausgewählt sind aus ethoxylierten C₈-C₂₄-Alkanolen mit durchschnittlich 10-100 Mol Ethylenoxid pro Mol, ethoxylierten C₈-C₂₄-Carbonsäuren mit durchschnittlich 10-100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit n = 2 bis 10 Glycerineinheiten und mit 1 bis 4 gesättigten oder ungesättigten, linearen oder verzweigten, gegebenenfalls hydroxylierten C₈- C₃₀-Fettsäureresten verestert, sofern sie einen HLB-Wert von mehr als 7 aufweisen, sowie Mischungen der vorgenannten Substanzen.
Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8 - 24 Kohlenstoffatomen und n, die mittlere Anzahl der Ethylenoxid-Einheiten pro Molekül, für Zahlen von 10 - 100, vorzugsweise 10 - 30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 10 - 100 Mol Ethylenoxid an technische Fettalkohole mit 12 - 18 Kohlenstoffatomen, wie beispielsweise Kokos-, Palm-, Palmkern- oder Talgfettalkohol, sind geeignet.
Die ethoxylierten C₈-C₂₄-Carbonsäuren haben die Formel R¹O(CH₂CH₂O)ₙH, wobei R¹O steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 10 -100, vorzugsweise 10 - 30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachyinsäure, Gadoleinsäure, Behensäure, Erucasäure und Brassidinsäure sowie deren technische Mischungen. Auch Addukte von 10 - 100 Mol Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.
Besonders bevorzugt eingesetzt werden die C₁₂-C₁₈-Alkanole oder die C₁₂-C₁₈-Carbonsäuren mit jeweils 10 - 30 Einheiten Ethylenoxid pro Molekül sowie Mischungen dieser Substanzen, insbesondere Ceteth-12, Ceteth-20, Ceteth-30, Steareth-12, Steareth-20, Steareth-30, Laureth-12 und Beheneth-20.
Weiterhin werden vorzugsweise C₈ - C₂₂-Alkylmono- und -oligoglycoside eingesetzt. C₈ -C₂₂-Alkylmono- und -oligoglycoside stellen bekannte, handelsübliche Tenside und Emulgatoren dar. Ihre Herstellung erfolgt insbesondere durch Umsetzung von Glucose oder Oligosacchariden mit primären Alkoholen mit 8 - 22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1-2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1 - 2, insbesondere 1,2 -1,4, liegt. Besonders bevorzugte C₈ - C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.
Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.
Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈ - C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintrüsostearat, Decaglycerintristearat, Decaglycerintrioleat und Decaglycerintrihydroxystearat.

Besonders bevorzugte erfindungsgemäße kosmetische Stifte sind dadurch gekennzeichnet, dass der nichtionische ÖI-in-Wasser-Emulgator b) in einer Gesamtmenge von 0,5 - 10 Gew.-%, besonders bevorzugt 1 - 4 Gew.-% und außerordentlich bevorzugt 1,5 - 3 Gew.-%, bezogen auf die Gesamtzusammensetzung, enthalten ist.

### Wasser-in-Öl-Emulgatoren

Die erfindungsgemäßen Stiftzusammensetzungen enthalten weiterhin mindestens einen nichtionischen Wasser-in-ÖI-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder. Der/die Wasser-in-Öl-Emulgator/en trägt/tragen vor allem zum Aufbau der lipophilen Gelphase bei, die die dispergierte Lipid-/Wachs-/Ölphase umgibt, wie auch, wenn auch in geringerem Maße, zum Aufbau der hydrophilen Gelphase, die die wässrige Phase stabilisiert. Als nichtionische Wasser-in-Öl-Emulgatoren eignen sich prinzipiell Emulgatoren mit einem HLB-Wert größer 1,0 und kleiner/gleich 7,0. Einige dieser Emulgatoren sind beispielsweise in Kirk-Othmer, "Encyclopedia of Chemical Technology", 3. Aufl., 1979, Band 8, Seite 913, aufgelistet. Für ethoxylierte Addukte lässt sich der HLB-Wert, wie bereits erwähnt, auch berechnen.

Als Wasser-in-ÖI-Emulgator bevorzugt sind:
- lineare gesättigte Alkanole mit 12- 30 Kohlenstoffatomen, insbesondere mit 16- 22 Kohlenstoffatomen, insbesondere Cetylalkohol, Stearylalkohol, Arachidylalkohol, Behenylalkohol und Lanolinalkohol oder Gemische dieser Alkohole, wie sie bei der technischen Hydrierung von pflanzlichen und tierischen Fettsäuren erhältlich sind,
- Ester und insbesondere Partialester aus einem Polyol mit 2- 6 C-Atomen und linearen gesättigten und ungesättigten Fettsäuren mit 12- 30, insbesondere 14-22 C-Atomen, die hydroxyliert sein können. Solche Ester oder Partialester sind z. B. die Mono- und Diester von Glycerin oder Ethylenglycol oder die Monoester von Propylenglycol mit linearen gesättigten und ungesättigten C₁₂ - C₃₀-Carbonsäuren, die hydroxyliert sein können, insbesondere diejenigen mit Palmitin- und Stearinsäure, die Sorbitanmono-, -di- oder -triester von linearen gesättigten und ungesättigten C₁₂ - C₃₀-Carbonsäuren, die hydroxyliert sein können, insbesondere diejenigen von Myristinsäure, Palmitinsäure, Stearinsäure oder von Mischungen dieser Fettsäuren, die Pentaerythritylmono-, -di-, -tri- und -tetraester und die Methylglucosemono- und -diester von linearen, gesättigten und ungesättigten C₁₂ - C₃₀-Carbonsäuren, die hydroxyliert sein können, wovon besonders bevorzugt sind die Mono-, Di-, Tri- und Tetraester von Pentaerythrit mit linearen gesättigten Fettsäuren mit 12 - 30, insbesondere 14 - 22 Kohlenstoffatomen, die hydroxyliert sein können, sowie Mischungen hiervon, als Konsistenzgeber und/oder Wasserbinder. Erfindungsgemäß besonders bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat und Mischungen hiervon;
- Sterine, also Steroide, die am C3-Atom des Steroid-Gerüstes eine Hydroxylgruppe tragen und sowohl aus tierischem Gewebe (Zoosterine, z. B. Cholesterin, Lanosterin) wie auch aus Pflanzen (Phytosterine, z. B. Ergosterin, Stigmasterin, Sitosterin) und aus Pilzen und Hefen (Mykosterine) isoliert werden und die niedrig ethoxyliert (1-5 EO) sein können;
- Alkanole und Carbonsäuren mit jeweils 8-24 C-Atomen, insbesondere mit 16-22 C-Atomen, in der Alkylgruppe und 1-4 Ethylenoxid-Einheiten pro Molekül, die einen HLB-Wert größer 1,0 und kleiner/gleich 7,0 aufweisen,
- Glycerinmonoether gesättigter und/oder ungesättigter, verzweigter und/oder unverzweigter Alkohole einer Kettenlänge von 8 - 30, insbesondere 12 -18 Kohlenstoffatomen,
- Partialester von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich als 7 aufweisen,
- sowie Mischungen der vorgenannten Substanzen.
   Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina® GMS verstanden, das eine Mischung aus Glycerylmonostearat und Glyceryldistearat darstellt.
   Besonders vorteilhaft einsetzbare Wasser-in-Öl-Emulgatoren sind Stearylalkohol, Cetylalkohol, Glycerylmonostearat, insbesondere in Form der Handelsprodukte Cutina® GMS und Cutina® MD (ex Cognis), Glyceryldistearat, Glycerylmonocaprinat, Glycerylmonocaprylat, Glycerylmonolaurat, Glycerylmonomyristat, Glycerylmonopalmitat, Glycerylmonohydroxystearat, Glycerylmonooleat, Glycerylmonolanolat, Glyceryldimyristat, Glyceryldipalmitat, Glyceryldioleat, Propylenglycolmonostearat, Propylenglycolmonolaurat, Sorbitanmonocaprylat, Sorbitanmonolaurat, Sorbitanmonomyristat, Sorbitanmonopalmitat, Sorbitanmonostearat, Sorbitansesquistearat, Sorbitandistearat, Sorbitandioleat, Sorbitansesquioleat, Saccharosedistearat, Arachidylalkohol, Behenylalkohol, Polyethylenglycol(2)stearylether (Steareth-2), Steareth-5, Oleth-2, Diglycerinmonostearat, Diglycerinmonoisostearat, Diglycerinmonooleat, Diglycerindihydroxystearat, Diglycerindistearat, Diglycerindioleat, Triglycerindistearat, Tetraglycerinmonostearat, Tetraglycerindistearat, Tetraglycerintristearat, Decaglycerinpentastearat, Decaglycerinpentahydroxystearat, Decaglycerinpentaisostearat, Decaglycerinpentaoleat, Soy Sterol, PEG-1 Soy Sterol, PEG-5 Soy Sterol, PEG-2-monolaurat und PEG-2-monostearat.
   Bevorzugte erfindungsgemäße kosmetische Stifte sind dadurch gekennzeichnet, dass der nicht-ionische Wasser-in-Öl-Emulgator c) mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, ausgewählt ist aus linearen, gesättigten C₁₂ - C₃₀-Alkanolen, Ethylenglycolmono- und diestern von linearen, gesättigten und ungesättigten C₁₂ - C₃₀-Carbonsäuren, die hydroxyliert sein können, Glycerinmono- und diestern von linearen, gesättigten und ungesättigten C₁₂- C₃₀-Carbonsäuren, die hydroxyliert sein können, Propylenglycolmono- und - diestern von linearen, gesättigten und ungesättigten C₁₂- C₃₀-Carbonsäuren, die hydroxyliert sein können, Sorbitanmono-, -di- und -triestern von linearen, gesättigten und ungesättigten C₁₂-C₃₀-Carbonsäuren, die hydroxyliert sein können, Pentaerythritylmono-, -di-, -tri- und -tetraestern von linearen, gesättigten und ungesättigten C₁₂- C₃₀-Carbonsäuren, die hydroxyliert sein können, Methylglucosemono- und -diestern von linearen, gesättigten und ungesättigten C₁₂- C₃₀-Carbonsäuren, die hydroxyliert sein können, Sterinen, Alkanolen und Carbonsäuren mit jeweils 8-24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen, Glycerinmonoethern gesättigter und/oder ungesättigter, verzweigter und/oder unverzweigter Alkohole einer Kettenlänge von 8 - 30, insbesondere 12 - 18 Kohlenstoffatomen, Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich als 7 aufweisen, sowie Mischungen der vorgenannten Substanzen.
   Besonders bevorzugte erfindungsgemäße kosmetische Stifte sind dadurch gekennzeichnet, dass der/die nichtionische/n Wasser-in-Öl-Emulgator/en c) mit einem HLB-Wert größer 1,0 und kleiner/gleich 7,0, der/die mit Wasser allein oder mit Wasser in Gegenwart eines hydrophilen Emulgators flüssigkristalline Strukturen ausbilden kann/können, ausgewählt ist aus den Mono-, Di-, Tri- und Tetraestern von Ethylenglycol oder Pentaerythrit mit linearen gesättigten und ungesättigten Fettsäuren mit 12 - 30, insbesondere 14 - 22 Kohlenstoffatomen, die hydroxyliert sein können. Erfindungsgemäß bevorzugt sind die Mono- und Diester. Erfindungsgemäß bevorzugte C₁₂-C₃₀-Fettsäurereste sind ausgewählt aus Laurinsäure-, Myristinsäure-, Palmitinsäure-, Stearinsäure-, Arachinsäure- und Behensäure-Resten; besonders bevorzugt ist der Stearinsäurerest. Erfindungsgemäß besonders bevorzugte nichtionische Wasser-in-ÖI-Emulgatoren mit einem HLB-Wert größer 1,0 und kleiner/gleich 7,0 sind ausgewählt aus Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon. Der/die Wasser-in-ÖI-Emulgator/en trägt/tragen vor allem zum Aufbau der lipophilen Gelphase bei, die die dispergierte Lipid-/Wachs-/Ölphase umgibt, wie auch, wenn auch in geringerem Maße, zum Aufbau der hydrophilen Gelphase, die die wässrige Phase stabilisiert. Die erfindungsgemäß besonders bevorzugten Wasser-in-ÖI-Emulgatoren mit einem HLB-Wert größer 1,0 und kleiner/gleich 7,0 sind zum Beispiel als Handelsprodukte Cutina PES (INCI: Pentaerythrityl distearate), Cutina AGS (INCI: Glycol distearate) oder Cutina EGMS (INCI: Glycol stearate) erhältlich. Diese Handelsprodukte stellen bereits Mischungen aus Mono- und Diestern (bei den Pentaerythritylestern sind auch Tri- und Tetraester enthalten) dar. Erfindungsgemäß kann es bevorzugt sein, nur einen einzigen Wasser-in-Öl-Emulgator einzusetzen. In einer anderen bevorzugten Ausführungsform enthalten die erfindungsgemäßen Zusammensetzungen Mischungen, insbesondere technische Mischungen, von mindestens zwei Wasser-in-Öl-Emulgatoren. Unter einer technischen Mischung wird beispielsweise ein Handelsprodukt wie Cutina® PES verstanden.
   Weitere besonders bevorzugte erfindungsgemäße kosmetische Stifte sind dadurch gekennzeichnet, dass der/die nichtionische/n Wasser-in-Öl-Emulgator/en c) mit einem HLB-Wert größer 1,0 und kleiner/gleich 7,0, der/die mit Wasser allein oder mit Wasser in Gegenwart eines hydrophilen Emulgators flüssigkristalline Strukturen ausbilden kann/ können, in einer Gesamtmenge von 0,1 - 15 Gew.-%, besonders bevorzugt 0,5 - 8 Gew.-% und außerordentlich bevorzugt 1 - 4 Gew.-%, bezogen auf das Gesamtgewicht der Zusammensetzung, enthalten ist/sind. Weiterhin können auch Mengen von 2 - 3 Gew.-%, bezogen auf das Gesamtgewicht der Zusammensetzung, erfindungsgemäß außerordentlich bevorzugt sein.
   In der nachfolgenden Tabelle sind verschiedene Öl-in-Wasser-Emulgatoren und Wasser-in-Öl-Emulgatoren und ihre HLB-Werte zusammengestellt. Die HLB-Werte können aber auch nach Griffin berechnet werden, wie es beispielsweise im RÖMPP Chemie Lexikon, insbesondere in der Online-Version von November 2003, und den dort unter dem Stichwort "HLB-System" zitierten Handbüchern von Fiedler, Kirk-Othmer und Janistyn dargestellt beziehungsweise tabelliert ist. Sofern es in der Literatur unterschiedliche Angaben zum HLB-Wert einer Substanz gibt, sollte derjenige HLB-Wert für die erfindungsgemäße Lehre genutzt werden, der dem nach Griffin berechneten Wert am nächsten kommt. Falls sich auf diese Art und Weise kein eindeutiger HLB-Wert ermitteln lässt, ist der HLB-Wert, den der Hersteller des Emulgators angibt, für die erfindungsgemäße Lehre zu nutzen. Falls auch dies nicht möglich ist, ist der HLB-Wert experimentell zu ermitteln.

### HLB-Wert Chemische Bezeichnung

- 1: Triglyceride gesättigter Fettsäuren
Glyceryltrioleat
- 1,5: Ethylenglycoldistearat
- 1,6: Pur-Cellinöl
- 1,8: Sorbitantrioleat
Glycerindioleat
- 2,1: Sorbitantristearat
- 2,4: Propylenglycollactostearat
- 2,7: Glycerinmonooleat
Sorbitdioleat
- 2,8: Glycerinmonostearat
Propylenglycolmono-/distearat, nicht selbstemulgierend
- 2,9: Ethylenglycolmonostearat
- 3,0: Decaglycerindecaoleat
Decaglycerindecastearat
Generol 122 (Rapeseed Sterols)
Sucrosedistearat
- 3,1: Decaglycerindecaoleat
Glycerylmonoricinoleat
Pentaerythritylmonostearat
Pentaerythritylsesquioleat
- 3,2: Ethylenglycolmonodistearat, nicht selbstemulgierend
Glycolstearat
- 3,3: Glycerinmonolaurat
- 3,4: Propylenglycolmonostearat
- 3,5: Ethylenglycolmonostearat
Pentaerythritylmonooleat
Polyethylenglycol(100)monooleat
- 3,6: Glycerinmono-/dioleat, nicht selbstemulgierend
Monoethoxylaurylether
- 3,7: Sorbitansesquioleat (Dehymuls SSO)
- 3,8: Glycerinmonodistearat, nicht selbstemulgierend
Polyethylenglycol(1 00)monostearat
Diglycerinsesquioleat
N,N-Dimethylcaproamid
Pentaerythritmonotallat
Propylenglycolmonolaurat
- 4,0: Decaglycerinoctaoleat
- 4,3: Sorbitanmonooleat (Dehymuls SMO)
Diethylenglycolmonostearat
- 4,4: 1.2-Propylenglycolmonodistearat, selbstemulgierend
- 4,5: Glycerinmonostearatpalmitat (90 %), nicht selbstemulgierend
Propylenglycolmonolaurat
- 4,7: Sorbitanmonostearat (Dehymuls SMS)
Diethylenglycolmonooleat
- 4,8: Pentaerythritmonolaurat
- 4,9: Polyoxyethylen(2)oleylalkohol (Polyoxyethylen(2)oleylether)
Polyoxyethylen(2)stearylalkohol (Polyoxyethylen(2)stearylether)
- 5,0: Ethylenglycolmonodistearat
Generol 122 E 5 (PEG-5 Soy Sterol)
Polyethylenglycol(100)monoricinoleat
Polyethylenglycol(200)distearat
Polyglyceryl-3-isostearate (z. B. Isolan GI 34 von Tego)
- 5,9: Polyethylenglycol(200)dilaurat
- 6,0: Decaglycerintetraoleat
Polyethylenglycol(100)monolaurat
Polyethylenglycol(200)dioleat
- 6,1: Diethylenglycolmonolaurat (Diglycollaurat)
- 6,3: Polyethylenglycol(300)dilaurat
- 6,4: Glycerinmonoricinoleat
Glycerinsorbitanmonolaurat
- 6,5: Diethylenglycolmonolaurat
Natriumstearoyl-2-lactylat
- 6,7: Sorbitanmonopalmitat
- 6,8: Glycerinmonococoat
Glycerinmonolaurat
- 7,0: Polyoxyethylen(2)C₁₀-C₁₄-fettalkoholether, Laureth-2 (Dehydol LS 2)
Saccharosedistearat
- 7,2: Polyethylenglycol(400)dioleat
Saccharosedioleat
- 7,4: Polyethylenglycol(100)monolaurat
Saccharosedipalmitat
- 7,5: Saccharosedipalmitat
- 7,6: Glycerinsorbitanlaurat
- 7,8: Polyethylenglycol(400)distearat
- 7,9: Polyethylenglycol(200)monostearat
Polyoxyethylen(3)tridecylalkohol
- 8-8,2: Polyethytenglycol(400)distearat
- 8,0: Polyoxyethylen(3)C₁₀-C₁₄-fettalkoholether, Laureth-3 (Dehydol LS 3)
N.N-Dimethyllauramid
Natriumlauroyllactylat, Natriumlauroyl-2-lactylat
Polyethylenglycol(200)monooleat
Polyethylenglycol(220)monotallat
Polyethylenglycol(1500)dioleat
Polyoxyethylen(4)oleylalkohol
Polyoxyethylen(4)stearylcetylether
- 8,2: Triglycerinmonooleat
- 8,3: Diethylenglycolmonölaurat
- 8,4: Polyoxyethylen(4)cetylether
Polyoxyethylenglycol(400)dioleat
- 8,5: Natriumcaproyllactylat
Polyethylenglycol(200)monostearat
Sorbitanmonooleat
- 8,6: Sorbitanmonolaurat (Dehymuls SML)
Polyethylenglycol(200)monolaurat
- 8,8: Polyoxyethylen(4)myristylether
Polyethylenglycol (400)dioleat
- 8,9: Nonylphenol, polyoxyethyliert mit 4 Mol EO
- 9,0: Oleth-5 (z. B. Eumulgin O 5)
- 9,2 - 9,7: Polyoxyethylen(4)laurylalkohol (je nach Handelsprodukt, z. B. Brij 30, Dehydol LS 4)
- 9,3: Polyoxyethylen(4)tridecylalkohol
- 9,6: Polyoxyethylen(4)sorbitanmonostearat
- 9,8: Polyethylenglycol (200)monolaurat
- 10-11: Polyethylenglycol(400)monooleat
- 10,0: Didodecyldimethylammoniumchlorid
- 10,0: Polyethylenglycol(200)monolaurat
Polyethylenglycol(400)dilaurat
Polyethylenglycol(600)dioleat
Polyoxyethylen(4)sorbitanmonostearat
Polyoxyethylen(5)sorbitanmonooleat
- 10,2: Polyoxyethylen(40)sorbitol hexaoleat
- 10,4 - 10,6: Polyoxyethylenglycol(600)distearat
- 10,5: Polyoxyethylen(20)sorbitantristearat
- 10,6: Saccharosemonostearat
- 10,7: Saccharosemonooleat
- 11 - 11,4: Polyethylenglycol(400)monooleat
- 11,0: Polyethylenglycol(350)monostearat
Polyethylenglycol(400)monotallat
Polyoxyethylenglycol(7)monostearat
Polyoxyethylenglycol(8)monooleat
Polyoxyethylen(20)sorbitantrioleat
Polyoxyethylen(6)tridecylalkohol
- 11,1: Polyethylenglycol(400)monostearat
- 11,2: Polyoxyethylen(9)monostearat
Saccharosemonooleat
Saccharosemonostearat
- 11,4: Polyoxyethylen(50)sorbitol hexaoleat
Saccharosemonotallat
Saccharosestearatpalmitat
- 11,6: Polyoxyethylenglycol(400)monoricinoleat
- 11,7: Saccharosemonomyristat
Saccharosemonopalmitat
- 12,0: PEG-10 Soy Sterol (z. B. Generol 122 E 10)
Triethanolaminoleat
- 12,2-12,3: Nonylphenol, ethoxyliert mit 8 Mol EO
- 12,2: Saccharosemonomyristat
- 12,4: Saccharosemonolaurat
Polyoxyethylen(10)oleylalkohol, Polyoxyethylen(10)oleylether
Polyoxyethylen(10)stearylalkohol, Polyoxyethylen(10)stearylether
- 12,5: Polyoxyethylen(10)stearylcetylether
- 12,7: Polyoxyethylen(8)tridecylalkohol
- 12,8: Polyoxyethylenglycol(400)monolaurat
Saccharosemonococoat
- 12,9: Polyoxyethylen(10)cetylether
- 13: Glycerinmonostearat, ethoxyliert (20 Mol EO)
- 13,0: Eumulgin O 10
Eumulgin 286
Eumulgin B 1 (Ceteareth-12)
- 13,0: C12-Fettamine, ethoxyliert (5 Mol EO)
- 13,1: Nonylphenol, ethoxyliert (9,5 Mol EO)
- 13,2: Polyethylenglycol(600)monostearat
Polyoxyethylen(16)tallöl
- 13,3: Polyoxyethylen(4)sorbitanmonolaurat
- 13,5: Nonylphenol, ethoxyliert (10,5 Mol EO)
Polyethylenglycol(600)monooleat
- 13,7: Polyoxyethylen(10)tridecylalkohol
Polyethylenglycol(660)monotallat
Polyethylenglycol(1500)monostearat
Polyoxyethylenglycol(1500)dioleat
- 13,9: Polyethylenglycol(400)monococoat
Polyoxyethylen(9)monolaurat
- 14-16: Eumulgin HRE 40 (Ricinusöl, mit 40 EO ethoxyliert und hydriert)
- 14,0: Polyoxyethylen(12)laurylether
Polyoxyethylen(12)tridecylalkohol
- 14,2: Polyoxyethylen(15)stearylalkohol
- 14,3: Polyoxyethylen(15)stearylcetylether
- 14,4: Gemisch aus C12-C15-Fettalkoholen mit 12 Mol EO
- 14,5: Polyoxyethylen(1 2)laurylalkohol
- 14,8: Polyoxyethylenglycol(600)monolaurat
- 14,9 -15,2: Sorbitanmonostearat, mit 20 EO ethoxyliert (z. B. Eumulgin SMS 20)
- 15 -15,9: Sorbitanmonooleat, mit 20 EO ethoxyliert (z. B. Eumulgin SMO 20)
- 15,0: PEG-20 Glyceryl stearate (z. B. Cutina E 24)
PEG-40 Castor Oil (z. B. Eumulgin RO 40)
Decylglucosid (Oramix NS 10)
Dodecylglucosid (Plantaren APG 600)
Dodecyltrimethylammoniumchlorid
Nonylphenol, ethoxyliert mit 15 Mol EO
Polyethylenglycol(1000)monostearat
Polyoxyethylen(600)monooleat
- 15-17: Eumulgin HRE 60 (Ricinusöl, mit 60 EO ethoxyliert und hydriert)
- 15,3: C12-Fettamine, polyoxyethyliert mit 12 Mol EO
Polyoxyethylen(20)oleylalkohol, Polyoxyethylen(20)oleylether
- 15,4: Polyoxyethylen(20)stearylcetylether (z. B. Eumulgin B 2 (Ceteareth-20))
- 15,5: Polyoxyethylen(20)stearylalkohol
- 15,6: Polyoxyethylenglycol(1000)monostearat
Polyoxyethylen(20)sorbitanmonopalmitat
- 15,7: Polyoxyethylen(20)cetylether
- 15,9: Dinatriumtriethanolamindistearylheptaglycolethersulfosuccinat
- 16,0: Nonylphenol ethoxyliert mit 20 Mol EO
Polyoxyethylen(25)propylenglycolstearat
- 16 - 16,8: Polyoxyethylen(30)monostearat
- 16,3-16,9: Polyoxyethylen(40)monostearat
- 16,5 - 16,7: Polyoxyethylen(20)sorbitanmonolaurat (z. B. Eumulgin SML 20)
- 16,6: Polyoxyethylen(20)sorbit
- 16,7: C18-Fettamine, polyoxyethyliert mit 5 Mol EO
Polyoxyethylen(23)laurylalkohol
- 17,0: Ceteareth-30, z. B. Eumulgin B 3
Octylglucosid (Triton CG 110)
Polyoxyethylen(30)glycerylmonolaurat
- 17,1: Nonylphenol, ethoxyliert mit 30 Mol EO
- 17,4: Polyoxyethylen(40)stearylalkohol

Weitere bevorzugte erfindungsgemäße Stiftzusammensetzungen sind dadurch gekennzeichnet, dass der Gesamtgehalt an nichtionischen und ionischen Emulgatoren und/oder Tensiden mit einem HLB-Wert über 8 maximal 18 Gew.-%, bevorzugt maximal 15 Gew.-%, besonders bevorzugt maximal 10 Gew.-%, besonders bevorzugt maximal 7 Gew.-%, weiterhin besonders bevorzugt maximal 4 Gew.-% und außerordentlich bevorzugt maximal 3 Gew.-%, jeweils bezogen auf die gesamte erfindungsgemäße Zusammensetzung, beträgt.

### Öle

Die erfindungsgemäßen Stiftzusammensetzungen enthalten weiterhin mindestens ein unter Normalbedingungen flüssiges Öl, das keine Duftstoffkomponente und kein etherisches Öl darstellt, wobei der (mittlere) Löslichkeitsparameter der Gesamtheit der enthaltenen Öle in Gegenwart von linearen gesättigten Fettalkoholen mit einer Kettenlänge von mindestens 8 Kohlenstoffatomen um maximal -0,7 (cal/cm³)^{0,5} bzw. maximal +0,7 (cal/cm³)^{0,5}, bevorzugt um maximal -0,6 (cal/cm³)^{0,5} bzw. maximal +0,6 (cal/cm³)^{0,5}, besonders bevorzugt um maximal -0,4 (cal/cm³)^{0,5} bzw. maximal +0,5 (cal/cm³)^{0,5}, und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -0,4 (cal/cm³)^{0,5} bzw. maximal +0,7 (cal/cm³)^{0,5}, bevorzugt um maximal -0,3 (cal/cm³)^{0,5} bzw. maximal +0,6 (cal/cm³)^{0,5}, besonders bevorzugt um maximal -0,2 (cal/cm³)^{0,5} bzw. maximal +0,5 (cal/cm³)^{0,5}, vom (mittleren) Löslichkeitsparameter des Wasser-in-ÖI-Emulgators/der Wasser-in-ÖI-Emulgatoren abweicht. Die Abstimmung des/der verwendeten Öls/Öle auf den/die verwendeten Wasser-in-ÖI-Emulgator/en stellt einen wichtigen Parameter dieser Erfindung dar. Passen der Wasser-in-Öl-Emulgator und die Ölkomponente/n vom Löslichkeitsparameter her nicht innerhalb der beanspruchten Grenzen zusammen, erhält man Stifte mit einer anwendungstechnisch nicht befriedigenden Härte und Stabilität.
Erfindungsgemäß bevorzugte Öle sind ausgewählt aus den Benzoesäureestern von linearen oder verzweigten C₈₋₂₂-Alkanolen. Besonders bevorzugt sind Benzoesäure-C12-C15-alkylester, z. B. erhältlich als Handelsprodukt Finsolv^{®} TN, Benzoesäureisostearylester, z. B. erhältlich als Handelsprodukt Finsolv^{®} SB, Ethylhexylbenzoat, z. B. erhältlich als Handelsprodukt Finsolv^{®} EB, und Benzoesäureoctyldocecylester, z. B. erhältlich als Handelsprodukt Finsolv^{®} BOD.
Erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind Hexyldecanol (Eutanol^{®} G 16, Guerbitol^{®} T 16), Octyldodecanol (Eutanol^{®} G, Guerbitol^{®} 20), 2-Ethylhexylalkohol und die Handelsprodukte Guerbitol^{®} 18, Isofol^{®} 12, Isofol^{®} 16, Isofol^{®} 24, Isofol^{®} 36, Isocarb^{®} 12, Isocarb^{®} 16 oder Isocarb^{®} 24.

Weitere bevorzugte Ölkomponenten sind Mischungen aus Guerbetalkoholen und Guerbetalkoholestern, z.B. das Handelsprodukt Cetiol® PGL (Hexyldecanol und Hexyldecyllaurat).
Weitere erfindungsgemäß bevorzugte Öle sind ausgewählt aus den Triglyceriden von linearen oder verzweigten, gesättigten oder ungesättigten, gegebenenfalls hydroxylierten C₈₋₃₀-Fettsäuren. Besonders geeignet kann die Verwendung natürlicher Öle, z.B. Sojaöl, Baumwollsaatöl, Sonnenblumenöl, Palmöl, Palmkernöl, Leinöl, Mandelöl, Rizinusöl, Maisöl, Olivenöl, Rapsöl, Sesamöl, Distelöl, Weizenkeimöl, Pfirsichkernöl und die flüssigen Anteile des Kokosöls und dergleichen sein. Geeignet sind aber auch synthetische Triglyceridöle, insbesondere Capric/Caprylic Triglycerides, z. B. die Handelsprodukte Myritol^{®} 318, Myritol^{®} 331 (Cognis) oder Miglyol^{®} 812 (Hüls) mit unverzweigten Fettsäureresten sowie Glyceryltrüsostearin und die Handelsprodukte Estol^{®} GTEH 3609 (Uniqema) oder Myritol^{®} GTEH (Cognis) mit verzweigten Fettsäureresten.
Weitere erfindungsgemäß besonders bevorzugte Öle sind ausgewählt aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.
Weitere erfindungsgemäß besonders bevorzugte Öle sind ausgewählt aus den Anlagerungsprodukten von 1 bis 5 Propylenoxid-Einheiten an ein- oder mehrwertige C₈-₂₂-Alkanole wie Octanol, Decanol, Decandiol, Laurylalkohol, Myristylalkohol und Stearylalkohol, z. B. PPG-2-Myristylether und PPG-3-Myristylether (Witconol^{®} APM).
Für den Einsatz der im folgenden aufgezählten Öle in den erfindungsgemäßen Stiftzusammensetzungen ist zu beachten, dass ihr Anteil an der gesamten Ölmischung nur so groß ist, dass der mittlere Löslichkeitsparameter der gesamten Ölmischung, wie erfindungsgemäß beansprucht und vorstehend beschrieben, an den mittleren Löslichkeitsparameter des Wasser-in-Öl-Emulgators angepasst ist. Entsprechende Öle sind ausgewählt aus den Estern der linearen oder verzweigten gesättigten oder ungesättigten Fettalkohole mit 2-30 Kohlenstoffatomen mit linearen oder verzweigten gesättigten oder ungesättigten Fettsäuren mit 2-30 Kohlenstoffatomen, die hydroxyliert sein können. Dazu zählen Hexyldecylstearat (Eutanol^{®} G 16 S), Hexyldecyllaurat, Isodecylneopentanoat, Isononylisononanoat, 2-Ethylhexylpalmitat (Cegesoft^{®} C 24) und 2-Ethylhexylstearat (Cetiol^{®} 868). Ebenfalls bedingt geeignet sind Isopropylmyristat, Isopropylpalmitat, Isopropylstearat, Isopropylisostearat, Isopropyloleat, Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Butylstearat, n-Hexyllaurat, n-Decyloleat, Oleyloleat, Oleylerucat, Erucyloleat, Erucylerucat, Ethylenglycoldioleat und -dipalmitat.
Weitere Öle, die mit Rücksicht auf die Löslichkeitsparameter-Abstimmung nur in geringen Mengen oder gar nicht einsetzbar sind, sind ausgewählt aus den Anlagerungsprodukten von mindestens 6 Ethylenoxid- und/oder Propylenoxid-Einheiten an ein- oder mehrwertige C₃₋₂₂-Alkanole wie Butanol, Butandiol, Myristylalkohol und Stearylalkohol, z. B. PPG-14-Butylether (Ucon Fluid^{®} AP), PPG-9-Butylether (Breox^{®} B25), PPG-10-Butandiol (Macol^{®} 57) und PPG-15-Stearylether (Arlamol^{®} E).
Weitere Öle, die mit Rücksicht auf die Löslichkeitsparameter-Abstimmung nur in geringen Mengen oder gar nicht einsetzbar sind, sind ausgewählt aus den C₈-C₂₂-Fettalkoholestern einwertiger oder mehrwertiger C₂-C₇-Hydroxycarbonsäuren, insbesondere die Ester der Glycolsäure, Milchsäure, Äpfelsäure, Weinsäure, Citronensäure und Salicylsäure. Solche Ester auf Basis von linearen C_{14/15}-Alkanolen, z. B. C₁₂-C₁₅-Alkyllactat, und von in 2-Position verzweigten C_{12/13}-Alkanolen sind unter dem Warenzeichen Cosmacol^{®} von der Firma Nordmann, Rassmann GmbH & Co, Hamburg, zu beziehen, insbesondere die Handelsprodukte Cosmacol^{®} ESI, Cosmacol^{®} EMI und Cosmacol^{®} ETI.
Weitere Öle, die mit Rücksicht auf die Löslichkeitsparameter-Abstimmung nur in geringen Mengen oder gar nicht einsetzbar sind, sind ausgewählt aus den symmetrischen, unsymmetrischen oder cyclischen Estern der Kohlensäure mit Fettalkoholen, z. B. Glycerincarbonat, Dicaprylylcarbonat (Cetiol^{®} CC) oder die Ester der DE 197 56 454 A1.
Weitere Öle, die mit Rücksicht auf die Löslichkeitsparameter-Abstimmung nur in geringen Mengen oder gar nicht einsetzbar sind, sind ausgewählt aus den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen.
Es kann erfindungsgemäß bevorzugt sein, Mischungen der vorgenannten Öle einzusetzen.
Bevorzugte erfindungsgemäße kosmetische Stifte sind dadurch gekennzeichnet, dass das unter Normalbedingungen flüssige ÖI d) ausgewählt ist aus verzweigten gesättigten oder ungesättigten Fettalkoholen mit 6 - 30 Kohlenstoffatomen, Triglyceriden von linearen oder verzweigten, gesättigten oder ungesättigten, gegebenenfalls hydroxylierten C₈₋₃₀-Fettsäuren, Dicarbonsäureestern von linearen oder verzweigten C₂-C₁₀-Alkanolen, Estern von verzweigten gesättigten oder ungesättigten Fettalkoholen mit 2 - 30 Kohlenstoffatomen mit linearen oder verzweigten gesättigten oder ungesättigten Fettsäuren mit 2 - 30 Kohlenstoffatomen, die hydroxyliert sein können, Anlagerungsprodukten von 1 bis 5 Propylenoxid-Einheiten an ein- oder mehrwertige C₈₋₂₂-Alkanole, Anlagerungsprodukten von mindestens 6 Ethylenoxid und/oder Propylenoxid-Einheiten an ein- oder mehrwertige C₃₋₂₂-Alkanole, C₈-C₂₂-Fettalkoholestern einwertiger oder mehrwertiger C₂-C₇-Hydroxycarbonsäuren, symmetrischen, unsymmetrischen oder cyclischen Estern der Kohlensäure mit Fettalkoholen, den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen, sowie Mischungen der vorgenannten Substanzen.

Besonders bevorzugte erfindungsgemäße kosmetische Stifte sind dadurch gekennzeichnet, dass das/die unter Normalbedingungen flüssige/n ÖI/e d) in einer Gesamtmenge von 3-20 Gew.-%, bevorzugt 5-14, besonders bevorzugt 6-12 Gew.-%, bezogen auf das Gesamtgewicht der Zusammensetzung, enthalten ist/sind.

In einer weiteren besonders bevorzugten Ausführungsform der Erfindung beträgt der Anteil an Öl/en, deren Löslichkeitsparameter um mehr als -0,4 beziehungsweise -0,7 (cal/cm³)^{0,5} oder um mehr als +0,7 (cal/cm³)^{0,5} vom mittleren Löslichkeitsparameter der verwendeten Wasser-in-Öl-Emulgatoren abweicht, maximal 20 Gew.-%, bezogen auf das Gesamtgewicht an unter Normalbedingungen flüssigen Ölen. In einer weiteren besonders bevorzugten Ausführungsform der Erfindung sind keine unter Normalbedingungen flüssigen Öle enthalten, deren Löslichkeitsparameter um mehr als ± 1,0 (cal/cm³)^{0,5} vom (mittleren) Löslichkeitsparameter des Wasser-in-Öl-Emulgators/der Wasser-in-Öl-Emulgatoren c) abweicht.
Entsprechende weniger geeignete oder (je nach verwendetem Wasser-in-Öl-Emulgator) sogar ungeeignete Ölkomponenten sind zum Beispiel Siliconöle und Kohlenwasserstofföle.
Siliconöle, zu denen z. B. Dialkyl- und Alkylarylsiloxane, wie beispielsweise Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen, weisen Löslichkeitsparameter im Bereich von etwa 5,7 bis 6,3 (cal/cm³)^{0,5} auf, was um mehr als 0,4 (cal/cm³)^{0,5} vom Wert der meisten der erfindungsgemäß verwendeten Wasser-in-Öl-Emulgatoren abweicht.
Natürliche und synthetische Kohlenwasserstoffe wie beispielsweise Paraffinöle, Isoparaffine, insbesondere Isohexadecan, Isoeicosan, Polyisobutene oder Polydecene, die beispielsweise unter der Bezeichnung Emery^{®} 3004, 3006, 3010 oder unter der Bezeichnung Ethylflo^{®} von Albemarle oder Nexbase^{®} 2004G von Nestle erhältlich sind, sowie 1,3-Di-(2-ethylhexyl)-cyclohexan (Cetiol^{®}S) gehören ebenfalls zu den erfindungsgemäß weniger bevorzugten Ölkomponenten.
Der Anteil der Siliconöle und/oder Kohlenwasserstoffe sollte daher in einer bevorzugten Ausführungsform der Erfindung nicht größer sein als 20 %, bezogen auf das Gesamtgewicht an unter Normalbedingungen flüssigen Ölen, ansonsten erreichen die erfindungsgemäßen Stifte nicht die anwendungstechnisch gewünschte Härte und Stabilität. In einer besonders bevorzugten Ausführungsform der Erfindung sind keine Siliconöle und/oder Kohlenwasserstoffe, insbesondere keine Paraffin- oder Isoparaffin-Kohlenwasserstoffe, enthalten.

Erfindungsgemäß besonders bevorzugte Stiftzusammensetzungen sind dadurch gekennzeichnet, dass sie frei sind von öllöslichen oder öldispergierbaren ölphasenverdickenden Komponenten, ausgewählt aus den Amiden der 12-Hydroxystearinsäure, Estern und Amiden von N-Acylaminosäuren, Estern und Amiden von Di- und Tricarbonsäuren, Sterolen, Sterolestern, wie insbesondere Oryzanol, Cellobiosefettsäureestern, Zuckerestern, wie insbesondere acylierter Maltose, und nicht-vernetzten öllöslichen oder öldispergierbaren polymeren Olphasen-Verdickungsmitteln, wie insbesondere Styren-Blockcopolymeren mit Ethylen, Propylen und/oder Butylen sowie mit Mischungen dieser Alkene.

### Polyole

Die erfindungsgemäßen Stiftzusammensetzungen enthalten weiterhin mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten sowie Mischungen hiervon. Bevorzugt sind diese Komponenten ausgewählt aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen. Geeignete wasserlösliche Polyethylenglycole sind ausgewählt aus PEG-3, PEG-4, PEG-6, PEG-7, PEG-8, PEG-9, PEG-10, PEG-12, PEG-14, PEG-16, PEG-18 und PEG-20 sowie Mischungen hiervon, wobei PEG-3 bis PEG-8 bevorzugt sind. Auch Zucker und bestimmte Zuckerderivate wie Fructose, Glucose, Maltose, Maltitol, Mannit, Inosit, Sucrose, Trehalose und Xylose sind erfindungsgemäß geeignet.
Bevorzugte erfindungsgemäße kosmetische Stifte sind dadurch gekennzeichnet, dass das mindestens eine wasserlösliche mehrwertige C₂- C₉-Alkanol mit 2- 6 Hydroxylgruppen und/oder mindestens eine wasserlösliche Polyethylenglycol mit 3-20 Ethylenoxid-Einheiten ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

Besonders bevorzugte erfindungsgemäße kosmetische Stifte sind dadurch gekennzeichnet, dass das mindestens eine wasserlösliche mehrwertige C₂ - C₉-Alkanol mit 2 - 6 Hydroxylgruppen und/oder mindestens eine wasserlösliche Polyethylenglycol mit 3 - 20 Ethylenoxid-Einheiten insgesamt in Mengen von 3 - 25 Gew.-%, bevorzugt 8 - 18 Gew.-%, bezogen auf die Gesamtzusammensetzung, enthalten ist.

### Wasser

Der Anteil des Wassers an der erfindungsgemäßen Zusammensetzung beträgt 5 bis weniger als 50 Gew.-%, bevorzugt 10-45 Gew.-%, besonders bevorzugt 20 - 40 Gew.-%, außerordentlich bevorzugt 30 - 38 Gew.-%, jeweils bezogen auf die Gesamtzusammensetzung.

Weitere besonders bevorzugte erfindungsgemäße kosmetische Stifte sind dadurch gekennzeichnet, dass der Gesamtgehalt an wasserlöslichen mehrwertigen C₂ - C₉-Alkanolen mit 2 - 6 Hydroxylgruppen und wasserlöslichen Polyethylenglycolen mit 3 - 20 Ethylenoxid-Einheiten und Wasser insgesamt 8 - 70 Gew.-%, bevorzugt 15 - 65 Gew.-%, besonders bevorzugt 18 - 60 Gew.-% und außerordentlich bevorzugt 25 - 30 Gew.-%, weiterhin bevorzugt 35 - 40 Gew.-%, weiterhin bevorzugt 45 - 55 Gew.-%, und außerordentlich bevorzugt 46 - 50 Gew.-%, jeweils bezogen auf die Gesamtzusammensetzung, beträgt.

Besonders bevorzugte erfindungsgemäße Stiftzusammensetzungen sind dadurch gekennzeichnet, dass
a) die mindestens eine Lipid- oder Wachskomponente mit einem Schmelzpunkt > 50°C, die nicht den Komponenten b) oder c) zuzurechnen ist, ausgewählt ist aus i) gehärtetem Rizinusöl, ii) Cetearylbehenat oder C₂₀₋₄₀-Alkylstearaten, iii) Myristinsäure, Palmitinsäure, Stearinsäure und/oder Behensäure sowie Mischungen von i), ii) und iii),
b) die nichtionischen Öl-in-Wasser-Emulgatoren mit einem HLB-Wert von mehr als 7 innerhalb eines Öl-in-Wasser-Emulgatorgemisches mit einem mittleren HLB-Wert im Bereich von 10 - 19 ausgewählt sind aus Mischungen von mit 15 - 40 Ethylenoxid-Einheiten veretherten C₁₄ - C₂₀-Alkoholen, insbesondere Myristeth-20, Myristeth-30, Ceteth-20, Steareth-20, Ceteth-30, Steareth-30, Ceteareth-20 und Ceteareth-30, und
c) der mindestens eine nichtionischen Wasser-in-Öl-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder, ausgewählt ist aus den Mono-und Diestern von Ethylenglycol oder den Mono-, Di-, Tri- und Tetraestern von Pentaerythrit mit linearen gesättigten und ungesättigten Fettsäuren mit 12 - 30, insbesondere 14 - 22 Kohlenstoffatomen, die hydroxyliert sein können, insbesondere Pentaerythritylmonostearat, Pentaerythrityldistearat, Pentaerythrityltristearat, Pentaerythrityltetrastearat, Ethylenglycolmonostearat, Ethylenglycoldistearat sowie Mischungen hiervon, und
d) das mindestens eine unter Normalbedingungen flüssige ÖI, das keine Duftstoffkomponente und kein etherisches ÖI darstellt, wobei der (mittlere) Löslichkeitsparameter der Gesamtheit der enthaltenen Öle d) in Gegenwart von linearen gesättigten Fettalkoholen mit mindestens 8 Kohlenstoffatomen um maximal -0,7 (cal/cm³)^{0,5} bzw. maximal +0,7 (cal/cm³)^{0,5} und in Gegenwart von Wasser-in-Öl-Emulgatoren, die von linearen gesättigten Fettalkoholen mit mindestens 8 Kohlenstoffatomen verschieden sind, in Abwesenheit von linearen gesättigten Fettalkoholen mit mindestens 8 Kohlenstoffatomen um maximal -0,4 (cal/cm³)^{0,5} bzw. maximal +0,7 (cal/cm³)^{0,5} vom (mittleren) Löslichkeitsparameter des Wasser-in-Öl-Emulgators/ der Wasser-in-Öl-Emulgatoren c) abweicht, ausgewählt ist aus den Dicarbonsäureestern von linearen oder verzweigten C₂-C₁₀-Alkanolen, insbesondere Diisopropyladipat, Di-n-butyladipat, Di-(2-ethylhexyl)adipat, Dioctyladipat, Diethyl-/Di-n-butyl/Dioctylsebacat, Diisopropylsebacat, Dioctylmalat, Dioctylmaleat, Dicaprylylmaleat, Diisooctylsuccinat, Di-2-ethylhexylsuccinat und Di-(2-hexyldecyl)-succinat.

Die erfindungsgemäßen Stiftzusammensetzungen enthalten weiterhin mindestens einen kosmetischen Wirkstoff, ausgewählt aus Monomeren, Oligomeren und Polymeren von Aminosäuren, N-C₂-C₂₄-Acylaminosäuren, den Estern und/oder den physiologisch verträglichen Metallsalzen dieser Substanzen, DNA- oder RNA-Oligonucleotiden, feuchtigkeitsspendenden Wirkstoffen, Vitaminen, Provitaminen und Vitaminvorstufen der Gruppen A, B, C, E und H und den Estern der vorgenannten Substanzen, α-Hydroxycarbonsäuren, α-Ketocarbonsäuren, β-Hydroxycarbonsäuren und deren Ester-, Lacton- oder Salzform, Flavonoiden und Flavonoid-reichen Pflanzenextrakten, Isoflavonoiden und Isofiavonoid-reichen Pflanzenextrakten, Polyphenolen und Polyphenol-reichen Pflanzenextrakten, Ubichinon und Ubichinol sowie deren Derivaten, Silymarin, Purin und Purin-Derivaten, insbesondere den natürlich vorkommenden Xanthin-Derivaten, ausgewählt aus Coffein, Theophyllin, Theobromin und Aminophyllin, Aporphin-Alkaloiden, Ectoin, anorganischen und organischen UV-Filtersubstanzen, selbstbräunenden Wirkstoffen, hautaufhellenden Wirkstoffen, hautberuhigenden Wirkstoffen, sebumregulierenden Wirkstoffen, hyperämisierenden Wirkstoffen, antimikrobiellen Wirkstoffen, präbiotischen Wirkstoffen sowie färbenden, farbgebenden, mattierenden oder glanzgebenden Pigmenten.

Die Monomere der Aminosäuren und/oder der N-C₂-C₂₄-Acylaminosäuren sind ausgewählt aus Alanin, Arginin, Asparagin, Asparaginsäure, Canavanin, Citrullin, Cystein, Cystin, Desmosin, Dipalmitoylhydroxyprolin, Glutamin, Glutaminsäure, Glycin, Histidin, Homophenylalanin, Hydroxylysin, Hydroxyprolin, Isodesmosin, Isoleucin, Leucin, Lysin, Methionin, Methylnorleucin, Ornithin, Phenylalanin, Prolin, Pyroglutaminsäure, Sarcosin, Serin, Taurin, Threonin, Thyroxin, Tryptophan, Tyrosin, Valin, N-Acetyl-L-cystein, Zinkpyroglutamat, Natriumoctanoylglutamat, Natriumdecanoylglutamat, Natriumlauroylglutamat, Natriummyristoylglutamat, Natriumcetoylglutamat und Natriumstearoylglutamat. Besonders bevorzugt sind Lysin, Serin, N-Acetyl-L-cystein, Zink- und Natriumpyroglutamat und Natriumlauroylglutamat.
Der C₂ - C₂₄-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Elaidoyl-, Arachidoyl- oder Behenoyl-Rest. Mischungen von C₈-C₁₈-Acylresten werden auch als Cocoyl-Rest bezeichnet und sind ebenfalls bevorzugte Substituenten.
Mit den vorgenannten C₂ - C₂₄-Acylresten können die Aminosäuren, die eine OH-Gruppe tragen, auch an dieser OH-Gruppe verestert sein. Ein erfindungsgemäß bevorzugtes Beispiel hierfür ist Hydroxyprolin, das mit zwei, bevorzugt linearen, C₂-C₂₂-Fettsäureresten N-acyliert und verestert ist, besonders bevorzugt Dipalmitoylhydroxyprolin, das z. B. unter der Bezeichnung Sepilift DPHP von der Firma Seppic erhältlich ist.
Die physiologisch verträglichen Salze der erfindungsgemäß bevorzugten Wirkstoffe, die Säuregruppen enthalten und Salze bilden können, sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Unter Aminosäureoligomeren werden erfindungsgemäß Peptide mit 2 - 30, bevorzugt 2 - 15, Aminosäuren, verstanden. Die Oligomere der Aminosäuren und/oder der N-C₂-C₂₄-Acylaminosäuren sind bevorzugt ausgewählt aus Di-, Tri-, Tetra-, Penta-, Hexa- oder Pentadecapeptiden, die N-acyliert und/oder verestert sein können. Zahlreiche dieser Aminosäureoligomere stimulieren die Collagensynthese beziehungsweise sind in der Lage, Zellen des Immunsystems, wie Mastzellen und Makrophagen, zu rekrutieren, die dann über die Freisetzung von Wachstumsfaktoren Reparaturprozesse im Gewebe, z.B. die Collagensynthese, induzieren, beziehungsweise sind in der Lage, an die Sequenz Arg-Phe-Lys in Thrombospondin I (TSP-1) zu binden und damit aktives TGF-β *(tissue growth factor),* der die Synthese von Collagen in dermalen Fibroblasten induziert, freizusetzen. Derartige Aminosäureoligomere können als Wirkstoffe gegen die Hautalterung verwendet werden.
Erfindungsgemäß bevorzugte, gegebenenfalls N-acylierte und/oder veresterte Dipeptide sind Acetyl-Citrullyl-Arginin (z. B. Exsy-Algine von Exsymol mit der INCI-Bezeichnung Acetyl Citrull Amido Arginine), Tyr-Arg (Dipeptide-1), Val-Trp (Dipeptide-2), Asn-Phe, Asp-Phe, N-Palmitoyl-β-Ala-His, N-Acetyl-Tyr-Arg-hexyldecylester (z. B. Calmosensine von Sederma), Carnosin (β-Ala-His) und N-Palmitoyl-Pro-Arg. Erfindungsgemäß bevorzugte, gegebenenfalls N-acylierte und/oder veresterte Tripeptide sind Gly-His-Lys, das z. B. unter der Bezeichnung "Omega-CH-Aktivator" von der Firma GfN oder in acylierter Form (N-Palmitoyl-Gly-His-Lys) unter der Bezeichnung Biopeptide CL von Sederma erhältlich ist, aber (in acylierter Form) auch einen Bestandteil des Produktes Matrixyl 3000 von Sederma darstellt. Das Tripeptid Gly-His-Lys kann auch als Kupfersalz (Cu²⁺) eingesetzt werden und ist als solches über ProCyte Corporation zu beziehen. Weiterhin können Analoga von Gly-His-Lys eingesetzt werden, wobei maximal zwei Aminosäuren durch geeignete andere Aminosäuren substituiert sind. Zur Substitution von Gly sind erfindungsgemäß Ala, Leu und Ile geeignet. Die erfindungsgemäß bevorzugten Aminosäuren, die His oder Lys ersetzen können, beinhalten eine Seitenkette mit einem Stickstoffatom, das bei pH 6 überwiegend geladen vorliegt, z. B. Pro, Lys, Arg, His, Desmosin und Isodesmosin. Besonders bevorzugt wird Lys durch Arg, Orn, oder Citrullin ersetzt. Ein weiteres erfindungsgemäß bevorzugtes Tripeptid ist Gly-His-Arg (INCI-Bezeichnung: Tripeptide-3) sowie dessen Derivat N-Myristoyl-Gly-His-Arg, das z. B. unter der Bezeichnung Collasyn 314-GR von Therapeutic Peptide Inc. erhältlich ist; weitere erfindungsgemäß bevorzugte Tripeptide sind ausgewählt aus Lys-Val-Lys, Lys-Val-Dab (Dab = Diaminobuttersäure), Lys-Phe-Lys, Lys-Ile-Lys, Dab-Val-Lys, Lys-Val-Orn, Lys-Val-Dap (Dap = Diaminopropionsäure), Dap-Val-Lys, Palmitoyl-Lys-Val-Lys, z. B. erhältlich von der Firma Pentapharm unter der Bezeichnung SYN^{®}-COLL, Lys-Pro-Val, Tyr-Tyr-Val, Tyr-Val-Tyr, Val-Tyr-Val (Tripeptide-2), Tripeptide-4 (z. B. ATPeptide, zu beziehen über IMPAG), His-Ala-Orn N-Elaidoyl-Lys-Phe-Lys und N-Acetyl-Arg-Lys-Arg-NH₂.

Erfindungsgemäß bevorzugte, gegebenenfalls N-acylierte und/oder veresterte Tetrapeptide sind ausgewählt aus Rigin und Rigin-basierten Tetrapeptiden sowie ALAMCAT-Tetrapeptiden. Rigin weist die Sequenz Gly-Gln-Pro-Arg auf. Rigin-basierte Tetrapeptide umfassen die Rigin-Analoga und Rigin-Derivate, insbesondere das erfindungsgemäß besonders bevorzugte N-Palmitoyl-Gly-Gln-Pro-Arg, das z. B. unter der Bezeichnung Eyeliss von Sederma erhältlich ist, aber auch einen Bestandteil des Produktes Matrixyl 3000 von Sederma darstellt. Zu den Rigin-Analoga zählen solche, bei denen die vier Aminosäuren umarrangiert sind und/oder bei denen gegenüber Rigin maximal zwei Aminosäuren substituiert sind, z. B. die Sequenz Ala-Gln-Thr-Arg. Bevorzugt hat mindestens eine der Aminosäuren der Sequenz ein Pro oder Arg und besonders bevorzugt beinhaltet das Tetrapeptid sowohl Pro als auch Arg, wobei ihre Reihenfolge und Position variieren können. Die substituierenden Aminosäuren können aus jeder Aminosäure, die im folgenden definiert ist, ausgewählt werden. Besonders bevorzugte Rigin-basierte Tetrapetide umfassen: Xaa-Xbb-Arg-Xcc, Xaa-Xbb-Xcc-Pro, Xaa-Xbb-Pro-Arg, Xaa-Xbb-Pro-Xcc, Xaa-Xbb-Xcc-Arg, wobei Xaa, Xbb und Xcc gleiche oder voneinander verschiedene Aminosäuren sein können und wobei Xaa ausgewählt ist aus Gly und den Aminosäuren, die Gly substituieren können, Xbb ausgewählt ist aus GIn und den Aminosäuren, die GIn substituieren können, Xcc ausgewählt ist aus Pro oder Arg und den Aminosäuren, die Pro und Arg substituieren können.
Die bevorzugten Aminosäuren, die Gly ersetzen können, beinhalten eine aliphatische Seitenkette, z. B. β-Ala, Ala, Val, Leu, Pro, Sarcosin (Sar) und Isoleucin (Ile).
Die bevorzugten Aminosäuren, die Gln ersetzen können, beinhalten eine Seitenkette mit einer Aminogruppe, die bei neutralem pH (pH 6-7) überwiegend ungeladen vorliegt, z.B. Asn, Lys, Orn, 5-Hydroxyprolin, Citrullin und Canavanin.
Die bevorzugten Aminosäuren, die Arg ersetzen können, beinhalten eine Seitenkette mit einem Stickstoffatom, das bei pH 6 überwiegend geladen vorliegt, z.B. Pro, Lys, His, Desmosin und Isodesmosin.
Als Rigin-Analoga sind erfindungsgemäß Gly-Gln-Arg-Pro und Val-Val-Arg-Pro bevorzugt.
ALAMCAT-Tetrapeptide sind Tetrapeptide, die mindestens eine Aminosäure mit einer aliphatischen Seitenkette enthalten, z. B. β-Ala, Ala, Val, Leu, Pro, Sarcosin (Sar) und Isoleucin (Ile). Weiterhin beinhalten ALAMCAT-Tetrapeptide mindestens eine Aminosäure mit einer Seitenkette mit einer Aminogruppe, die bei neutralem pH (pH 6-7) überwiegend ungeladen vorliegt, z.B. Gln, Asn, Lys, Orn, 5-Hydroxyprolin, Citrullin und Canavanin. Weiterhin beinhalten ALAMCAT-Tetrapeptide mindestens eine Aminosäure mit einer Seitenkette mit einem Stickstoffatom, das bei pH 6 überwiegend geladen vorliegt, z. B. Arg, Pro, Lys, His, Desmosin und Isodesmosin. Als vierte Aminosäure können ALAMCAT-Tetrapeptide jede beliebige Aminosäure enthalten; bevorzugt ist jedoch auch die vierte Aminosäure aus den drei vorstehend genannten Gruppen ausgewählt.
Erfindungsgemäß bevorzugte, gegebenenfalls N-acylierte und/oder veresterte Pentapeptide sind ausgewählt aus Lys-Thr-Thr-Lys-Ser und seinen N-acylierten Derivaten, besonders bevorzugt N-Palmitoyl-Lys-Thr-Thr-Lys-Ser, das unter der Bezeichnung Matrixyl von der Firma Sederma erhältlich ist, weiterhin N-Palmitoyl-Tyr-Gly-Gly-Phe-Met, Val-Val-Arg-Pro-Pro, N-Palmitoyl-Tyr-Gly-Gly-Phe-Leu, Gly-Pro-Phe-Pro-Leu und N-Benzyloxycarbonyl-Gly-Pro-Phe-Pro-Leu (die beiden letztgenannten stellen Serinproteinase-Inhibitoren zur Inhibition der Desquamation dar). Erfindungsgemäß bevorzugte, gegebenenfalls N-acylierte und/oder veresterte Hexapeptide sind Val-Gly-Val-Ala-Pro-Gly und seine N-acylierten Derivate, besonders bevorzugt N-Palmitoyl-Val-Gly-Val-Ala-Pro-Gly, das unter der Bezeichnung Biopeptide EL von der Firma Sederma erhältlich ist, weiterhin Acetyl-Hexapeptide-3 (Argireline von Lipotec), Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience) und Hexapeptide-10 (z. B. Collaxyl von Vincience oder Seriseline von Lipotec), Ala-Arg-His-Leu-Phe-Trp (Hexapeptide-1), Acetyl Hexapeptide-1 (z. B. Modulene von Vincience), Acetyl Glutamyl Hexapeptide-1 (z. B. SNAP-7 von Centerchem), Hexapeptide-2 (z. B. Melanostatine-DM von Vincience), Ala-Asp-Leu-Lys-Pro-Thr (Hexapeptide-3, z. B. Peptide 02 von Vincience), Val-Val-Arg-Pro-Pro-Pro, Hexapeptide-4 (z. B. Collasyn 6KS von Therapeutic Peptide Inc. (TPI)), Hexapeptide-5 (z. B. Collasyn 6VY von TPI), Myristoyl Hexapeptide-5 (z. B. Collasyn 614VY von TPI), Myristoyl Hexapeptide-6 (z. B. Collasyn 614VG von TPI), Ala-Arg-His-Methylnorleucin-Homophenylalanin-Trp (Hexapeptide-7), Hexapeptide-8 (z. B. Collasyn 6KS von TPI), Myristoyl Hexapeptide-8 (z. B. Collasyn Lipo-6KS von TPI), Hexapeptide-9 (z. B. Collaxyl von Vincience), Hexapeptide-10 (z. B. Collaxyl von Vincience oder Seriseline von Lipotec) und Hexapeptide-11 (z. B. Peptamide-6 von Arch Personal Care). Ein erfindungsgemäß bevorzugtes Pentadecapeptid ist z. B. der Rohstoff Vinci 01 von Vincience (Pentadecapeptide-1). Ein weiteres bevorzugtes Aminosäureoligomer ist das Peptidderivat L-Glutamylaminoethyl-indol (Glistin von Exsymol).
Erfindungsgemäß besonders bevorzugt ist die Kombination aus N-Palmitoyl-Gly-His-Lys und N-Palmitoyl-Gly-Gln-Pro-Arg, wie sie beispielsweise in dem Rohstoff Matrixyl 3000 von der Firma Sederma erhältlich ist.

Die Polymere der Aminosäuren und/oder der N-C₂-C₂₄-Acylaminosäuren sind bevorzugt ausgewählt aus pflanzlichen und tierischen Proteinhydrolysaten und/oder Proteinen mit mehr als 30 Aminosäureeinheiten. Tierische Proteinhydrolysate sind z. B. Elastin-, Collagen-, Keratin-, Seiden-, Conchiolin- und Milcheiweiß-Proteinhydrolysate, die auch in Form von Salzen vorliegen können. Erfindungsgemäß bevorzugt sind pflanzliche Proteinhydrolysate, z. B. Soja-, Weizen-, Mandel-, Erbsen-, Kartoffel- und Reisproteinhydrolysate. Entsprechende Handelsprodukte sind z. B. DiaMin^{®} (Diamalt), Gluadin^{®} (Cognis), Lexein^{®} (Inolex) und Crotein^{®} (Croda). Besonders bevorzugt sind Sojaproteinhydrolysate, besonders bevorzugt Sojaproteinhydrolysate mit einem mittleren Molekulargewicht im Bereich von 1200 - 1800 Dalton, bevorzugt im Bereich von 1400 - 1700 Dalton, z. B. unter dem Handelsnamen Ridulisse C^{®} von der Firma Silab erhältlich, und Sojaproteinhydrolysate mit einem mittleren Molekulargewicht im Bereich von 600 - 1000 Dalton, bevorzugt 800 Dalton, z. B. unter dem Handelsnamen Phytokine^{®} von Coletica erhältlich. mit Kokosfettsäuren N-acylierten und/oder veresterten Sojaproteinhydrolysaten in Form ihrer Alkalimetallsalze. Kokosfettsäuren umfassen überwiegend Alkancarbonsäuren mit einer Anzahl an Kohlenstoffatomen von 8 - 18, insbesondere Caprylsäure, Caprinsäure, Laurinsäure, Myristinsäure, Palmitinsäure und Stearinsäure. Bevorzugte Alkalimetallsalze sind ausgewählt aus Lithium-, Natrium- und Kaliumsalzen, wobei die Kaliumsalze besonders bevorzugt sind.
Ein weiteres, erfindungsgemäß besonders bevorzugtes Sojaproteinhydrolysat ist ein mit Kokosfettsäuren N-acyliertes und/oder verestertes Sojaproteinhydrolysat in Form des Kaliumsalzes, das unter der Handelsbezeichnung Coccopolipeptide di Soja von der Firma Sinerga erhältlich ist.
Weiterhin erfindungsgemäß bevorzugt sind Keratinhydrolysate, insbesondere Wollkeratinhydrolysate. Ein besonders bevorzugtes Wollkeratinhydrolysat ist unter der Bezeichnung Keratec Pep von der Firma Croda erhältlich. Keratec Pep weist eine kleinere Molekulargewichtsfraktion mit einem durchschnittlichen Molekulargewicht von 150 Dalton und eine größere Molekulargewichtsfraktion mit einem durchschnittlichen Molekulargewicht von 1265 Dalton auf. Weiterhin erfindungsgemäß bevorzugt sind Conchiolinhydrolysate, insbesondere solche, die unter den Bezeichnungen Pearl Protein Extract und Pearl Protein Extract BG von der Firma Maruzen erhältlich sind. Conchiolin ist ein komplexes Protein, das aus dem äußeren Epithelium von Mollusken, insbesondere von Perlmuscheln und diversen Schneckenarten, erzeugt wird und das durch Einlagerung von Calciumcarbonat-Kristallen die sehr stabile Schale dieser Mollusken bildet.
Proteinhydrolysate können naturgemäß auch monomere Aminosäuren und Oligopeptide enthalten; ihre Zusammensetzung ist normalerweise nicht definiert.
Ebenfalls bevorzugt ist der Einsatz von Acylderivaten der Proteinhydrolysate, z. B. in Form ihrer Fettsäure-Kondensationsprodukte. Entsprechende Handelsprodukte sind z. B. Lamepon^{®} (Cognis), Gluadin^{®} (Cognis), Lexein^{®} (Inolex), Crolastin^{®} oder Crotein^{®} (Croda).

Erfindungsgemäß bevorzugt sind auch kationisierte Proteinhydrolysate. Besondes bevorzugt sind kationische Proteinhydrolysate, deren zugrunde liegender Proteinanteil ein Molekulargewicht von 100 bis zu 25000 Dalton, bevorzugt 250 bis 5000 Dalton aufweist. Weiterhin sind unter kationischen Proteinhydrolysaten quaternierte Aminosäuren und deren Gemische zu verstehen. Weiterhin können die kationischen Proteinhydrolysate auch noch weiter derivatisiert sein. Als typische Beispiele für erfindungsgemäß verwendete kationische Proteinhydrolysate und -derivate sind einige der unter den INCI- Bezeichnungen im "International Cosmetic Ingredient Dictionary and Handbook", (seventh edition 1997, The Cosmetic, Toiletry, and Fragrance Association 1101 17^{th} Street, N.W., Suite 300, Washington, DC 20036-4702) genannten und im Handel erhältlichen Produkte aufgeführt: Cocodimonium Hydroxypropyl Hydrolyzed Collagen, Steardimonium Hydroxypropyl Hydrolyzed Collagen, Cocodimonium Hydroxypropyl Hydrolyzed Rice Protein, Cocodimonium Hydroxypropyl Hydrolyzed Silk, Cocodimonium Hydroxypropyl Hydrolyzed Soy Protein, Cocodimonium Hydroxypropyl Hydrolyzed Wheat Protein, Cocodimonium Hydroxypropyl Silk Amino Acids, Hydroxypropyl Arginine Lauryl/Myristyl Ether HCl. Ganz besonders bevorzugt sind die kationischen Proteinhydrolysate und -derivate auf pflanzlicher Basis.

In einer weiteren bevorzugten Ausführungsform sind die in den erfindungsgemäßen Stiftzusammensetzungen enthaltenen Polymere der Aminosäuren ausgewählt aus DNA-Reparaturenzymen. Erfindungsgemäß bevorzugte DNA-Reparaturenzyme sind Photolyase und T4 Endonuclease V, letztere im weiteren mit "T4N5" abgekürzt. Diese beiden Enzyme sind im Stand der Technik bereits als sogenannte DNA-Reparatur-Enzyme bekannt. Unter DNA-Reparatur ist definitionsgemäß die Spaltung bzw. Entfernung von UV-induzierten Pyrimidindimeren aus der DNA zu verstehen.
Photolyase ist die Kurzbezeichnung für Desoxyribodipyrimidin-Photolyase bzw. DNA-Photolyase, ein Enzym mit der Klassifizierungsnummer EC 4.1.99.3. Eine besonders effiziente Photolyase stammt aus *Anacystis nidulans,* einem phototrophen marinen Mikroorganismus. Die Photolyase aus *A. nidulans* wird in technisch relevanten Mengen mittlerweile aus E. coli gewonnen. Photolyase ist zur Aktivierung auf Licht angewiesen.
Das Enzym T4 Endonuclease V wird vom denV-Gen der Bakteriophage T4 produziert und gehört zu den Phosphodiesterasen, die die Nucleinsäuren an der (5'-3')-Bindung hydrolytisch spalten. T4N5 ist auch ohne Lichteinfluss aktiv.
Erfindungsgemäß besonders bevorzugt ist der Einsatz von liposomenverkapselten DNA-Reparaturenzymen. Liposomenverkapselte Photolyase ist im Handel z. B. unter der Produktbezeichnung Photosome™, liposomenverkapselte T4N5 z. B. unter der Bezeichnung Ultrasome™ von der Firma AGI Dermatics, USA, erhältlich.
Erfindungsgemäß besonders bevorzugte Stiftzusammensetzungen sind dadurch gekennzeichnet, dass sie mindestens eines der Handelsprodukte Photosomes™ oder Ultrasomes™ in Gesamtmengen von 0,1 - 10 Gew.-%, bevorzugt 0,5 - 5,0 Gew.-% und besonders bevorzugt 1,0 - 4,0 Gew.-%, bezogen auf den gesamten erfindungsgemäßen Stift, enthalten.
Erfindungsgemäß besonders bevorzugte Stiftzusammensetzungen sind dadurch gekennzeichnet, dass sie mindestens ein Monomer, Oligomer oder Polymer von Aminosäuren, N-C₂-C₂₄-Acylaminosäuren und/oder den Estern und/ oder den physiologisch verträglichen Metallsalzen dieser Substanzen in Gesamtmengen von 0,0000001 - 10 Gew.-%, bevorzugt 0,001 - 5 Gew.-% und besonders bevorzugt 0,01 - 1-2-3 Gew.-%, jeweils bezogen auf den Aktivsubstanzgehalt in der gesamten erfindungsgemäßen Stiftzusammensetzung, enthalten.

In einer weiteren bevorzugten Ausführungsform enthalten die erfindungsgemäßen Stiftzusammensetzungen mindestens ein DNA-Oligonucleotid oder mindestens ein RNA-Oligonucleotid.
Erfindungsgemäß werden unter einem Oligonucleotid Polymerisate aus 2 bis 20, bevorzugt 2 bis 10 Mononucleotiden verstanden, die ebenso wie bei Polynucleotiden und Nucleinsäuren durch Phosphorsäurediester-Brücken verknüpft sind. Die Nucleotide bestehen aus Nucleobasen (meist Pyrimidin- oder Purin-Derivaten), Pentosen (meist D-Ribofuranose oder 2-Desoxy-D-ribofuranose in β-N-glykosidischer Bindung an die Nucleobase) und Phosphorsäure. Die Mononucleotide sind zum Beispiel Adenosinphosphate, Cytidinphosphate, Guanosinphosphate, Uridinphosphate und Thymidinphosphate, insbesondere CMP (Cytidin-5'-monophosphat), UDP (Uridin-5'-diphosphat), ATP (Adenosin-5 '-triphosphat) und GTP (Guanosin-5'-triphosphat). Ein erfindungsgemäß besonders bevorzugtes Oligonucleotid ist das Thymidin-Dinucleotid.
Erfindungsgemäß besonders bevorzugte Stiftzusammensetzungen sind dadurch gekennzeichnet, dass sie mindestens ein DNA-Oligonucleotid und/oder ein RNA-Oligonucleotid in Gesamtmengen von 0,000001 - 5 Gew.-%, bevorzugt 0,0001 - 0,5 Gew.-% und besonders bevorzugt 0,001 - 0,05 Gew.-%, bezogen auf die gesamte Zusammensetzung, enthalten.

In einer weiteren bevorzugten Ausführungsform enthalten die erfindungsgemäßen Stiftzusammensetzungen mindestens eine natürliche Betainverbindung. Erfindungsgemäß verwendete natürliche Betainverbindungen sind natürlich vorkommende Verbindungen mit der Atomgruppierung R₃N⁺-CH₂-X-COO⁻ gemäß IUPAC-Regel C-816.1. Sogenannte Betaintenside (synthetisch) fallen nicht unter die erfindungsgemäß verwendeten Betainverbindungen, ebenso wenig andere zwitterionische Verbindungen, in denen sich die positive Ladung an N oder P und die negative Ladung formal an O, S, B oder C befindet, die aber nicht der IUPAC-Regel C-816.1 entsprechen. Erfindungsgemäß bevorzugte Betainverbindungen sind Betain (Me₃N⁺-CH₂-COO⁻) und Carnitin (Me₃N⁺-CH₂-CHOH-CH₂-COO⁻), jeweils mit Me = Methyl und X = C-C-Einfachbindung (im Falle des Betains) oder X = -CHOH-CH₂- (im Falle des Carnitins).
Erfindungsgemäß besonders bevorzugte Stiftzusammensetzungen sind dadurch gekennzeichnet, dass sie mindestens eine natürliche Betainverbindung in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Stiftzusammensetzung, enthalten.

In einer weiteren bevorzugten Ausführungsform enthalten die erfindungsgemäßen Stiftzusammensetzungen mindestens ein Vitamin, Provitamin oder eine als Vitaminvorstufe bezeichnete Verbindung aus den Vitamingruppen A, B, C, E und H und den Estern der vorgenannten Substanzen.

Zur Gruppe der als Vitamin A bezeichneten Substanzen gehören das Retinol (Vitamin A₁) sowie das 3,4-Didehydroretinol (Vitamin A₂). Das β-Carotin ist das Provitamin des Retinols. Als Vitamin A-Komponente erfindungsgemäß besonders bevorzugt sind Vitamin A-Säure und deren Ester, Vitamin A-Aldehyd und Vitamin A-Alkohol sowie dessen Ester, wie Retinylpalmitat und Retinylacetat. Erfindungsgemäß besonders bevorzugte Zusammensetzungen sind dadurch gekennzeichnet, dass sie neben dem mindestens einen alkyl- oder hydroxyalkylsubstituierten Harnstoff gemäß Formel (A), insbesondere (2-Hydroxyethyl)harnstoff, mindestens ein Vitamin, Provitamin oder eine als Vitaminvorstufe bezeichnete Verbindung aus den Vitamingruppe A oder mindestens einen Ester hiervon in Gesamtmengen von 0,001 - 2 Gew.-%, bevorzugt 0,05 - 0,05 - 1 Gew.-%, bezogen auf die gesamte Zusammensetzung, enthalten.

Zur Vitamin B-Gruppe oder zu dem Vitamin B-Komplex gehören unter anderem
- Vitamin B₁, Trivialname Thiamin, chemische Bezeichung 3-[(4'-Amino-2'-methyl-5'-pyrimidinyl)-methyl]-5-(2-hydroxyethyl)-4-methylthiazoliumchlorid. Bevorzugt wird Thiaminhydrochlorid in Mengen von 0,0005 bis 0,1 - 1 Gew.-%, bezogen auf die gesamte erfindungsgemäße Zusammensetzung, eingesetzt.
- Vitamin B₂, Trivialname Riboflavin, chemische Bezeichung 7,8-Dimethyl-10-(1-D-ribityl)-benzo[g]pteridin-2,4(3*H*, 10*H*)-dion. Bevorzugt werden Riboflavin oder seine Derivate in Mengen von 0,0005 bis 0,1 - 1 Gew.-%, bezogen auf die gesamte erfindungsgemäße Zusammensetzung, eingesetzt.
- Vitamin B₃. Unter dieser Bezeichnung werden die Verbindungen Nicotinsäure und Nicotinsäureamid (Niacinamid) geführt. Erfindungsgemäß bevorzugt ist das Nicotinsäureamid, das in den erfindungsgemäßen Zusammensetzungen bevorzugt in Mengen von 0,0005 bis 0,1 - 1 Gew.-%, bezogen auf die gesamte erfindungsgemäße Zusammensetzung, enthalten ist.
- Vitamin B₅ (Pantothensäure und Panthenol). Bevorzugt wird Panthenol eingesetzt. Erfindungsgemäß bevorzugte Derivate des Panthenols sind insbesondere die Ester und Ether des Panthenols sowie kationisch derivatisierte Panthenole. In einer weiteren bevorzugten Ausführungsform der Erfindung werden an Stelle von sowie zusätzlich zu Pantothensäure oder Panthenol auch Derivate des 2-Furanon mit der allgemeinen Strukturformel (VIT-1) eingesetzt. 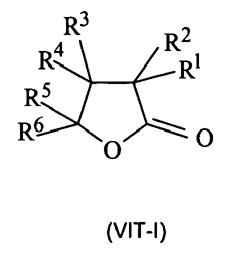 Besonders bevorzugt sind die 2-Furanon-Derivate, in denen die Substituenten R¹ bis R⁶ unabhängig voneinander ein Wasserstoffatom, einen Hydroxylrest, einen Methyl-, Methoxy-, Aminomethyl- oder Hydroxymethylrest, einen gesättigten oder ein- oder zweifach ungesättigten, linearen oder verzweigten C₂-C₄- Kohlenwasserstoffrest, einen gesättigten oder ein- oder zweifach ungesättigten, verzweigten oder linearen Mono-, Di- oder Trihydroxy-C₂-C₄ - Kohlenwasserstoffrest oder einen gesättigten oder ein- oder zweifach ungesättigten, verzweigten oder linearen Mono-, Di- oder Triamino-C₂-C₄ - Kohlenwasserstoffrest darstellen.
   Besonders bevorzugte Derivate sind die auch im Handel erhältlichen Substanzen Dihydro-3-hydroxy-4,4-dimethyl-2(3H)-furanon mit dem Trivialnamen Pantolacton (Merck), 4-Hydroxymethyl-γ-butyrolacton (Merck), 3,3-Dimethyl-2-hydroxy-γ-butyrolacton (Aldrich) und 2,5-Dihydro-5-methoxy-2-furanon (Merck), wobei ausdrücklich alle Stereoisomeren eingeschlossen sind. Das erfindungsgemäß außerordentlich bevorzugte 2-Furanon-Derivat ist Pantolacton (Dihydro-3-hydroxy-4,4-dimethyl-2(3H)-furanon), wobei in Formel (VIT-I) R¹ für eine Hydroxylgruppe, R² für ein Wasserstoffatom, R³ und R⁴ für eine Methylgruppe und R⁵ und R⁶ für ein Wasserstoffatom stehen. Das Stereoisomer (R)-Pantolacton entsteht beim Abbau von Pantothensäure.
   Erfindungsgemäß besonders bevorzugte Zusammensetzungen sind dadurch gekennzeichnet, dass sie mindestens eine der genannten Verbindungen des Vitamin B₅-Typs sowie der 2-Furanonderivate in einer Gesamtmenge von 0,05 bis 5 Gew.-%, bevorzugt 0,1 bis 3 Gew.-%, besonders bevorzugt 0,5 bis 2 Gew.-%, jeweils bezogen auf die gesamte Zusammensetzung, enthalten.
- Vitamin B₆, wobei man hierunter keine einheitliche Substanz, sondern die unter den Trivialnamen Pyridoxin, Pyridoxamin und Pyridoxal bekannten Derivate des 5-Hydroxymethyl-2-methylpyridin-3-ols versteht. Erfindungsgemäß besonders bevorzugte Zusammensetzungen sind dadurch gekennzeichnet, dass sie mindestens eine Vitamin B₆-Komponente in einer Gesamtmenge von 0,0001 bis 1,0 Gew.-%, insbesondere in Mengen von 0,001 bis 0,01 Gew.-%, enthalten.
- Vitamin B₇ (Biotin), auch als Vitamin H oder "Hautvitamin" bezeichnet. Bei Biotin handelt es sich um (3aS,4S, 6a*R*)-2-Oxohexahydrothienol[3,4-*d*]-imidazol-4-valeriansäure. Erfindungsgemäß besonders bevorzugte Zusammensetzungen sind dadurch gekennzeichnet, dass sie mindestens eine Komponente, ausgewählt aus Biotin und den Biotinestern, in einer Gesamtmenge von 0,0001 bis 1,0 Gew.-%, insbesondere 0,001 bis 0,01 Gew.-%, enthalten.
- Folsäure (Vitamin B₉, Vitamin B_{c}). Internationaler Freiname für N-[4-(2-Amino-3,4-dihydro-4-oxo-6-pteridinylmethylamino)-benzoyl]-L-glutaminsäure (N-Pteroyl-L-glutaminsäure, PteGlu). Folat wird synonym zu Pteroylglutamat gebraucht, Folate ist der Sammelbegriff für alle Folsäure-wirksamen Verbindungen und bezeichnet eine Substanzklasse, die einen mit 4-Aminobenzoesäure und L-Glutaminsäure verbundenen Pteridin-Ring enthält. Folsäure ist ein Wachstumsfaktor für verschiedene Mikroorganismen und eine Verbindung mit Vitamincharakter, die in der Natur meist als Polyglutamat und in reduzierter Form (7,8-Dihydrofolsäure, H₂Folat, DHF; Tetrahydrofolsäure, H₄Folat, THF; 5'-Methyl-Tetrahydrofolsäure, CH₃-H₄Folat, MeTHF) vorkommt. Erfindungsgemäß besonders bevorzugte Zusammensetzungen sind dadurch gekennzeichnet, dass sie mindestens eine Komponente, ausgewählt aus Folsäure, Folaten und deren Estern, in einer Gesamtmenge von 0,0001 bis 1,0 Gew.-%, insbesondere 0,01 bis 0,5 Gew.-%, bezogen auf die Zusammensetzung, enthalten.
- Orotsäure (Vitamin B₁₃, 1,2,3,6-Tetrahydro-2,6-dioxo-4-pyrimidin-carbonsäure, Uracil-6-carbonsäure, Molkensäure). Orotsäure, ihr Cholinester oder Orotsäure-Metallsalze (Orotate von Ca, Cr, Fe, K, Co, Cu, Li, Mg, Mn, Na, Zn, Sn) sind erfindungsgemäß besonders bevorzugt. Erfindungsgemäß besonders bevorzugte Zusammensetzungen sind dadurch gekennzeichnet, dass sie mindestens eine Komponente, ausgewählt aus Orotsäure, Orotaten und deren Estern, in einer Gesamtmenge von 0,0001 - 1,0 Gew.-%, insbesondere 0,01 - 0,5 Gew.-%, bezogen auf die Zusammensetzung, enthalten.

In einer weiteren bevorzugten Ausführungsform enthalten die erfindungsgemäßen Zusammensetzungen mindestens eine Substanz, die ausgewählt ist aus den Vitaminen, Provitaminen und Vitaminvorstufen der Gruppe B₁, B₂, B₃, B₆, B₇, B₉, B₁₃ und deren Estern und/oder Salzen und aus Pantolacton.

Bevorzugte Vitamine, Provitamine und Vitaminvorstufen der Gruppe C und deren Ester sind Vitamin C (Ascorbinsäure) und die Derivate Ascorbylpalmitat, -stearat, -dipalmitat, -acetat, Magnesiumascorbylphosphat, Natriumascorbylphosphat, Natrium- und Magnesiumascorbat, Dinatriumascorbylphosphat und -sulfat, Kaliumascorbyltocopherylphosphat, Chitosanascorbat oder Ascorbylglucosid. Die Kombination mit Tocopherolen kann ebenfalls bevorzugt sein. Erfindungsgemäß besonders bevorzugte Zusammensetzungen sind dadurch gekennzeichnet, dass sie mindestens eine der genannten Verbindungen des Vitamin C-Typs in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.
Zur Vitamin E-Gruppe zählen Tocopherol, insbesondere α-Tocopherol, und seine Derivate. Bevorzugte Derivate sind insbesondere die Ester, wie Tocopherylacetat, -nicotinat, -phosphat, - succinat, -linoleat, -oleat, Tocophereth-5, Tocophereth-10, Tocophereth-12, Tocophereth-18, Tocophereth-50 und Tocophersolan. Erfindungsgemäß besonders bevorzugte Zusammensetzungen sind dadurch gekennzeichnet, dass sie mindestens eine Substanz, ausgewählt aus Tocopherol und seinen Derivaten, in einer Gesamtmenge von 0,05 bis 5 Gew.-%, bevorzugt 0,1 bis 3 Gew.-%, besonders bevorzugt 0,5 bis 1 - 2 Gew.-%, jeweils bezogen auf die gesamte Zusammensetzung, enthalten.

Vitamin H ist eine andere Bezeichnung für Biotin oder Vitamin B₇ (siehe oben).

Vitamin A-palmitat (Retinylpalmitat), Pantolacton, Nicotinsäureamid, Pyridoxin, Pyridoxamin, Pyridoxal, Biotin, Ascorbylpalmitat, Ascorbylacetat, Mg-Ascorbylphosphat, Na-Ascorbylphosphat, Natrium- und Magnesiumascorbat und die Tocopherolester, besonders Tocopherylacetat, sind erfindungsgemäß besonders bevorzugt.

In einer weiteren bevorzugten Ausführungsform enthalten die erfindungsgemäßen Stiftzusammensetzungen mindestens eine α-Hydroxycarbonsäure, α-Ketocarbonsäure oder β-Hydroxycarbonsäure oder deren Ester-, Lacton- oder Salzform. Erfindungsgemäß bevorzugte α-Hydroxycarbonsäuren oder α-Ketocarbonsäuren sind Glycolsäure, Milchsäure, Weinsäure, Citronensäure, 2-Hydroxybutansäure, 2,3-Dihydroxypropansäure, 2-Hydroxypentansäure, 2-Hydroxyhexansäure, 2-Hydroxyheptansäure, 2-Hydroxyoctansäure, 2-Hydroxydecansäure, 2-Hydroxydodecansäure, 2-Hydroxytetradecansäure, 2-Hydroxyhexadecansäure, 2-Hydroxyoctadecansäure, Mandelsäure, 4-Hydroxymandelsäure, Äpfelsäure, Erythrarsäure, Threarsäure, Glucarsäure, Galactarsäure, Mannarsäure, Gularsäure, 2-Hydroxy-2-methylbernsteinsäure, Gluconsäure, Brenztraubensäure, Glucuronsäure und Galacturonsäure. Besonders bevorzugte α-Hydroxycarbonsäuren sind Milchsäure, Citronensäure, Glycolsäure und Gluconsäure. Eine besonders bevorzugte β-Hydroxycarbonsäure ist Salicylsäure. Die Ester der genannten Säuren sind ausgewählt aus den Methyl-, Ethyl-, Propyl-, Isopropyl-, Butyl-, Amyl-, Pentyl-, Hexyl-, 2-Ethylhexyl-, Octyl-, Decyl-, Dodecyl- und Hexadecylestern. Erfindungsgemäß besonders bevorzugte Stiftzusammensetzungen sind dadurch gekennzeichnet, dass sie mindestens eine α-Hydroxycarbonsäure, α-Ketocarbonsäure und/oder β-Hydroxycarbonsäure oder ein Derivat, insbesondere einen Ester, ein Lacton oder ein Salz hiervon, in einer Gesamtmenge von 0,01 - 10 Gew.-%, bevorzugt 0,1 - 5 Gew.-%, besonders bevorzugt 0,5 - 1 - 2 Gew.-%, jeweils bezogen auf die gesamte Zusammensetzung, enthalten.

In einer weiteren bevorzugten Ausführungsform enthalten die erfindungsgemäßen Zusammensetzungen mindestens ein Flavonoid oder mindestens einen Flavonoid-reichen Pflanzenextrakt.
Die erfindungsgemäß bevorzugten Flavonoide umfassen die Glycoside der Flavone, der Flavanone, der 3-Hydroxyflavone (Flavonole), der Aurone und der Isoflavone. Besonders bevorzugte Flavonoide sind ausgewählt aus Naringin (Aurantiin, Naringenin-7-rhamnoglucosid), α-Glucosylrutin, α-Glucosylmyricetin, α-Glucosylisoquercetin, α-Glucosylquercetin, Dihydroquercetin (Taxifolin), Hesperidin (3',5,7-Trihydroxy-4'-methoxyflavanon-7-rhamnoglucosid, Hesperitin-7-O-rhamnoglucosid), Neohesperidin, Rutin (3,3',4',5,7-Pentahydroxyflavon-3-rhamnoglucosid, Quercetin-3-rhamnoglucosid), Troxerutin (3,5-Dihydroxy-3',4',7-tris(2-hydroxyethoxy)-flavon-3-(6-O-(6-deoxy-α-L-mannopyranosyl)-β-D-glucopyranosid)), Monoxerutin (3,3',4',5-Tetrahydroxy-7-(2-hydroxy-ethoxy)-flavon-3-(6-O-(6-deoxy-α-L-mannopyranosyl)-β-D-glucopyranosid)), Diosmin (3',4',7-Trihydroxy-5-methoxyflavanon-7-rhamnoglucosid), Eriodictin und Apigenin-7-glucosid (4',5,7-Trihydroxyflavon-7-glucosid).
Erfindungsgemäß außerordentlich bevorzugte Flavonoide sind α-Glucosylrutin, Naringin und Apigenin-7-glucosid.
Ebenfalls bevorzugt sind die aus zwei Flavonoideinheiten aufgebauten Biflavonoide, die z. B. in Gingko-Arten vorkommen. Weitere bevorzugte Flavonoide sind die Chalkone, vor allem Phloricin, Hesperidinmethylchalkon und Neohesperidindihydrochalkon.

Erfindungsgemäß besonders bevorzugte Zusammensetzungen sind dadurch gekennzeichnet, dass sie mindestens ein Flavonoid in einer Gesamtmenge von 0,0001 bis 1 Gew.-%, bevorzugt 0,0005 bis 0,5 Gew.-% und besonders bevorzugt 0,001 bis 0,1 Gew.-%, jeweils bezogen auf die Flavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Isoflavonoid oder mindestens einen Isofiavonoid-reichen Pflanzenextrakt. Zu den Isoflavonoiden werden an dieser Stelle die Isoflavone und die Isoflavon-Glycoside gezählt.
Unter Isoflavonen sind im Sinne der vorliegenden Erfindung Stoffe zu verstehen, die Hydrierungs-, Oxidations- oder Substitutionsprodukte des 3-Phenyl-4H-1-benzopyrans darstellen, wobei eine Hydrierung in der 2,3-Stellung des Kohlenstoffgerüsts vorliegen kann, eine Oxidation unter Ausbildung einer Carbonylgruppe in der 4-Stellung vorliegen kann, und unter Substitution der Ersatz eines oder mehrerer Wasserstoffatome durch Hydroxy- oder Methoxy-Gruppen zu verstehen ist. Zu den erfindungsgemäß bevorzugten Isoflavonen zählen beispielsweise Daidzein, Genistein, Prunetin, Biochanin, Orobol, Santal, Pratensein, Irigenin, Glycitein, Biochanin A und Formononetin. Als Isoflavone besonders bevorzugt sind Daidzein, Genistein, Glycitein und Formononetin.
In den erfindungsgemäß bevorzugten Isoflavon-Glycosiden sind die Isoflavone über mindestens eine Hydroxygruppe mit mindestens einem Zucker glycosidisch verknüpft. Als Zucker kommen Mono- oder Oligosaccharide, insbesondere D-Glucose, D-Galactose, D-Glucuronsäure, D-Galacturonsäure, D-Xylose, D-Apiose, L-Rhamnose, L-Arabinose und Rutinose in Betracht. Erfindungsgemäß besonders bevorzugte Isoflavon-Glycoside sind Daidzin und Genistin.
Erfindungsgemäß weiterhin bevorzugt ist es, wenn die Isoflavone und/oder deren Glycoside als Bestandteile eines aus einer Pflanze gewonnenen Substanzgemisches, insbesondere eines pflanzlichen Extraktes, in den Zubereitungen enthalten sind. Solche pflanzlichen Substanzgemische können in dem Fachmann geläufiger Weise beispielsweise durch Auspressen oder Extrahieren aus Pflanzen wie Soja, Rotklee oder Kichererbsen gewonnen werden. Besonders bevorzugt werden in den erfindungsgemäßen Zubereitungen Isoflavone oder Isoflavon-Glycoside in Form von aus Soja gewonnenen Extrakten eingesetzt, wie sie beispielsweise unter der Produktbezeichnung Soy Protein Isolate SPI (Protein Technology International, St. Louis) oder Soy Phytochemicals Concentrate SPC (Archer Daniels Midland, Decatur) im Handel erhältlich sind. Ein weiterer besonders bevorzugter Isoflavonoid-reicher Pflanzenextrakt ist Apfelkernextrakt, insbesondere das Handelsprodukt Ederline von Seporga. Ederline enthält Phytohormone, Isoflavonoide, Phytosterole, Triterpenoide, Tocopherole und natürliche Wachse.
Erfindungsgemäß besonders bevorzugte Zusammensetzungen sind dadurch gekennzeichnet, dass sie mindestens ein Isoflavonoid in einer Gesamtmenge von 0,00001 bis 1 Gew.-%, bevorzugt 0,0005 bis 0,5 Gew.-% und besonders bevorzugt 0,001 bis 0,1 Gew.-%, jeweils bezogen auf die Isoflavonoidaktivsubstanz in der gesamten kosmetischen Zusammensetzung, enthalten.

In einer weiteren bevorzugten Ausführungsform enthalten die erfindungsgemäßen Zusammensetzungen mindestens ein Polyphenol oder einen Polyphenol-reichen Pflanzenextrakt.
Unter Polyphenolen sind erfindungsgemäß aromatische Verbindungen zu verstehen, die mindestens zwei phenolische Hydroxy-Gruppen im Molekül enthalten. Hierzu zählen die drei Dihydroxybenzole Brenzcatechin, Resorcin und Hydrochinon, weiterhin Phloroglucin, Pyrogallol und Hexahydroxybenzol. In der Natur treten freie und veretherte Polyphenole beispielsweise in Blütenfarbstoffen (Anthocyanidine, Flavone), in Gerbstoffen (Catechine, Tannine), als Flechten- oder Farn-Inhaltsstoffe (Usninsäure, Acylpolyphenole), in Ligninen und als Gallussäure-Derivate auf. Bevorzugte Polyphenole sind Flavone, Catechine, Usninsäure, und als Tannine die Derivate der Gallussäure, Digallussäure und Digalloylgallussäure. Besonders bevorzugte Polyphenole sind die monomeren Catechine, das heißt die Derivate der Flavan-3-ole, und Leukoanthocyanidine, das heißt die Derivate der Leukoanthocyanidine, die bevorzugt in 5,7,3',4',5'-Stellung phenolische Hydroxygruppen tragen, bevorzugt Epicatechin und Epigallocatechin, sowie die daraus durch Selbstkondensation entstehenden Gerbstoffe. Solche Gerbstoffe werden bevorzugt nicht in isolierter Reinsubstanz, sondern als Extrakte gerbstoffreicher Pflanzenteile eingesetzt, z. B. Extrakte von Catechu, Quebracho, Eichenrinde und Pinienrinde sowie anderen Baumrinden, Blättern von Grünem Tee (camellia sinensis) und Mate. Ebenfalls besonders bevorzugt sind die Tannine.
Ein besonders bevorzugter Polyphenol-reicher kosmetischer Wirkstoff ist das Handelsprodukt Sepivinol R, ein Extrakt aus Rotwein, erhältlich von der Firma Seppic. Ein weiterer besonders bevorzugter Polyphenol-reicher kosmetischer Wirkstoff ist das Handelsprodukt Crodarom Chardonnay L, ein Extrakt aus den Kernen der Chardonnay-Traube, erhältlich von der Firma Croda.
Erfindungsgemäß bevorzugt werden die Polyphenole in Mengen von 0,001 bis 10 Gew.-%, besonders bevorzugt 0,005 bis 5 Gew.-% und außerordentlich bevorzugt 0,01 bis 3 Gew.-%, jeweils bezogen auf das Gewicht des Handelsproduktes, das mindestens ein Polyphenol enthält, in der gesamten erfindungsgemäßen Zusammensetzung, eingesetzt.

In einer weiteren bevorzugten Ausführungsform enthalten die erfindungsgemäßen Zusammensetzungen mindestens ein Ubichinon oder ein Ubichinol oder deren Derivate. Ubichinole sind die reduzierte Form der Ubichinone. Die erfindungsgemäß bevorzugten Ubichinone weisen die Formel (UBI-I) auf: 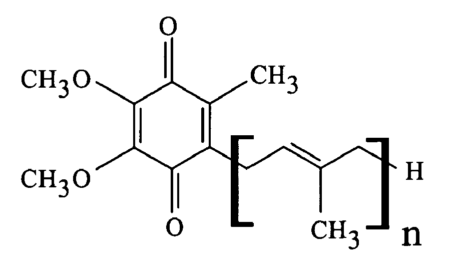 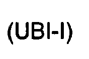 mit n = 6, 7, 8, 9 oder 10.
Besonders bevorzugt ist das Ubichinon der Formel (UBI-I) mit n = 10, auch bekannt als Coenzym Q10.
Erfindungsgemäß besonders bevorzugte Zusammensetzungen sind dadurch gekennzeichnet, dass sie mindestens ein Ubichinon, Ubichinol oder ein Derivat hiervon in einer Gesamtmenge von 0,0001 bis 1 Gew.-%, bevorzugt 0,001 bis 0,5 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen Silymarin. Silymarin stellt erfindungsgemäß ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören.
Erfindungsgemäß besonders bevorzugte Zusammensetzungen sind dadurch gekennzeichnet, dass sie Silymarin in Mengen von 0,00001 bis 1 Gew.-%, bevorzugt 0,0001 bis 0,01 Gew.-% und besonders bevorzugt 0,005 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens eine Substanz, ausgewählt aus Purin und Purin-Derivaten.
Purin (7*H*-Imidazo[4,5-*d*]pyrimidin) kommt frei in der Natur nicht vor, bildet jedoch den Grundkörper der Purine. Purine ihrerseits sind eine Gruppe wichtiger, in der Natur weit verbreiteter und an menschlichen, tierischen, pflanzlichen und mikrobiellen Stoffwechselvorgängen beteiligter Verbindungen, die sich vom Grundkörper durch Substitution mit OH, NH₂, SH in 2-, 6- und 8-Stellung und/oder mit CH₃ in 1-, 3-, 7-Stellung ableiten. Purin kann beispielsweise aus Aminoacetonitril und Formamid hergestellt werden. Purine und Purinderivate werden oft aus Naturstoffen isoliert, sind aber auch auf vielen Wegen synthetisch zugänglich.

Bevorzugte erfindungsgemäße Zusammensetzungen enthalten Purin und/oder Purinderivat(e) in engeren Mengenbereichen. Hier sind erfindungsgemäß bevorzugte kosmetische Stiftzusammensetzungen dadurch gekennzeichnet, dass sie - bezogen auf ihr Gewicht - eine Gesamtmenge von 0,001 - 2,5 Gew.-%, vorzugsweise 0,01 - 1 Gew.-%, besonders bevorzugt 0,1 - 0,8 Gew.-% und insbesondere 0,2 - 0,5 Gew.-% Purin(e) und/oder Purinderivat(e) enthalten.

Besonders bevorzugte Purin-Derivate sind ausgewählt aus Verbindungen der allgemeinen Struktur (PUR-I), 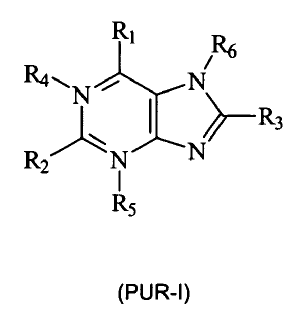 in der die Reste R¹, R² und R³ unabhängig voneinander ausgewählt sind aus -H,- OH, -NH₂, - SH und die Reste R⁴, R⁵ und R⁶ unabhängig voneinander ausgewählt sind aus -H, einem C₁- bis C₄-Alkylrest, einem C₁- bis C₄-Monohydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe.

Erfindungsgemäß besonders bevorzugte kosmetische Zusammensetzungen sind dadurch gekennzeichnet, dass sie Purin und/oder Purinderivat(e) der Formel (PUR-I) enthalten, in der die Reste R¹, R² und R³ unabhängig voneinander ausgewählt sind aus -H, - OH, -NH₂, -SH und die Reste R⁴, R⁵ und R⁶ unabhängig voneinander ausgewählt sind aus -H, -CH₃ und -CH₂-CH₃, wobei folgende Verbindungen außerordentlich bevorzugt sind:
- Purin (R¹ = R² = R³ = R⁴ = R⁵ = R⁶ = H)
- Adenin (R¹ = NH₂, R² = R³ = R⁴ = R⁵ = R⁶ = H)
- Guanin (R¹ = OH, R² = NH₂, R³ = R⁴ = R⁵ = R⁶ = H)
- Harnsäure (R¹ = R² = R³ = OH, R⁴ = R⁵ = R⁶ = H)
- Hypoxanthin (R¹ = OH, R² = R³ = R⁴ = R⁵ = R⁶ = H)
- 6-Purinthiol (R¹ = SH, R² = R³ = R⁴ = R⁵ = R⁶ = H)
- 6-Thioguanin (R¹ = SH, R² = NH₂, R³ = R⁴ = R⁵ = R⁶ = H)
- Xanthin (R¹ = R² = OH, R³ = R⁴ = R⁵ = R⁶ = H)
- Coffein (R¹ = R² = OH, R³ = H, R⁴ = R⁵ = R⁶ = CH₃)
- Theobromin (R¹ = R² = OH, R³ = R⁴ = H, R⁵ = R⁶ = CH₃)
- Theophyllin (R¹ = R² = OH, R³ = H, R⁴ = CH₃, R⁵ = CH₃, R⁶ = H).

Verbindungen der allgemeinen Formel (PUR-1), in denen R¹ und/oder R² einer OH-Gruppe entsprechen, existieren in tautomeren Verbindungen, insbesondere solchen der allgemeinen Formel (PUR-II), ausgehend von (PUR-I) mit R¹ = R² = OH : 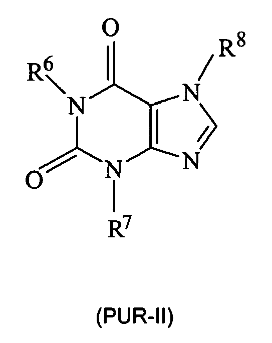 wobei in der Formel (PUR-II) die Reste R⁶, R⁷ und R⁸, die gleich oder verschieden sein können, ausgewählt sind aus einem Wasserstoffatom, einem C₁- bis C₄-Alkylrest, einem C₁- bis C₄-Mono-hydroxyalkylrest, einem C₂- bis C₄-Polyhydroxyalkylrest, einem (C₁- bis C₄)-Alkoxy-(C₁- bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁- bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe und einer Aryl-C₁-C₆-alkylgruppe, wobei Coffein (R⁶ = R⁷ = R⁸ = CH₃), Theobromin (R⁶ = H, R⁷ = R⁸ = CH₃) und Theophyllin (R⁶ = R⁷ = CH₃, R⁸ = H) außerordentlich bevorzugt sind.

Je nach gewünschtem Anwendungszweck der kosmetischen Zusammensetzung kann dabei die Art und Menge des Purinderivates variieren. Erfindungsgemäß bevorzugt sind die Purin-Derivate in Mengen von 0,0001 bis 2 Gew.-%, besonders bevorzugt 0,001 bis 1,5 Gew.-% und außerordentlich bevorzugt 0,005 bis 0,5 Gew.-%, jeweils bezogen auf die gesamte Zusammensetzung, enthalten. In erfindungsgemäßen Stiften zur Anticellulite-Behandlung der Haut hat sich insbesondere Coffein bewährt, das beispielsweise in Anticellulite-Mitteln bevorzugt in Mengen von 0,01 - 2 Gew.-%, weiter bevorzugt 0,1 - 1,5 Gew.-% und besonders bevorzugt 0,2 - 0,8 Gew.-%, jeweils bezogen auf das Mittel, enthalten ist.

Besonders bevorzugte Purin-Derivate sind ausgewählt aus den natürlich vorkommenden Xanthin-Derivaten, insbesondere ausgewählt aus Coffein, Theophyllin, Theobromin und Aminophyllin.

Erfindungsgemäß bevorzugte Aporphin-Alkaloide sind ausgewählt ist aus Verbindungen der allgemeinen Struktur (APO-ALK-1), 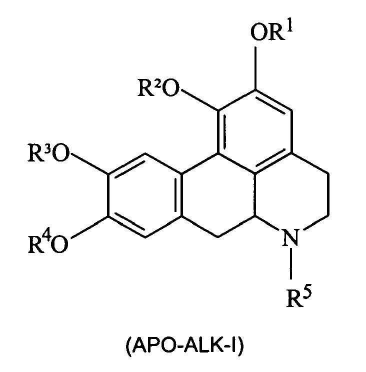 in der die Reste R¹, R², R³, R⁴ und R⁵, die gleich oder verschieden sein können, ausgewählt sind aus einem Wasserstoffatom, einem Halogenatom, einem C₁- bis C₄-Alkylrest, einem C₁- bis C₄-Monohydroxyalkylrest, einem C₂- bis C₄-Polyhydroxyalkylrest, einem (C₁- bis C₄)-Aikoxy-(C₁-bis C₄)-alkylrest, einem C₁- bis C₄-Aminoalkylrest, einem Hydroxy-(C₁- bis C₄)-alkylaminorest, einem C₁- bis C₄-Hydroxyalkoxyrest, einem C₁- bis C₄-Hydroxyalkyl-(C₁-bis C₄)-aminoalkylrest oder einem (Di-C₁-bis C₄-Alkylamino)-(C₁- bis C₄)-alkylrest, einer gegebenenfalls substituierten Arylgruppe, insbesondere einer Phenylgruppe, einer gegebenenfalls substituierten Heteroarylgruppe, einer Aryl-C₁-C₆-alkylgruppe, einer Acylgruppe, einer Sulfonylgruppe oder einem Zucker, und den kosmetisch verträglichen Salzen von (APO-ALK-I) mit einer Säure, in Form aller 15 optischen Isomere, in isomerenreiner Form oder als Mischung optischer Isomere.
Beispiele für die als Substituenten in den erfindungsgemäß kombinierten Verbindungen genannten C₁- bis C₄-Alkylreste sind die Gruppen Methyl, Ethyl, Propyl, Isopropyl und Butyl. Ethyl und Methyl sind bevorzugte Alkylreste. Erfindungsgemäß bevorzugte C₁- bis C₄-Alkoxyreste sind beispielsweise eine Methoxy- oder eine Ethoxygruppe. Weiterhin können als bevorzugte Beispiele für eine C₁- bis C₄-Hydroxyalkylgruppe eine Hydroxymethyl-, eine 2-Hydroxyethyl-, eine 3-Hydroxypropyl- oder eine 4-Hydroxybutylgruppe genannt werden. Eine 2-Hydroxyethylgruppe ist besonders bevorzugt. Eine besonders bevorzugte C₂- bis C₄-Polyhydroxyalkylgruppe ist die 1,2-Dihydroxyethylgruppe. Beispiele für Halogenatome sind erfindungsgemäß F-, Cl- oder Br-Atome, CI-Atome sind ganz besonders bevorzugt.
Als Zuckerrest können beliebige Mono- oder Oligo-, insbesondere Disaccharide, eingesetzt werden. Üblicherweise werden Zucker mit 5 bzw. 6 Kohlenstoffatomen sowie die entsprechenden Oligosaccharide eingesetzt. Solche Zucker sind beispielsweise Glucose, Fructose, Galactose, Arabinose, Ribose, Xylose, Lyxose, Allose, Altrose, Mannose, Gulose, Idose, Talose und Sucrose. Bevorzugte Zuckerbausteine sind Glucose, Fructose, Galactose, Arabinose und Sucrose; Glucose ist besonders bevorzugt.

Besonders bevorzugte Aporphin-Alkaloide sind ausgewählt aus Verbindungen der allgemeinen Struktur (APO-ALK-l), in denen R¹ = R² = R³ = R⁴ = R⁵ = CH₃ ist. Diese Verbindung wird auch als 1,2,9,10-Tetramethoxyaporphin bezeichnet.

Bevorzugte erfindungsgemäße Zusammensetzungen enthalten mindestens ein Aporphin-Alkaloid in engeren Mengenbereichen. Hier sind erfindungsgemäß bevorzugte kosmetische Zusammensetzungen dadurch gekennzeichnet, dass sie - bezogen auf ihr Gewicht - eine Gesamtmenge von 0,0001 - 1 Gew.-%, vorzugsweise 0,001 - 0,5 Gew.-%, besonders bevorzugt 0,002 - 0,1 Gew.-% und insbesondere 0,005 - 0,01 Gew.-% mindestens eines Aporphin-Alkaloids enthalten.

In einer weiteren bevorzugten Ausführungsform enthalten die erfindungsgemäßen Zusammensetzungen Ectoin. Ectoin ist der Trivialname für 2-Methyl-1,4,5,6-tetrahydropyrimidin-4-carboxylat. Erfindungsgemäß bevorzugt ist Ectoin in Mengen von 0,0001 bis 1 Gew.-%, besonders bevorzugt 0,001 bis 0,5 Gew.-% und außerordentlich bevorzugt 0,005 bis 0,01 Gew.-%, jeweils bezogen auf die gesamte Zusammensetzung, enthalten.

In einer weiteren bevorzugten Ausführungsform enthalten die erfindungsgemäßen Zusammensetzungen Kreatin. Kreatin ist der Trivialname für N-Methyl-guanidino-essigsäure bzw. N-Amidinosarkosin. Erfindungsgemäß bevorzugt ist Kreatin in Mengen von 0,0001 bis 1 Gew.-%, besonders bevorzugt 0,001 bis 0,5 Gew.-% und außerordentlich bevorzugt 0,01 bis 0,1 Gew.-%, jeweils bezogen auf die gesamte Zusammensetzung, enthalten.

In einer weiteren bevorzugten Ausführungsform enthalten die erfindungsgemäßen Zusammensetzungen mindestens einen Olivenblattextrakt (Olea Europaea (Olive) Leaf Extract). Ein erfindungsgemäß besonders bevorzugter Olivenblattextrakt ist unter der Handelsbezeichnung Oleanoline DPG von der Firma Vincience erhältlich. Ein weiterer erfindungsgemäß besonders bevorzugter Olivenblattextrakt ist unter der Handelsbezeichnung Olea europ Fol extr. S. sicc. von der Firma Fruitarom erhältlich.
Erfindungsgemäß besonders bevorzugte Zusammensetzungen sind dadurch gekennzeichnet, dass sie mindestens einen Olivenblattextrakt in einer Gesamtmenge von 0,01 bis 5 Gew.-%, bevorzugt 0,1 bis 3 Gew.-% und besonders bevorzugt 0,5 bis 1 - 2 Gew.-%, jeweils bezogen auf den Extrakt als Handelsprodukt tel quel in der gesamten erfindungsgemäßen Zusammensetzung, enthalten.

Olivenblattextrakte können einen hohen Gehalt an Oleanol, Oleanolsäure und/oder Oleuropein aufweisen. In einer weiteren bevorzugten Ausführungsform enthalten die erfindungsgemäßen Zusammensetzungen Oleanol, Oleanolsäure und/oder Oleuropein. Erfindungsgemäß besonders bevorzugte Zusammensetzungen sind dadurch gekennzeichnet, dass sie Oleanol, Oleanolsäure und/oder Oleuropein in einer Gesamtmenge von 0,00001 - 2 Gew.-%, bevorzugt 0,001 - 1 Gew.-% und besonders bevorzugt 0,05 - 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen Ursolsäure. Erfindungsgemäß besonders bevorzugte Zusammensetzungen sind dadurch gekennzeichnet, dass sie Ursolsäure in einer Gesamtmenge von 0,00001 bis 2 Gew.-%, bevorzugt 0,001 bis 1 Gew.-% und besonders bevorzugt 0,05 bis 0,1 Gew.-%, jeweils bezogen auf die gesamte erfindungsgemäße Zusammensetzung, enthalten.

In einer weiteren bevorzugten Ausführungsform enthalten die erfindungsgemäßen Zusammensetzungen mindestens einen Wirkstoff, der ausgewählt ist aus den Mono- und Polyhydroxystilbenen und deren Estern. Unter Polyhydroxystilbenen werden erfindungsgemäß Stilbene verstanden, die mit 2, 3, 4, 5, 6, 7, 8, 9 oder 10 Hydroxygruppen an den beiden Phenylresten substituiert sind, wobei diese verestert sein können. Mono- und Polyhydroxystilbene und deren Ester erhöhen und/oder verbessern die Interaktion zwischen der extrazellulären Matrix und den Fibroblasten. Erfindungsgemäß besonders bevorzugte Hydroxystilbene und deren Ester sind ausgewählt aus Resveratrol (trans-Stilben-3,4'-5-triol), den Resveratrolmono-, -di- und -triphosphorsäureestern und deren Salzen. Ein erfindungsgemäß besonders bevorzugter Resveratrolphosphorsäureester ist Trisodium Resveratrol Triphosphate, z. B. erhältlich von Ajinomoto.
Erfindungsgemäß besonders bevorzugte kosmetische oder dermatologische Zusammensetzungen sind dadurch gekennzeichnet, dass sie mindestens einen Wirkstoff, der ausgewählt ist aus den Mono- und Polyhydroxystilbenen und deren Estern, in einer Gesamtmenge von 0,000001 - 5 Gew.-%, bevorzugt 0,00001 - 1 Gew.-%, besonders bevorzugt 0,0001 - 0,1 Gew.-% und außerordentlich bevorzugt 0,005 - 0,05 Gew.-%, enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Derivat von methyliertem Silanol, vorzugsweise mindestens einen Ester von methyliertem Silanol. Bevorzugte Derivate von methyliertem Silanol sind ausgewählt aus:
- sodium mannuronate methylsilanol (Algisium, Exsymol)
- methylsilanol mannuronate (Algisium C®, Exsymol)
- methylsilanol mannuronate Nylon-12 (Algisium C powder®, Exsymol)
- ascorbylmethylsilanol (Ascorbosilane concentrate C®, Exsymol)
- ascorbylmethylsilanol pectinate (Ascorbosilane C®, Exsymol)
- dimethyl oxobenzodioxsilane (DSBC®, Exsymol)
- dimethyl oxobenzodioxasilane Nylon-12 (DSBC powder®, Exsymol)
- sodium hyaluronate dimethylsilanol (DSH®, Exsymol)
- dimethylsilanol hyaluronate (DSHC®, Exsymol)
- methysilanol glycyrrhizinate (Glysinol®, Exsymol)
- methylsilanolhydroxyproline (Hydroxyprolisilane®, Exsymol)
- methylsilanolhydroxyproline aspartate (Hydroxyprolisilane C®, Exsymol)
- sodium lactate methylsilanol (Lasilium®, Exsymol)
- lactoylmethylsilanol elastinate (Lasilium C®, Exsymol)
- dioleyl tocopheryl methylsilanol (Liposiliol C®, Exsymol)
- methylsilanol acetylmethionate (Methiosilane®, Exsymol)
- acetylmethionylmethylsifanol elastinate (Methiosilane C®, Exsymol)
- methylsilanol PEG 7 glyceryl cocoate (Monosiliol®, Exsymol)
- methylsilanol tri PEG 7 glyceryl cocoate (Monosiliol C®, Exsymol)
- methylsilanol elastinate (Proteosilane C®, Exsymol)
- pyrollidone carboxylate caustic methylsilanol (Silhydrate®, Exsymol)
- pyrollidone carboxylate copper methylsilanol (Silhydrate C®, Exsymol)
- methylsilanolcarboxymethyl theophylline (Theophyllisilane®, Exsymol)
- methylsilancarboxymethyl theophylline alginate (Theophyllisilane C® Exsymol)
- methylsilanol acetyltyrosine (Tyrosilane®, Exsymol)
- copper acetyl tyrosinate methylsilanol (Tyrosilane C®, Exsymol).
Besonders bevorzugt sind Sodium Hyaluronate Dimethylsilanol, Dimethylsilanol Hyaluronate, Methylsilanol Mannuronate, Methylsilanol Hydroxyproline und Methylsilanol Hydroxyproline Aspartate. In einer weiteren bevorzugten Ausführungsform enthalten die erfindungsgemäßen Zusammensetzungen mindestens ein Derivat von methyliertem Silanol in Gesamtmengen von 0,001 - 5 Gew.-%, bevorzugt 0,005 - 1 Gew.-% und besonders bevorzugt 0,01 - 0,5 Gew.-%, jeweils bezogen auf die Aktivsubstanz in der gesamten erfindungsgemäßen Zusammensetzung.

In einer weiteren bevorzugten Ausführungsform enthalten die erfindungsgemäßen Zusammensetzungen Phytinsäure. Erfindungsgemäß besonders bevorzugte kosmetische oder dermatologische Zusammensetzungen sind dadurch gekennzeichnet, dass sie Phytinsäure in einer Gesamtmenge von 0,001 - 1 Gew.-%, bevorzugt 0,01 - 0,5 Gew.-% und besonders bevorzugt 0,05 - 0,1 Gew.-% enthalten, jeweils bezogen auf die gesamte Zusammensetzung.

In einer weiteren bevorzugten Ausführungsform enthalten die erfindungsgemäßen Zusammensetzungen mindestens einen Extrakt aus Maiskörnern (Zea Mays (Corn) Kernel Extract). Ein erfindungsgemäß besonders bevorzugter Extrakt aus Maiskörnern ist unter der Handelsbezeichnung Deliner von der Firma Coletica erhältlich. Dieser Extrakt erhöht und/oder verbessert die Interaktion zwischen der extrazellulären Matrix und den Fibroblasten.
Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Maiskörnern (Zea Mays (Corn) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Maiskörnern (Zea Mays (Corn) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Haferkörnern (Avena Sativa (Oat) Kernel Extract). Ein erfindungsgemäß besonders bevorzugter Extrakt aus Haferkörnern ist unter der Handelsbezeichnung Drago Beta Glucan (02/060800) von der Firma Symrise erhältlich. Dieser Extrakt erhöht und/oder verbessert die Interaktion zwischen der extrazellulären Matrix und den Fibroblasten.
Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,01 - 5 Gew.-%, bevorzugt 0,1 - 3 Gew.-%, besonders bevorzugt 1 - 2 Gew.-% enthalten, jeweils bezogen auf den Gehalt an Extrakt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Extrakten aus Haferkörnern (Avena Sativa (Oat) Kernel Extract), in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens ein Produkt, das durch Fermentation von gezuckertem Schwarztee mit den zwei symbiotischen Mikroorganismen Saccharomyces und Xylinum gewonnen wird und die INCI-Bezeichnung Saccharomyces/ Xylinum/Black Tea Ferment trägt. Derartige Produkte erhöhen und/oder verbessern die Interaktion zwischen der extrazellulären Matrix und den Fibroblasten. Ein besonders bevorzugtes Produkt ist unter dem Handelsnamen Kombuchka von der Firma Sederma erhältlich (INCI-Bezeichnung: Saccharomyces/Xylinum/Black Tea Ferment, Glycerin, Hydroxyethylcellulose).
In einer weiteren bevorzugten Ausführungsform enthalten die erfindungsgemäßen Zusammensetzungen mindestens ein Produkt, das durch Fermentation von gezuckertem Schwarztee mit den zwei symbiotischen Mikroorganismen Saccharomyces und Acetobacter ylinum gewonnen wird, als KOmbucha bezeichnet wird und die INCI-Bezeichnung Saccharomyces/ Xylinum/Black Tea Ferment trägt. Derartige Produkte erhöhen und/oder verbessern die Interaktion zwischen der extrazellulären Matrix und den Fibroblasten. Ein besonders bevorzugtes Produkt ist unter dem Handelsnamen Kombuchka von der Firma Sederma erhältlich (INCI-Bezeichnung: Saccharomyces/ Xylinum/Black Tea Ferment, Glycerin, Hydroxyethylcellulose).
Weitere erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Xylinum gewonnen werden und die INCI-Bezeichnung Saccharomyces/Xylinum/Black Tea Ferment tragen, in einer Gesamtmenge von 0,01 - 5 Gew.-%, bevorzugt 0,1 - 3 Gew.-%, besonders bevorzugt 1-2 Gew.-% enthalten, jeweils bezogen auf den Gehalt an Produkt tel quel in der gesamten Zusammensetzung. Erfindungsgemäß besonders bevorzugte kosmetische oder dermatologische Zusammensetzungen sind dadurch gekennzeichnet, dass sie mindestens einen Wirkstoff, der ausgewählt ist aus Produkten, die durch Fermentation von gezuckertem Schwarztee mit den zwei symbiotischen Mikroorganismen Saccharomyces und Acetobacter Xylinum gewonnen werden und die INCI-Bezeichnung Saccharomyces/Xylinum/Black Tea Ferment tragen, in einer Gesamtmenge von 0,00001 - 1 Gew.-%, bevorzugt 0,0001 - 0,1 Gew.-%, besonders bevorzugt 0,001 - 0,05 Gew.-% enthalten, jeweils bezogen auf den Gehalt an Aktivsubstanz in der gesamten Zusammensetzung.

In einer weiteren bevorzugten Ausführungsform enthalten die erfindungsgemäßen Zusammensetzungen mindestens einen Extrakt aus Apfelkernextrakten (Pyrus Malus (Apple) Fruit Extract). Derartige Extrakte erhöhen und/oder verbessern die Interaktion zwischen der extrazellulären Matrix und den Fibroblasten. Erfindungsgemäß besonders bevorzugte Apfelkernextrakte sind unter der Handelsbezeichnung Ederline von der Firma Seporga erhältlich. Das Produkt Ederline enthält Phytohormone, Isoflavonoide, Phytosterole, Triterpenoide, Tocopherole und natürliche Wachse. Ederline ist einmal in wasserlöslicher Form als Ederline-H (INCI: PEG-40 Hydrogenated Castor Oil, PPG-2-Ceteareth-9, Pyrus Malus (Apple) Fruit Extract), zum anderen in fettlöslicher Form als Ederline-L (INCI: Hexyldecanol, Pyrus Malus (Apple) Fruit Extract) erhältlich.
Erfindungsgemäß besonders bevorzugte kosmetische oder dermatologische Zusammensetzungen sind dadurch gekennzeichnet, dass sie den Rohstoff Ederline in Mengen von 0,1 - 10 Gew.-%, bevorzugt 1 - 8 Gew.-% und besonders bevorzugt 3 - 5 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Apfelkernextrakt in Mengen von 0,00001 - 2 Gew.-%, bevorzugt 0,001 - 1,6 Gew.-% und besonders bevorzugt 0,03 - 1 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Lotuskeimen (Nelumbo Nucifera Germ Extract). Derartige Extrakte erhöhen und/oder verbessern die Interaktion zwischen der extrazellulären Matrix und den Fibroblasten. Ein erfindungsgemäß besonders bevorzugter Lotuskeim-Extrakt ist unter der Handelsbezeichnung Lotus Germ Extract mit der INCI-Bezeichnung Water, Butylene Glycol, Nelumbo Nucifera Germ Extract von der Firma Maruzen erhältlich.
Erfindungsgemäß besonders bevorzugte kosmetische oder dermatologische Zusammensetzungen sind dadurch gekennzeichnet, dass sie mindestens einen Extrakt aus Lotuskeimen (Nelumbo Nucifera Germ Extract) in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Lotuskeimen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Rotwein. Derartige Extrakte erhöhen und/ oder verbessern die Interaktion zwischen der extrazellulären Matrix und den Fibroblasten. Ein erfindungsgemäß besonders bevorzugter Rotweinextrakt ist unter dem Handelsnamen Sepivinol R von der Firma Seppic erhältlich.
Erfindungsgemäß besonders bevorzugte kosmetische oder dermatologische Zusammensetzungen sind dadurch gekennzeichnet, dass sie mindestens einen Extrakt aus Rotwein in Mengen von 0,1 - 10 Gew.-%, bevorzugt 1 - 8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Rotwein in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Traubenkernen (Vitis Vinifera (Grape) Seed Extract). Derartige Extrakte erhöhen und/oder verbessern die Interaktion zwischen der extrazellulären Matrix und den Fibroblasten. Besonders bevorzugt stammen die Traubenkernextrakte aus der Chardonnay-Traube. Erfindungsgemäß besonders bevorzugte Traubenkernextrakte sind unter der Handelsbezeichnung Herbalia Grape von der Firma Cognis oder unter der Handelsbezeichnung Crodarom Chardonnay von Croda erhältlich.
Erfindungsgemäß besonders Stiftzusammensetzungen sind dadurch gekennzeichnet, dass sie mindestens einen Extrakt aus Traubenkernen in Mengen von 0,1 - 10 Gew.-%, bevorzugt 1-8 Gew.-% und besonders bevorzugt 2 - 3 Gew.-%, jeweils bezogen auf die gesamte Zusammensetzung, enthalten. Erfindungsgemäß besonders bevorzugte kosmetische oder dermatologische Zusammensetzungen sind dadurch gekennzeichnet, dass sie mindestens einen Extrakt aus Traubenkernen in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Extrakt aus Schwarzen Holunderblüten (Sambucus Nigra Flower Extract). Derartige Extrakte erhöhen und/oder verbessern die Interaktion zwischen der extrazellulären Matrix und den Fibroblasten. Ein erfindungsgemäß besonders bevorzugter Extrakt aus Schwarzen Holunderblüten ist unter der Handelsbezeichnung Sambucus AO von der Firma Alpaflor/Centerchem bzw. von Permcos erhältlich.
Erfindungsgemäß besonders bevorzugte Stiftzusammensetzungen sind dadurch gekennzeichnet, dass sie mindestens einen Extrakt aus Schwarzen Holunderblüten in Mengen von 0,1 - 10 Gew.-%, bevorzugt 1 - 5 Gew.-% und besonders bevorzugt 2 - 3 Gew.-%, jeweils bezogen auf die gesamte Zusammensetzung, enthalten. Erfindungsgemäß besonders bevorzugte Stiftzusammensetzungen sind dadurch gekennzeichnet, dass sie mindestens einen Extrakt aus Schwarzen Holunderblüten in Mengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten Zusammensetzung, enthalten.

In einer weiteren bevorzugten Ausführungsform enthalten die erfindungsgemäßen Zusammensetzungen mindestens einen Wirkstoff, der die beta-Endorphinsynthese in Keratinozyten stimuliert.
Erfindungsgemäß besonders bevorzugte Stimulatoren der beta-Endorphin-Synthese sind ausgewählt aus Mischungen aus mindestens einem Extrakt aus den Blättern der Mentha piperita und mindestens einem Extrakt aus Kakaobohnen, wobei wässrige, glycolische oder wässrig-glycolische Zubereitungen dieser Extraktmischungen, die unter den Handelsbezeichnungen Caomint, Caophenol, Caobromine, Caospice und Caoorange von der Firma Solabia erhältlich sind, besonders bevorzugt sind. Ein weiterer besonders bevorzugter Stimulator der beta-Endorphin-Synthese ist das Dipeptidderivat N-Acetyl-Tyr-Arg-hexyldecylester mit der INCI-Bezeichnung Acetyl Dipeptide-1 Cetyl Ester, das z. B. als wässrige Zubereitung unter der Handelsbezeichnung Calmosensine von der Firma Sederma erhältlich ist.
Weitere bevorzugte Stimulatoren der beta-Endorphin-Synthese sind Extrakte aus Helichrysum italicum, z. B. erhältlich unter der Handelsbezeichnung Areaumat Perpetua von der Firma Codif, Extrakte aus Crithmum Maritimum, z. B. erhältlich unter den Handelsbezeichnungen Areaumat Samphira und Aroleat Samphira von der Firma Codif, Extrakte aus Lavendula stoechas, z. B. erhältlich unter der Handelsbezeichnung Areaumat Lavanda von der Firma Codif, Extrakte aus Mentha piperita, wie sie z. B. unter den Handelsbezeichnungen Authenticals of Peppermint (Solabia) und Calmiskin (Silab) erhältlich sind, Glutamylamidoethyl Indole, z. B. erhältlich unter der Handelsbezeichnung Glistin von der Firma Exsymol, ein durch mikrobielle Fermentation gewonnenes verzweigtes Polysaccharid mit Rhamnose-, Galactose- und Glucuronsäure-Einheiten mit der INCI-Bezeichnung Biosaccharide Gum-2, z. B. erhältlich unter der Handelsbezeichnung Rhamnosoft von der Firma Solabia, Extrakte aus den Samen von Tephrosia Purpurea mit der INCI-Bezeichnung Tephrosia Purpurea Seed Extract, z. B. erhältlich unter der Handelsbezeichnung Tephroline von der Firma Vincience, Mischungen aus dem Öl von Mentha arvensis-Blättern, Limonenschalenöl, Zypressenöl, Lavendelöl und Cistus Ladaniferus-Öl mit der INCI-Bezeichnung Mentha Arvensis Leaf Oil and Citrus Medica Limonum (Lemon) Peel Oil and Cupressus Sempervirens Oil and Lavandula Hybrida Oil and Cistus Ladaniferus Oil, z. B. erhältlich unter der Handelsbezeichnung V-Tonic (Gattefossé), und Hexasaccharide gemäß FR 2842201 sowie beliebige Mischungen dieser Wirkstoffe.

Erfindungsgemäß besonders bevorzugte kosmetische oder dermatologische Zusammensetzungen sind dadurch gekennzeichnet, dass sie mindestens einen Wirkstoff zur Stimulation der beta-Endorphin-Synthese in Gesamtmengen von 0,01 - 10 Gew.-%, bevorzugt 0,1 - 5 Gew.-% und besonders bevorzugt 1 - 3 Gew.-%, jeweils bezogen auf das Handelsprodukt, das den Wirkstoff enthält, in der gesamten erfindungsgemäßen Zusammensetzung, enthalten. Erfindungsgemäß besonders bevorzugte kosmetische oder dermatologische Zusammensetzungen sind dadurch gekennzeichnet, dass sie mindestens einen Wirkstoff zur Stimulation der beta-Endorphin-Synthese in Gesamtmengen von 0,00001 - 1 Gew.-%, bevorzugt 0,0001 - 0,1 Gew.-% und besonders bevorzugt 0,001 - 0,05 Gew.-%, jeweils bezogen auf den Gehalt an Aktivsubstanz in der gesamten erfindungsgemäßen Zusammensetzung, enthalten.

In einer weiteren bevorzugten Ausführungsform enthalten die erfindungsgemäßen Zusammensetzungen mindestens eine anorganische und/oder mindestens eine organische UV-Filtersubstanz.
Bei den UV-Filtersubstanzen handelt es sich um bei Raumtemperatur flüssig oder kristallin vorliegende Substanzen, die in der Lage sind, ultraviolette Strahlen zu absorbieren und die aufgenommene Energie in Form längerwelliger Strahlung, z. B. Wärme, wieder abzugeben. Man unterscheidet UVA-Filter und UVB-Filter. Die UVA- und UVB-Filter können sowohl einzeln als auch in Mischungen eingesetzt werden. Der Einsatz von Filter-Mischungen ist erfindungsgemäß bevorzugt.
Die erfindungsgemäß verwendeten organischen UV-Filter sind ausgewählt aus den physiologisch verträglichen Derivaten von Dibenzoylmethan, Zimtsäureestern, Diphenylacrylsäureestern, Benzophenon, Campher, p-Aminobenzoesäureestern, o-Aminobenzoesäureestern, Salicylsäureestern, Benzimidazolen, symmetrisch oder unsymmetrisch substituierten 1,3,5-Triazinen, monomeren und oligomeren 4,4-Diarylbutadiencarbonsäureestern und -carbonsäureamiden, Ketotricyclo(5.2.1.0)decan, Benzalmalonsäureestern, Benzoxazol sowie beliebigen Mischungen der genannten Komponenten. Die organischen UV-Filter können öllöslich oder wasserlöslich sein. Erfindungsgemäß besonders bevorzugte öllösliche UV-Filter sind 1-(4-tert.-Butylphenyl)-3-(4'-methoxyphenyl)propan-1,3-dion (Parsol® 1789), 1-Phenyl-3-(4'-isopropylphenyl)-propan-1,3-dion, 3-(4'-Methylbenzyliden)-D,L-campher, 4-(Dimethylamino)-benzoesäure-2-ethylhexylester, 4-(Dimethylamino) benzoesäure-2-octylester, 4-(Dimethylamino)-benzoesäureamylester, 4-Methoxyzimtsäure-2-ethylhexylester, 4-Methoxyzimtsäurepropylester, 4-Methoxyzimtsäureisopentylester, 2-Cyano-3,3-phenylzimtsäure-2-ethylhexylester (Octocrylene), Salicylsäure-2-ethylhexylester, Salicylsäure-4-isopropylbenzylester, Salicylsäurehomomenthylester (3,3,5-Trimethyl-cyclohexylsalicylat), 2-Hydroxy-4-methoxybenzophenon, 2-Hydroxy-4-methoxy-4'-methylbenzophenon, 2,2'-Dihydroxy-4-methoxybenzophenon, 4-Methoxybenzmalonsäuredi-2-ethylhexylester, 2,4,6-Trianilino-(p-carbo-2'-ethyl-1'-hexyloxy)-1,3,5-triazin (Octyl Triazone, Uvinul^{®} T 150), Dimethicodiethylbenzal malonate (CAS-Nr. 207574-74-1, Parsol^{®} SLX), Dioctyl Butamido Triazone (Uvasorb^{®} HEB), 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A) und sowie beliebige Mischungen der genannten Komponenten.
Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, Sulfonsäurederivate von Benzophenonen, vorzugsweise 2-Hydroxy-4-methoxybenzophenon-5-sulfonsäure und ihre Salze, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze.
Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4'methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Bei den erfindungsgemäß bevorzugten anorganischen Lichtschutzpigmenten handelt es sich um feindisperse oder kolloiddisperse Metalloxide und Metallsalze, beispielsweise Titandioxid, Zinkoxid, Eisenoxid, Aluminiumoxid, Ceroxid, Zirkoniumoxid, Silicate (Talk) und Bariumsulfat. Die Partikel sollten dabei einen mittleren Durchmesser von weniger als 100 nm, vorzugsweise zwischen 5 und 50 nm und insbesondere zwischen 15 und 30 nm aufweisen, so genannte Nanopigmente. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z. B. Titandioxid T 805 (Degussa) oder Eusolex^{®} T2000 (Merck). Als hydrophobe Coatingmittel kommen dabei vor allem Silicone und dabei speziell Trialkoxyoctylsilane oder Simethicone in Frage. Besonders bevorzugt sind Titandioxid und Zinkoxid.
Erfindungsgemäß besonders bevorzugte kosmetische oder dermatologische Zusammensetzungen sind dadurch gekennzeichnet, dass sie mindestens eine organische UV-Filtersubstanz in einer Gesamtmenge von 0,1 - 30 Gew.-%, bevorzugt 0,5 - 20 Gew.-%, besonders bevorzugt 1,0 - 15 Gew.-% und außerordentlich bevorzugt 3,0 - 10 Gew.-%, jeweils bezogen auf die gesamte Zusammensetzung, enthalten.
Erfindungsgemäß besonders bevorzugte kosmetische oder dermatologische Zusammensetzungen sind dadurch gekennzeichnet, dass sie mindestens eine anorganische UV-Filtersubstanz in einer Gesamtmenge von 0,1 - 15 Gew.-%, bevorzugt 0,5 - 10 Gew.-%, besonders bevorzugt 1,0 - 5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen mindestens einen selbstbräunenden Wirkstoff. Erfindungsgemäß bevorzugte selbstbräunende Wirkstoffe sind ausgewählt aus Dihydroxyaceton, Tyrosin, Tyrosinderivaten, 5,6-Dihydroxyindolin und Erythrulose.
Erfindungsgemäß besonders bevorzugte Stiftzusammensetzungen sind dadurch gekennzeichnet, dass sie mindestens einen selbstbräunenden Wirkstoff in einer Gesamtmenge von 0,1- 15 Gew.-%, bevorzugt 0,5-10 Gew.-%, besonders bevorzugt 1,0-5 Gew.-% und außerordentlich bevorzugt 2,0 - 4,0 Gew.-%, jeweils bezogen auf die gesamte Zusammensetzung, enthalten.

In einer weiteren bevorzugten Ausführungsform enthalten die erfindungsgemäßen Zusammensetzungen mindestens einen hautaufhellenden Wirkstoff. Erfindungsgemäß bevorzugte hautaufhellende Wirkstoffe sind ausgewählt aus Ascorbinsäure, den Estern der Ascorbinsäure mit Phosphorsäure und/oder organischen C₂-C₂₀-Carbonsäuren sowie deren Alkali- und Erdalkalimetallsalzen, Kojisäure, Hydrochinon, Arbutin, Maulbeerbaumextrakt und Süßholzextrakt sowie Mischungen hiervon. Sowohl als Einzelsubstanz wie auch in Mischung bevorzugt sind die Ascorbinsäurederivate sowie Kojisäure bevorzugt. Besonders bevorzugt sind Natriumascorbylphosphat, Magnesiumascorbylphosphat, Ascorbylmonopalmitat, Ascorbyldipalmitat, Ascorbylmonostearat, Ascorbyldistearat, Ascorbylmonoethylhexanoat, Ascorbyldiethylhexanoat, Ascorbylmonooctanoat, Ascorbyldioctanoat, Ascorbylmonoisostearat und Ascorbyldiisostearat. Die erfindungsgemäß außerordentlich bevorzugten Ascorbinsäurederivate sind Natriumascorbylphosphat und Magnesiumascorbylphosphat.
Erfindungsgemäß besonders bevorzugte Stiftzusammensetzungen sind dadurch gekennzeichnet, dass sie mindestens einen hautaufhellenden Wirkstoff in einer Gesamtmenge von 0,05 bis 5 Gew.-%, bevorzugt von 0,1 - 2 Gew-%, jeweils bezogen auf die gesamte Zusammensetzung, enthalten.

In einer weiteren bevorzugten Ausführungsform enthalten die erfindungsgemäßen Zusammensetzungen mindestens einen Wirkstoff, der die Prostaglandinsynthese und/oder die Leukotrien-Synthese inhibiert.
Bevorzugte Wirkstoffe, die die Prostaglandinsynthese inhibieren, sind ausgewählt aus Wirkstoffen, die das Enzym Cyclooxygenase inhibieren und Wirkstoffen, die die Ausschüttung von Interleukinen, insbesondere von Interleukin-1-alpha, inhibieren. Im Sinne der vorliegenden Erfindung kann unter der Inhibierung der Cyclooxygenase sowohl eine Senkung der Menge dieses Enzyms als auch eine Minderung seiner Aktivität sowie beides hiervon verstanden werden.
Bevorzugte Wirkstoffe, die die Leukotrien-Synthese inhibieren, sind ausgewählt aus Wirkstoffen, die das Enzym 5-Lipoxygenase inhibieren. Im Sinne der vorliegenden Erfindung kann unter der Inhibierung der 5-Lipoxygenase sowohl eine Senkung der Menge dieses Enzyms als auch eine Minderung seiner Aktivität sowie beides hiervon verstanden werden.
Erfindungsgemäß bevorzugte Inhibitoren der Prostaglandin-Synthese, insbesondere Inhibitoren der Cyclooxygenase und/oder der Interleukin-Ausschüttung, sind ausgewählt aus Silymarin, das besonders bevorzugt in liposomenverkapselter Form eingesetzt wird (erhältlich z. B. unter der Handelsbezeichnung Silymarin Phytosome (INCI: Silybum Marianum Extract and Phospholipids) von der Firma Indena SpA. Silymarin stellt ein früher als einheitliche Substanz angesehenes Wirkstoff-Konzentrat aus den Früchten der Mariendistel (Silybum marianum) dar. Die Hauptbestandteile des Silymarins sind Silybin (Silymarin I), Silychristin (Silymarin II) und Silydianin, die zur Gruppe der Flavanolignane gehören. Weitere erfindungsgemäß bevorzugte Inhibitoren der Prostagladin-Synthese, insbesondere Inhibitoren der Cyclooxygenase und/oder der Interleukin-Ausschüttung, sind ausgewählt aus Extrakten aus Centella asiatica, beispielsweise erhältlich unter der Bezeichnung Madecassicoside von DSM, Glycyrrethinsäure, die besonders bevorzugt in Liposomen verkapselt vorliegt und in dieser Form z. B. unter der Handelsbezeichnung Calmsphere von Soliance erhältlich ist, Mischungen aus Getreidewachsen, Extrakten aus Schibutter und Argania Spinosa-Öl mit der INCI-Bezeichnung "Spent grain wax and butyrospermum parkii (shea butter) extract and Argania Spinosa Kernel Oil", wie sie z. B. unter der Handelsbezeichnung Stimu-Tex AS von der Firma Pentapharm erhältlich sind, Extrakten aus Vanilla Tahitensis, wie sie z. B. unter der Handelsbezeichnung Vanirea (INCI : Vanilla Tahitensis Fruit Extract) von der Firma Solabia erhältlich sind, Extrakten aus Olivenblättern (INCI: Olea Europaea (Olive) Leaf Extract), wie sie insbesondere unter der Handelsbezeichnung Oleanoline DPG von der Firma Vincience erhältlich sind, weiterhin Alginhydrolysaten, wie sie z. B. unter der Handelsbezeichnung Phycosaccharide, insbesondere Phycosaccharide Al, von der Firma Codif erhältlich sind, Extrakten aus Bacopa Monniera, wie sie z. B. unter der Handelsbezeichnung Bacocalmine von der Firma Sederma erhältlich sind, Extrakten aus der Rooibos-Pflanze, wie sie z. B. unter der Handelsbezeichnung Rooibos Herbasec MPE von der Firma Cosmetochem erhältlich sind, den physiologisch verträglichen Salzen von Sterolsulfaten, wie sie z. B. unter der Handelsbezeichnung Phytocohesine (INCI: Sodium Beta-Sitosterylsulfate) von der Firma Vincience erhältlich sind, sowie beliebigen Mischungen dieser Wirkstoffe.

Erfindungsgemäß besonders bevorzugte kosmetische oder dermatologische Zusammensetzungen sind dadurch gekennzeichnet, dass sie mindestens einen Inhibitor der Prostaglandin-Synthese in einer Gesamtmenge von 0,0001- 10,0 Gew.-%, bevorzugt 0,001- 2,0 Gew.-%, besonders bevorzugt 0,05 - 1,0 Gew.-% und außerordentlich bevorzugt 0,1- 0,5 Gew.-%, jeweils bezogen auf die gesamte Zusammensetzung, enthalten.

Erfindungsgemäß bevorzugte Inhibitoren der Leukotrien-Synthese, insbesondere Inhibitoren der 5-Lipoxygenase, sind ausgewählt aus Alginhydrolysaten, Aminodicarbonsäuren mit einer. C-Kettenlänge von 3-6 Kohlenstoffatomen sowie deren physiologisch verträglichen Salzen, N-alkylierten C₂-C₁₁-Aminosäuren mit C₁-C₂₂-Alkylresten sowie deren physiologisch verträglichen Salzen, N-acylierten C₂-C₁₁-Aminosäuren mit C₂-C₂₂-Acylresten sowie deren physiologisch verträglichen Salzen, Hefeextrakten, α-Bisabolol, α-Liponsäure, Allantoin sowie beliebigen Mischungen dieser Wirkstoffe.

In einer bevorzugten Ausführungsform sind die erfindungsgemäßen Alginhydrolysate ausgewählt aus den Produkten, die z. B. unter der Handelsbezeichnung Phycosaccharide, insbesondere Phycosaccharide Al, von der Firma Codif erhältlich sind.

In einer weiteren bevorzugten Ausführungsform sind die erfindungsgemäß bevorzugten Aminodicarbonsäuren mit einer C-Kettenlänge von 3-6 Kohlenstoffatomen ausgewählt aus Aminomalonsäure, Aminobernsteinsäure (= Asparaginsäure), Aminoglutarsäure und Aminoadipinsäure sowie deren physiologisch verträglichen Salzen. Besonders bevorzugt sind Asparaginsäure und ihre physiologisch verträglichen Salze, insbesondere Kaliumaspartat und Magnesiumaspartat.
Erfindungsgemäß bevorzugt werden die Aminodicarbonsäuren mit einer C-Kettenlänge von 3 - 6 Kohlenstoffatomen sowie deren Salze in Mengen von 0,01 - 5 Gew.-%, bevorzugt 0,1 - 2 Gew.-% und besonders bevorzugt 0,5 - 1 Gew.-%, jeweils bezogen auf die gesamte erfindungsgemäße Zusammensetzung, eingesetzt.

In einer weiteren bevorzugten Ausführungsform sind die erfindungsgemäß bevorzugten N-alkylierten C₂-C₁₁-Aminosäuren mit einem C₁-C₂₂-Alkylrest ausgewählt aus Alanin, Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin sowie deren physiologisch verträglichen Salzen, die am Stickstoffatom der Aminogruppe einen C₁-C₂₂-Alkylrest, ausgewählt aus einer Gruppe Methyl, Ethyl, Propyl, Butyl, Pentyl, Hexyl, Heptyl, Octyl, Nonyl, Decyl, Undecyl, Dodecyl (Lauryl), Tridecyl, Tetradecyl (Myristyl), Pentadecyl, Hexadecyl (Palmityl, Cetyl), Heptadecyl, Octadecyl (Stearyl), Nonadecyl, Eicosanyl (Arachidyl) und Behenyl, aufweisen. Besonders bevorzugt ist N-Methylglycin (= Sarcosin).
Erfindungsgemäß bevorzugt werden die N-alkylierten C₂-C₁₁-Aminosäuren mit einem C₁-C₂₂-Alkylrest sowie deren physiologisch verträglichen Salze in Mengen von 0,01 bis 10 Gew.-%, bevorzugt 0,1 bis 5 Gew.-% und besonders bevorzugt 0,5 bis 2 Gew.-%, jeweils bezogen auf die gesamte erfindungsgemäße Zusammensetzung, eingesetzt.

In einer weiteren bevorzugten Ausführungsform sind die erfindungsgemäß bevorzugten N-acylierten C₂-C₁₁-Aminosäuren mit einem C₂-C₂₂-Acylrest ausgewählt aus Glutaminsäure, Pyroglutaminsäure, Lysin, Arginin, Histidin, Valin, Leucin, Isoleucin, Prolin, Tryptophan, Phenylalanin, Methionin, Glycin, Serin, Tyrosin, Threonin, Cystein, Asparagin und Glutamin sowie deren physiologisch verträglichen Salzen. Die Aminosäuren können einzeln oder im Gemisch eingesetzt werden. Erfindungsgemäß geeignet sind insbesondere Aminosäuren-Gemische, die aus Pflanzen, insbesondere Getreidepflanzen, gewonnen wurden. Der C₂ - C₂₂-Acylrest, mit dem die genannten Aminosäuren an der Aminogruppe derivatisiert sind, ist ausgewählt aus einem Acetyl-, Propanoyl-, Butanoyl-, Pentanoyl-, Hexanoyl-, Heptanoyl-, Octanoyl-, Nonanoyl-, Decanoyl-, Undecanoyl-, Lauroyl-, Tridecanoyl-, Myristoyl-, Pentadecanoyl-, Cetoyl-, Palmitoyl-, Stearoyl-, Arachidoyl- oder Behenoyl-Rest. Mischungen von C₈-C₁₈-Acylresten werden auch als Cocoyl-Rest bezeichnet und sind ebenfalls bevorzugte Substituenten. Besonders bevorzugt sind Natriumcocoylaminosäuren, Natriumoctanoylglutamat, Natriumdecanoylglutamat, Natriumlauroylglutamat, Natriummyristoylglutamat, Natriumcetoylglutamat und Natriumstearoylglutamat und die Lauroyl-Derivate von aus Getreidepflanzen gewonnenen Aminosäuren.
Die Getreidepflanzen, aus denen die erfindungsgemäß geeigneten Aminosäuren gewonnen werden, unterliegen keiner Einschränkung. Geeignet sind beispielsweise Hafer, Weizen, Gerste und Roggen; besonders geeignet ist Hafer.
Ein besonders bevorzugter 5-Lipoxygenase-Inhibitor ist das Handelsprodukt Seppicalm von der Firma Seppic mit der INCI-Bezeichnung "Sodium Cocoyl Aminoacids, Sarcosine, Potassium Aspartate, Magnesium Aspartate".
Erfindungsgemäß bevorzugt werden die N-acylierten C₂-C₁₁-Aminosäuren mit einem C₂-C₂₂-Acylrest sowie deren physiologisch verträglichen Salze in Mengen von 0,01 - 10 Gew.-%, bevorzugt 0,1 - 5 Gew.-% und besonders bevorzugt 0,5 - 2 Gew.-%, jeweils bezogen auf die gesamte topische Zusammensetzung, eingesetzt.

In einer weiteren bevorzugten Ausführungsform werden die erfindungsgemäß als 5-Lipoxygenase-Inhibitoren bevorzugten Hefeextrakte in Mengen von 0,001 - 5 Gew.-%, bevorzugt 0,01 - 2 Gew.-% und besonders bevorzugt 0,1 - 1 Gew.-%, jeweils bezogen auf den Extrakt tel quel in der gesamten erfindungsgemäßen Zusammensetzung, eingesetzt. Ein besonders bevorzugt verwendetes Handelsprodukt ist Drieline (INCI-Bezeichnung "Sorbitol, Yeast Extract"), erhältlich von der Firma Lanatech.

In einer weiteren bevorzugten Ausführungsform wird der erfindungsgemäß bevorzugte 5-Lipoxygenase-Inhibitor α-Bisabolol in Mengen von 0,001 bis 5 Gew.-%, bevorzugt 0,01 bis 2 Gew.-% und besonders bevorzugt 0,1 bis 1 Gew.-%, jeweils bezogen auf die gesamte erfindungsgemäße Stiftzusammensetzung, eingesetzt.

In einer weiteren bevorzugten Ausführungsform wird der erfindungsgemäß bevorzugte 5-Lipoxygenase-Inhibitor α-Liponsäure in Mengen von 0,001 - 5 Gew.-%, bevorzugt 0,01 - 2 Gew.-% und besonders bevorzugt 0,1 - 1 Gew.-%, jeweils bezogen auf die gesamte erfindungsgemäße Stiftzusammensetzung, eingesetzt.

In einer weiteren bevorzugten Ausführungsform wird der erfindungsgemäß bevorzugte 5-Lipoxygenase-Inhibitor Allantoin in Mengen von 0,001 - 5 Gew.-%, bevorzugt 0,01 - 2 Gew.-% und besonders bevorzugt 0,1 - 1 Gew.-%, jeweils bezogen auf die gesamte erfindungsgemäße Stiftzusammensetzung, eingesetzt.

In einer weiteren bevorzugten Ausführungsform sind die erfindungsgemäß als 5-Lipoxygenase-Inhibitoren bevorzugten physiologisch verträglichen Salze der Sterolsulfate ausgewählt aus den Salzen von β-Sitosterolsulfat, Ergosterolsulfat, Stigmasterolsulfat, Cholesterolsulfat und Lanosterolsulfat. Besonders bevorzugt sind die Salze von β-Sitosterolsulfat. Die Sterolsulfatsalze werden in Mengen von 0,001 - 5 Gew.-%, bevorzugt 0,01 - 2 Gew.-% und besonders bevorzugt 0,1 - 1 Gew.-%, jeweils bezogen auf die gesamte erfindungsgemäße Stiftzusammensetzung, eingesetzt. Die Sterolsulfatsalze können dabei sowohl einzeln als auch in beliebigen Mischungen eingesetzt werden. Ein besonders bevorzugt eingesetztes Handelsprodukt ist Phytocohesine (INCI-Bezeichnung "Sodium Beta-Sitosteryl Sulfate"), erhältlich von der Firma Vincience.

Die physiologisch verträglichen Salze der vorgenannten 5-Lipoxygenase-Inhibitoren sind ausgewählt aus den Ammonium-, Alkalimetall-, Magnesium-, Calcium-, Aluminium-, Zink- und Mangan-Salzen. Bevorzugt sind die Natrium-, Kalium-, Magnesium-, Aluminium-, Zink- und Mangan-Salze.

Erfindungsgemäß besonders bevorzugte kosmetische oder dermatologische Zusammensetzungen sind dadurch gekennzeichnet, dass sie mindestens einen Inhibitor der Leukotrien-Synthese in einer Gesamtmenge von 0,0001 - 10,0 Gew.-%, bevorzugt 0,001 - 2,0 Gew.-%, besonders bevorzugt 0,05 - 1,0 Gew.-% und außerordentlich bevorzugt 0,1 - 0,5 Gew.-%, jeweils bezogen auf die gesamte Zusammensetzung, enthalten.

In einer weiteren bevorzugten Ausführungsform enthalten die erfindungsgemäßen Zusammensetzungen mindestens einen sebumregulierenden Wirkstoff. Erfindungsgemäß bevorzugte sebumregulierende Wirkstoffe sind ausgewählt aus 10-Hydroxydecansäure, Sebacinsäure, Azelainsäure und den Estern der Azelainsäure, insbesondere Kaliumazeloyldiglycinat, 1,10-Decandiol und mindestens einem Extrakt aus Spiraea Ulmaria sowie Mischungen der vorgenannten Substanzen. Bevorzugte Mischungen sind beispielsweise erhältlich als Handelsprodukt Achacidol PG (Propylene Glycol, 10-Hydroxydecanoic acid, Sebacic acid, 1,10-Decandiol) von Vincience erhältlich. Ein bevorzugter Extrakt aus Spiraea Ulmaria ist z. B. im Produkt Seboregul 2 der Firma Silab enthalten. Kaliumazeloyldiglycinat ist z. B. in dem Produkt Azeloglicina der Firma Sinerga enthalten. Erfindungsgemäß besonders bevorzugte kosmetische oder dermatologische Zusammensetzungen sind dadurch gekennzeichnet, dass sie mindestens einen sebumregulierenden Wirkstoff in Gesamtmengen von 0,00001 bis 10 Gew.-%, bevorzugt 0,01 bis 5 Gew.-% und besonders bevorzugt 0,1 bis 1 -2 Gew.-%, jeweils bezogen auf Aktivsubstanz in der gesamten erfindungsgemäßen Zusammensetzung, enthalten.

Weitere erfindungsgemäß besonders bevorzugte Stifzusammensetzungen sind dadurch gekennzeichnet, dass sie mindestens einen hyperämisierenden Wirkstoff enthalten.
Erfindungsgemäß bevorzugte hyperämisierende Wirkstoffe sind ausgewählt aus
- Nicotinsäureestern, insbesondere Nicotinsäurebenzylester (Benzylnicotinat), Nicotinsäurebutoxyethylester (Nicoboxil), Nicotinsäuremethylester, Nicotinsäureethylester, Nicotinsäurehexylester, Nicotinsäureisopropylester und Nicotinsäuremyristylester,
- Pyridin-3-carbaldehyd (β-Pyridincarbaldehyd, Nicotinaldehyd),
- Salicylsäureestern, insbesondere Phenylsalicylat,
- Vanillylethern, insbesondere Vanillylbutylether,
- Carbonsäurevanillylamiden, insbesondere Nonivamid,
- Scharfstoffen, insbesondere Capsaicin, Cantharidin, Piperin, Gingerol, Zingeron, Myristicin, Safrol, Allicin, Sedamin, Sacculatal, Chalciporon, Isochalciporon, Velleral, Vellerol, und Isothiocyanaten (Senfölen), insbesondere Benzylsenföl, aber auch Allylsenföl (Allylisothiocyanat), 3-Methylthiopropylsenföl, Butylsenföl, 3-Butenylsenföl, Erucin, Erysolin, Hirsutin, Isopropylsenföl, β-Phenylethylsenföl, Sulforaphan und Sulforaphen (Raphanin),
- Extrakten aus Scharfstoff-Pflanzen, insbesondere aus Capsicum, insbesondere aus Capsicum annuum und Capsicum frutescens, aber auch aus Capsicum chinense, Capsicum baccatum und Capsicum pubescens, weiterhin aus Mauerpfeffer und aus der Zaunrübe,
- Terpentinöl,
- sowie Mischungen hiervon.

Erfindungsgemäß bevorzugte hyperämisierende Wirkstoffe sind im Handel in für kosmetische oder dermatologische Zusammensetzungen aufbereiteter Form erhältlich. Erfindungsgemäß außerordentlich bevorzugte Wirkstoffe sind Ethylnicotinat, Vanillylbutylether, z.B. der Rohstoff Hotact VBE von Takasago, Capsaicin, erhältlich z. B. als Rohstoff Herbasol Extract Capsicum (INCI: Propylene Glycol, Aqua (Water), Capsicum Frutescens Fruit Extract, Sorbitol) und N-Vanillylnonamid (Pseudocapsaicin).
Bevorzugte erfindungsgemäße Stiftzusammensetzungen sind dadurch gekennzeichnet, dass sie mindestens einen hyperämisierenden Wirkstoff in einer Aktivsubstanz-Gesamtmenge von 0,005 - 3,0 Gew.-%, bevorzugt 0,01 - 1,0, besonders bevorzugt 0,05 - 0,5 Gew.-% und außerordentlich bevorzugt 0,1 - 0,35 Gew.-%, jeweils bezogen auf das Gesamtgewicht der erfindungsgemäßen Stiftzusammensetzung, enthalten.

Weitere erfindungsgemäß besonders bevorzugte Stifzusammensetzungen sind dadurch gekennzeichnet, dass sie mindestens einen feuchtigkeitsspendenden Wirkstoff enthalten. Erfindungsgemäß bevorzugte feuchtigkeitsspendende Wirkstoffe sind ausgewählt aus Desoxyzuckern, besonders bevorzugt Rhamnose und Fucose, Polysacchariden, die mindestens einen Desoxyzucker-Baustein enthalten, besonders bevorzugt aus den Handelsprodukten Fucogel^{®} (INCI-Bezeichnung Biosaccharide Gum-1) von Solabia, Rhamnosoft^{®} (INCI-Bezeichnung Biosaccharide Gum-2) von Solabia, Fucogenol^{®} (INCI-Bezeichnung Biosaccharide Gum-3) von Solabia und Glycofilm^{®} (INCI-Bezeichnung Biosaccharide Gum-4) von Solabia, weiterhin Mischungen der vorgenannten, mindestens einen Desoxyzucker-Baustein enthaltenden Polysaccharide, beispielsweise der Mischung aus Biosaccharide Gum-2 und Biosaccharide Gum-3, erhältlich als Handelsprodukt Elastinol plus^{®} von Solabia, weiterhin Harnstoff, weiterhin alkyl- oder hydroxyalkylsubstituierten Harnstoff der allgemeinen Formel (UREA), 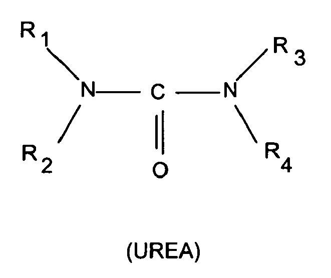 in der R₁, R₂, R₃ und R₄ unabhängig voneinander für ein Wasserstoffatom, eine Methyl-, Ethyl-, n-Propyl-, Isopropyl-, n-Butyl-, sek.-Butyl-, tert. Butyl- oder C₂-C₆-Hydroxyalkyl-Gruppe, die mit 1 bis 5 Hydroxylgruppen oder C₁-C₄-Hydroxyalkyl-Gruppen substituiert ist, stehen, mit der Maßgabe, dass mindestens einer der Reste R₁ - R₄ eine C₂-C₆-Hydroxyalkyl-Gruppe, die mit 1 bis 5 Hydroxylgruppen oder C₁-C₄-Hydroxyalkyl-Gruppen substituiert ist, darstellt, insbesondere (2-Hydroxyethyl)harnstoff und N,N'-Bis(2-hydroxyethyl)harnstoff, Betain (Me₃N⁺-CH₂-COO⁻), Chitosanen, Glycosaminoglycanen, besonders bevorzugt Hyaluronsäure, Dextran, Dextransulfat, Chondroitin-4-sulfat und Chondroitin-6-sulfat, sowie beliebigen Mischungen dieser Substanzen, insbesondere Mischungen aus N,N'-Bis(2-hydroxyethyl)harnstoff und Harnstoff sowie Mischungen aus N,N'-Bis(2-hydroxyethyl)harnstoff, Harnstoff und Lactaten, insbesondere Ammoniumlactat.

Erfindungsgemäß besonders bevorzugte Stiftzusammensetzungen sind dadurch gekennzeichnet, dass sie mindestens einen feuchtigkeitsspendenden Wirkstoff in einer Gesamtmenge von 0,001 - 10 Gew.-%, besonders bevorzugt 0,01 - 5 Gew.-% und außerordentlich bevorzugt 0,1 - 1 oder 2 Gew.-%, jeweils bezogen auf die gesamte Zusammensetzung, enthalten.

Weitere erfindungsgemäß besonders bevorzugte Stiftzusammensetzungen sind dadurch gekennzeichnet, dass sie mindestens einen antimikrobiellen Wirkstoff enthalten.
Unter keimhemmenden oder antimikrobiellen Wirkstoffen werden erfindungsgemäß solche Wirkstoffe verstanden, die die Zahl von Mikroben, insbesondere von Hautkeimen, reduzieren bzw. deren Wachstum hemmen. Zu diesen Keimen zählen unter anderem verschiedene Spezies aus der Gruppe der Staphylokokken, der Gruppe der Corynebakterien, Anaerokokken und Mikrokokken.
Als keimhemmende oder antimikrobielle Wirkstoffe erfindungsgemäß bevorzugt sind insbesondere Organohalogenverbindungen sowie -halogenide, quartäre Ammoniumverbindungen, eine Reihe von Pflanzenextrakten und Zinkverbindungen. Hierzu zählen u. a. Triclosan, Chlorhexidin und Chlorhexidingluconat, 3,4,4'-Trichlorcarbanilid, Bromchlorophen, Dichlorophen, Chlorothymol, Chloroxylenol, Hexachlorophen, Dichloro-m-xylenol, Dequaliniumchlorid, Domiphenbromid, Ammoniumphenolsulfonat, Benzalkoniumhalogenide, Benzalkoniumcetylphosphat, Benzalkoniumsaccharinate, Benzethoniumchlorid, Cetylpyridiniumchlorid, Laurylpyridiniumchlorid, Laurylisoquinoliniumbromid, Methylbenzedoniumchlorid. Weiterhin sind Phenol, Phenoxyethanol, Dinatriumdihydroxyethylsulfosuccinylundecylenat, Natriumbicarbonat, Zinklactat, Natriumphenolsulfonat und Zinkphenolsulfonat, Ketoglutarsäure, Terpenalkohole wie z. B. Farnesol, Chlorophyllin-Kupfer-Komplexe, α-Monoalkylglycerinether mit einem verzweigten oder linearen gesättigten oder ungesättigten, gegebenenfalls hydroxylierten C₆ - C₂₂-Alkylrest, besonders bevorzugt α-(2-Ethylhexyl)glycerinether, im Handel erhältlich als Sensiva^{®} SC 50 (ex Schülke & Mayr), Carbonsäureester des Mono-, Di- und Triglycerins (z. B. Glycerinmonolaurat, Diglycerinmonocaprinat), Lantibiotika sowie Pflanzenextrakte (z. B. grüner Tee und Bestandteile des Lindenblütenöls) einsetzbar.
Weitere bevorzugte antimikrobielle Wirkstoffe sind ausgewählt aus den keimhemmend wirkenden Parfümölen und den Deosafe-Parfümölen, die von der Firma Symrise, vormals Haarmann und Reimer, erhältlich sind.
Weitere erfindungsgemäß besonders bevorzugte Stiftzusammensetzungen sind dadurch gekennzeichnet, dass sie mindestens einen antimikrobiellen Wirkstoff in einer Gesamtmenge von 0,01 - 10 Gew.-%, bevorzugt 0,1 - 5 Gew.-% und besonders bevorzugt 0,5 - 2 Gew.-%, enthalten.
Weitere erfindungsgemäß besonders bevorzugte Stiftzusammensetzungen sind dadurch gekennzeichnet, dass sie mindestens einen präbiotischen Wirkstoff enthalten.

Unter präbiotischen Wirkstoffen sind erfindungsgemäß solche Komponenten zu verstehen, die nur oder zumindest überwiegend unerwünschte Keime der Hautmikroflora hemmen, nicht aber die erwünschten, das heißt, die Keime, die zu einer gesunden Hautmikroflora gehören. Explizit sind hier die Wirkstoffe, die in den Offenlegungsschriften DE 10333245 und DE 10 2004 011 968 als präbiotisch wirksam offenbart sind, mit einbezogen; dazu gehören Nadelbaumextrakte, insbesondere aus der Gruppe der Pinaceae, und Pflanzenextrakte aus der Gruppe der Sapindaceae, Araliaceae, Lamiaceae und Saxifragaceae, insbesondere Extrakte aus *Picea spp., Paullinia sp., Panax sp., Lamium album* oder *Ribes nigrum* sowie Mischungen dieser Substanzen.
Weitere erfindungsgemäß besonders bevorzugte Stiftzusammensetzungen sind dadurch gekennzeichnet, dass sie mindestens einen präbiotischen Wirkstoff in einer Gesamtmenge von 0,01 - 10 Gew.-%, bevorzugt 0,1 - 5 Gew.-% und besonders bevorzugt 0,5 - 2 Gew.-%, enthalten.

Weitere erfindungsgemäß besonders bevorzugte Stiftzusammensetzungen sind dadurch gekennzeichnet, dass sie mindestens ein färbendes, farbgebendes, mattierendes oder glanzgebendes Pigment enthalten. Bevorzugte Pigmente dieser Art können anorganisch oder organisch sein. Weitere bevorzugte Pigmente weisen einen mittleren Teilchendurchmesser von 0,1 - 200 µm, bevorzugt 0,5 - 100 µm, besonders bevorzugt 1 - 50 µm und außerordentlich bevorzugt 2 - 30 µm auf. Besonders bevorzugte anorganische Pigmente sind ausgewählt aus den Oxiden von Silicium, Titan, Eisen, Zink, Zirkonium, Magnesium, Cer und Bismut, aus Bismutoxychlorid, Bornitrid, Glimmer, Flussspat und wasserunlöslichen Perlglanzpigmenten, die mit mindestens einer anorganischen und/oder organischen Verbindung beschichtet sein können.
Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Besonders bevorzugte Farbpigmente sind beispielsweise Titandioxid, Glimmer, Eisenoxide (z. B. Fe₂O₃, Fe₃O₄, FeO(OH)) und/oder Zinnoxid. Besonders bevorzugte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

| **Chemische oder sonstige Bezeichnung** | **CIN** | **Farbe** |
|---|---|---|
| Pigment Green | 10006 | grün |
| Acid Green 1 | 10020 | grün |
| 2,4-Dinitrohydroxynaphthalin-7- sulfosäure | 10316 | gelb |
| Pigment Yellow 1 | 11680 | gelb |
| Pigment Yellow 3 | 11710 | gelb |
| Pigment Orange 1 | 11725 | orange |
| 2,4-Dihydroxyazobenzol | 11920 | orange |
| Solvent Red 3 | 12010 | rot |
| 1-(2'-Chlor-4'-nitro- 1'-phenylazo)-2-hydroxynaphthalin | 12085 | rot |
| Pigment Red 3 | 12120 | rot |
| Ceresrot; Sudanrot; Fettrot G | 12150 | rot |
| Pigment Red 112 | 12370 | rot |
| Pigment Red 7 | 12420 | rot |
| Pigment Brown 1 | 12480 | braun |
| 4-(2'- Methoxy-5'-sulfosäurediethylamid-1'-phenylazo)-3-hydroxy-5"-chloro-2",4"- dimethoxy-2-naphthoesäureanilid | 12490 | rot |
| Disperse Yellow 16 | 12700 | gelb |
| 1-(4-Sulfo-1- phenylazo)-4-amino-benzol-5-sulfosäure | 13015 | gelb |
| 2,4-Dihydroxy-azobenzol-4'-sulfosäure | 14270 | orange |
| 2-(2,4-Dimethylphenylazo-5-suffosäure)-1-hydroxynaphthalin-4- sulfosäure | 14700 | rot |
| 2-(4-Sulfo-1-naphthylazo) -1-naphthol-4-sulfosäure | 14720 | rot |
| 2-(6-Sulfo- 2,4-xylylazo)-1-naphthol-5-sulfosäure | 14815 | rot |
| 1-(4'-Sulfophenylazo)-2-hydroxynaphthalin | 15510 | orange |
| 1-(2-Sulfosäure-4-chlor-5-carbonsäure-1-phenylazo)-2- hydroxynaphthalin | 15525 | rot |
| 1-(3-Methyl- phenylazo-4-sulfosäure)-2-hydroxynaphthalin | 15580 | rot |
| 1-(4',(8')-Sulfosäurenaphthylazo)-2-hydroxynaphthalin | 15620 | rot |
| 2-Hydroxy-1,2'-azonaphthalin-1'- sulfosäure | 15630 | rot |
| 3-Hydroxy-4-phenylazo-2- naphthylcarbonsäure | 15800 | rot |
| 1-(2-Sulfo-4- methyl-1-phenylazo)-2-naphthylcarbonsäure | 15850 | rot |
| 1-(2-Sulfo-4-methy)-5-chlor-1-phenylazo)-2-hydroxy-naphthalin-3-carbonsäure | 15865 | rot |
| 1-(2-Sulfo-1- naphthylazo)-2-hydroxynaphthalin-3-carbonsäure | 15880 | rot |
| 1-(3-Sulfo-1 -phenylazo)-2-naphthol-6-sulfosäure | 15980 | orange |
| 1-(4-Sulfo-1-phenylazo)-2-naphthol-6- sulfosäure | 15985 | gelb |
| Allura Red | 16035 | rot |
| 1-(4-Sulfo-1-naphthylazo)-2-naphthol-3,6- disulfosäure | 16185 | rot |
| Acid Orange 10 | 16230 | orange |
| 1-(4-Sulfo-1-naphthylazo)-2-naphthol- 6,8-disulfosäure | 16255 | rot |
| 1-(4-Sulfo-1- naphthylazo)-2-naphthol-3,6,8-trisulfosäure | 16290 | rot |
| 8-Amino-2 -phenylazo- 1 -naphthol-3,6-disulfosäure | 17200 | rot |
| Acid Red 1 | 18050 | rot |
| Acid Red 155 | 18130 | rot |
| Acid Yellow 121 | 18690 | gelb |
| Acid Red 180 | 18736 | rot |
| Acid Yellow 11 | 18820 | gelb |
| Acid Yellow 17 | 18965 | gelb |
| 4-(4- Sulfo-1-phenylazo)-1-(4-sulfophenyl)-5-hydroxy-pyrazolon-3- carbonsäure | 19140 | gelb |
| Pigment Yellow 16 | 20040 | gelb |
| 2,6-(4'-Sulfo-2", 4"-dimethyl)-bis- phenylazo)1,3-dihydroxybenzol | 20170 | orange |
| Acid Black 1 | 20470 | schwarz |
| Pigment Yellow 13 | 21100 | gelb |
| Pigment Yellow 83 | 21108 | gelb |
| Solvent Yellow | 21230 | gelb |
| Acid Red 163 | 24790 | rot |
| Acid Red 73 | 27290 | rot |
| 2-[4'-(4"-Sulfo-1 "-phenylazo)- 7'-sulfo-1'-naphthylazo]-1-hydroxy-7-amino-naphthalin-3,6-disulfosäure | 27755 | schwarz |
| 4'-[(4"-Sulfo-1"-phenylazo)-7'- sulfo-1'-naphthylazo]-1-hydroxy-8-acetyl-aminonaphthalin-3,5- disulfosäure | 28440 | schwarz |
| Direct Orange 34, 39, 44, 46, 60 | 40215 | orange |
| Food Yellow | 40800 | orange |
| trans-β-Apo-8'-Carotinaldehyd (C₃₀) | 40820 | orange |
| trans-Apo-8'- Carotinsäure (C₃₀)-ethylester | 40825 | orange |
| Canthaxanthin | 40850 | orange |
| Acid Blue 1 | 42045 | blau |
| 2,4-Disulfo-5-hydroxy-4'-4"- bis-(diethylamino)triphenyl-carbinol | 42051 | blau |
| 4-[(-4-N-Ethyl-p-sulfobenzylamino)-phenyl-(4-hydroxy-2-sulfophenyl)-(methylen)-1-(N-ethylN-p-sulfobenzyl)-2,5-cyclohexadienimin] | 42053 | grün |
| Acid Blue 7 | 42080 | blau |
| (N-Ethyl-p-sulfobenzyl-amino)-phenyl-(2-sulfophenyl)-methylen-(N-ethyl-N-p-sulfo-benzyl)-2,5-cyclohexadienimin | 42090 | blau |
| Acid Green 9 | 42100 | grün |
| Diethyl-di-sulfobenzyl-di-4-amino-2-chlor-di-2-methyl-fuchsonimmonium | 42170 | grün |
| Basic Violet 14 | 42510 | violett |
| Basic Violet 2 | 42520 | violett |
| 2'-Methyl-4'-(N-ethyl-N-m- sulfobenzyl)-amino-4"-(N-diethyl)-amino-2-methyl-N-ethylN-m-sulfobenzyl-fuchsonimmonium | 42735 | blau |
| 4'-(N- Dimethyl)-amino-4"-(N-phenyl)-aminonaphtho-N-dimethyl-fuchson-immonium | 44045 | blau |
| 12-Hydroxy-3,6-disulfo-4,4'-bis- dimethylamino-naphthofuchsonimmonium | 44090 | grün |
| Acid Red 52 | 45100 | rot |
| 3-(2'-Methylphenylamino)-6-(2'-methyl-4'-sulfophenylamino)-9- (2"-carboxy-phenyl)-xantheniumsalz | 45190 | violett |
| Acid Red 50 | 45220 | rot |
| Phenyl-2- oxyfluoron-2-carbonsäure | 45350 | gelb |
| 14,5- Dibromfluorescein | 45370 | orange |
| 2,4,5,7- Tetrabromfluorescein | 45380 | rot |
| Solvent Dye | 45396 | orange |
| Acid Red 98 | 45405 | rot |
| 3',4',5',6'-Tetrachlor-2,4,5,7- tetrabromfluorescein | 45410 | rot |
| 4,5- Diiodfluorescein | 45425 | rot |
| 2,4,5,7- Tetraiodfluorescein | 45430 | rot |
| Chinophthalon | 47000 | gelb |
| Chinophthalon-disulfonsäure | 47005 | gelb |
| Acid Violet 50 | 50325 | violett |
| Acid Black 2 | 50420 | schwarz |
| Pigment Violet 23 | 51319 | violett |
| 1,2-Dioxyanthrachinon, Calcium-Aluminiumkomplex | 58000 | rot |
| 3-Oxypyren-5,8,10-sulfosäure | 59040 | grün |
| 1-Hydroxy-4-N-phenyl-aminoanthrachinon | 60724 | violett |
| 1-Hydroxy-4-(4'-methylphenylamino)- anthrachinon | 60725 | violett |
| Acid Violet 23 | 60730 | violett |
| 1,4-Di(4'-methyl-phenylamino)- anthrachinon | 61565 | grün |
| 1,4-Bis-(o-sulfo-p- toluidino)-anthrachinon | 61570 | grün |
| Acid Blue 80 | 61585 | blau |
| Acid Blue 62 | 62045 | blau |
| N,N'-Dihydro-1,2,1',2'-anthrachinonazin | 69800 | blau |
| Vat Blue 6; Pigment Blue 64 | 69825 | blau |
| Vat Orange 7 | 71105 | orange |
| Indigo | 73000 | blau |
| Indigo-disulfonsäure | 73015 | blau |
| 4,4'-Dimethyl-6,6'- dichlorthioindigo | 73360 | rot |
| 5,5'-Dichlor-7,7'- dimethylthioindigo | 73385 | violett |
| Quinacridone Violet 19 | 73900 | violett |
| Pigment Red 122 | 73915 | rot |
| Pigment Blue 16 | 74100 | blau |
| Phthalocyanine | 74160 | blau |
| Direct Blue 86 | 74180 | blau |
| Chlorierte Phthalocyanine | 74260 | grün |
| Natural Yellow 6,19; Natural Red 1 | 75100 | gelb |
| Bixin, Nor- Bixin | 75120 | orange |
| Lycopin | 75125 | gelb |
| trans-alpha-, beta- bzw. gamma-Carotin | 75130 | orange |
| Keto- und/oder Hydroxylderivate des Carotins | 75135 | gelb |
| Guanin oder Perlglanzmittel | 75170 | weiß |
| 1,7-Bis-(4-hydroxy- 3-methoxyphenyl)1,6-heptadien-3,5-dion | 75300 | gelb |
| komplexsalz (Na, Al, Ca) der Karminsäure | 75470 | rot |
| Chlorophyll a und b; Kupferverbindungen der Chlorophylle und Chlorophylline | 75810 | grün |
| Aluminium | 77000 | weiß |
| Tonerdehydrat | 77002 | weiß |
| Wasserhaltige Aluminiumsilikate | 77004 | weiß |
| Ultramarin | 77007 | blau |
| Pigment Red 101 und 102 | 77015 | rot |
| Bariumsulfat | 77120 | weiß |
| Bismutoxychlorid und seine Gemische mit Glimmer | 77163 | weiß |
| Calciumcarbonat | 77220 | weiß |
| Calciumsulfat | 77231 | weiß |
| Kohlenstoff | 77266 | schwar |
| Pigment Black 9 | 77267 | schwar |
| Carbo medicinalis vegetabilis | 77268:1 | schwarz |
| Chromoxid | 77288 | grün |
| Chromoxid, wasserhaltig | 77289 | grün |
| Pigment Blue 28, Pigment Green 14 | 77346 | grün |
| Pigment Metal 2 | 77400 | braun |
| Gold | 77480 | braun |
| Eisenoxide und -hydoxide | 77489 | orange |
| Eisenoxid | 77491 | rot |
| Eisenoxidhydrat | 77492 | gelb |
| Eisenoxid | 77499 | schwarz |
| Mischungen aus Eisen(II)- und Eisen(III)-hexacyanoferrat | 77510 | blau |
| Pigment White 18 | 77713 | weiß |
| Mangananimoniumdiphosphat | 77742 | violett |
| Manganphosphat; Mn₃(PO₄)₂· 7 H20 | 77745 | rot |
| Silber | 77820 | weiß |
| Titandioxid und seine Gemische mit Glimmer | 77891 | weiß |
| Zinkoxid | 77947 | weiß |
| 6,7-Dimethyl-9-(1'-D-ribityl)-isoalloxazin, Lactoflavin | | gelb |
| Zuckerkulör | | braun |
| Capsanthin, Capsorubin | | orange |
| Betanin | | rot |
| Benzopyryliumsalze, Anthocyane | | rot |
| Aluminium- , Zink-, Magnesium- und Calciumstearat | | weiß |
| Bromthymolblau | | blau |
| Bromkresolgrün | | grün |
| Acid Red 195 | | rot |

Es kann ferner erfindungsgemäß bevorzugt sein, als Farbstoff eine oder mehrere Substanzen aus der folgenden Gruppe zu wählen: 2,4- Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4- sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2- naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo) -2-naphthol-6-sulfosäure, Aluminiumsalz der 1-(4-Sulfo-1-naphthylazo)-2- naphthol-3,6-disulfosäure, 1-(4-Sulfo-1-naphthylazo)-2-naphthol-6,8- disulfosäure, Aluminiumsalz der 4-(4-Sulfo-1-phenylazo)-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7- Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigodisulfosäure, rotes und schwarzes Eisenoxid (CIN: 77 491 (rot) und 77 499 (schwarz)), Eisenoxidhydrat (CIN: 77 492), Manganammoniumdiphosphat und Titandioxid.
Weitere erfindungsgemäß bevorzugte Farbstoffpigmente sind öllösliche Naturfarbstoffe, wie z. B. Paprikaextrakte, β-Carotin oder Cochenille.
Erfindungsgemäß bevorzugte Perlglanzpigmente sind ausgewählt aus natürlichen Perlglanzpigmenten, wie z. B. "Fischsilber" (Guanin/Hypoxanthin-Mischkristalle aus Fischschuppen) und "Perlmutt" (vermahlene Muschelschalen), monokristallinen Perlglanzpigmenten, wie z. B. Bismutoxychlorid (BiOCI), und Schicht-Substrat Pigmenten, z. B. Glimmer / Metalloxid. Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere bevorzugt ist z. B. das unter der CIN 77163 aufgelistete Glanzpigment.
Erfindungsgemäß bevorzugt sind ferner die folgenden Perlglanzpigmentarten auf Basis von Metalloxid-beschichtetem Glimmer:

| **Gruppe** | **Beschichtung /Schichtdicke** | **Farbe** |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 - 100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂/ Fe₂O₃ | Goldtöne |
| | TiO₂/ Cr₂O₃ | grün |
| | TiO₂/ Berliner Blau | tiefblau |
| | TiO₂/ Carmin | rot |

Erfindungsgemäß besonders bevorzugt sind z.B. die von der Firma Merck unter den Handelsnamen Timiron, Colorona oder Dichrona erhältlichen Perlglanzpigmente.
Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich außer Glimmer auch andere Substrate mit weiteren Metalloxiden beschichten, wie z. B. Silica und dergleichen mehr. Vorteilhaft sind z. B. mit TiO₂ und Fe₂O₃ beschichtete SiO₂-Partikel ("Ronasphere"), die von der Firma Merck vertrieben werden und sich besonders für die optische Reduktion feiner Fältchen eignen.
Es kann darüber hinaus erfindungsgemäß bevorzugt sein, gänzlich auf ein Substrat wie Glimmer zu verzichten. Besonders bevorzugt sind Perlglanzpigmente, die unter der Verwendung von SiO₂ hergestellt werden. Solche Pigmente, die auch zusätzlich goniochromatische Effekte haben können, sind z. B. unter dem Handelsnamen Sicopearl Fantastico bei der Firma BASF erhältlich.
Weiterhin bevorzugt sind Pigmente der Firma Engelhard / Mearl auf Basis von Calcium Natrium Borosilikat, die mit Titandioxid beschichtet sind. Diese sind unter dem Namen Reflecks erhältlich. Sie weisen durch ihrer Partikelgröße von 40 - 180 µm zusätzlich zu der Farbe einen Glitzereffekt auf.
Besonders vorteilhaft sind ferner auch Effektpigmente, die unter der Handelsbezeichnung Metasomes Standard / Glitter in verschiedenen Farben (yellow, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (CI) Nummern 19140, 77007, 77289, 77491) vor.
Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.
Weitere bevorzugte Pigmente sind ausgewählt aus farbigen und farblosen Pigmenten. Einige der im folgenden genannten Pigmente dienen auch als UV-Absorber. Besonders bevorzugte farbige Pigmente sind ausgewählt aus den Eisenoxiden mit den Color Index-Nummern Cl 77491 (Eisenoxid rot), CI 77492 (Eisenoxidhydrat gelb) und CI 77499 (Eisenoxid schwarz), aus CI 77891 (Titandioxid) und Ruß.

Besonders bevorzugte Pigmente sind die Handelsprodukte SUNPMMA-S und SUNSIL Tin 30 der Firma Sunjin Chemicals Co. mit einem mittleren Teilchendurchmesser von 5 - 10 µm bzw. 2 - 7 µm.
Besonders bevorzugte anorganische Pigmente sind beschichtet. Die Beschichtung kann mit Hilfe von anorganischen und/oder organischen Verbindungen erfolgen. Erfindungsgemäß besonders bevorzugt sind anorganische Pigmente, die eine anorganische Beschichtung aufweisen. Außerordentlich bevorzugte Pigmente dieser Art sind ausgewählt aus Siliciumdioxid-Partikeln, die mit Titandioxid und/oder Eisenoxiden beschichtet sind. Ein besonders bevorzugtes Pigment dieser Art ist das Handelsprodukt Ronasphere^{®} LDP der Firma Merck KGaA. Bei diesem Produkt handelt es sich um sphärische Siliciumdioxid-Partikel, die mit Titandioxid und Eisenoxid beschichtet sind. Ronasphere^{®} LDP weist einen mittleren Teilchendurchmesser von 4 - 7 µm auf.
Weiterhin bevorzugt sind anorganisch beschichtete Glimmerpigmente, die keinen Perlglanz aufweisen.
Weitere bevorzugte anorganisch beschichtete anorganische Pigmente sind Glimmerpigmente, die mit Titandioxid in verschiedenen Schichtdicken beschichtet sind, beispielsweise die Produkte der Timiron^{®}-Serie von Rona/Merck KGaA, insbesondere Pigmente der Produktreihen Timiron^{®} MP, Timiron^{®} Super, Timiron^{®} Starlight und Timiron^{®} Silk. Die genannten Produkte weisen mittlere Teilchendurchmesser von 5 - 60 µm bzw. 10 - 60 µm bzw. 10 - 125 µm bzw. 5 - 25 µm auf. Ebenfalls bevorzugt sind Glimmerpartikel mit einer Beschichtung aus Titandioxid und Eisenoxid, z. B. die Handelsprodukte Timiron^{®} MP-20, MP-24, MP-25, MP-28, MP-29, MP-60 und MP-65. Weiterhin erfindungsgemäß bevorzugt sind mit Titandioxid und/oder rotem und/oder schwarzem Eisenoxid beschichtete Glimmerpartikel, z. B. die Produkte der Colorona^{®}-Serie. Weitere bevorzugte Pigmente sind mit Kieselgel beschichtete Glimmerpigmente, z. B. das Handelsprodukt Micronasphere^{®} M. Weitere erfindungsgemäß bevorzugte Pigmente sind anorganisch beschichtete anorganische Pigmente, deren Beschichtung einen Anteil von 0,1 - 1 Gew.-% Zinnoxid aufweist.
Ebenfalls erfindungsgemäß bevorzugt sind anorganische Pigmente, die mit organischen Substanzen beschichtet sind. Bevorzugte Beispiele hierfür sind mit Aluminiumstearat beschichtete Titandioxid-Pigmente (z. B. das Handelsprodukt MT 100 T von der Firma Tayca), mit Dimethylpolysiloxan (Dimethicone) beschichtetes Zinkoxid, mit Dimethicone beschichtetes Bornitrid (Très BN^{®} UHP 1106 von Carborundum), mit einem Gemisch aus Dimethylpolysiloxan und Silicagel (Simethicone) und Aluminiumoxidhydrat (Alumina) beschichtetes Titandioxid (Eusolex^{®} T 2000 von Merck), mit Octylsilanol beschichtetes Titandioxid oder sphärische Polyalkylsesquisiloxan-Partikel (Aerosil^{®} R972 von Degussa).
Ein weiteres besonders bevorzugtes Pigment ist das Handelsprodukt SB-705 der Firma Miyoshi Kasei, ein sphärisches Kieselgel mit der INCI-Bezeichnung Silica, das einen mittleren Teilchendurchmesser von 5 - 6 µm und eine Oberfläche von etwa 600 m²/g aufweist.

Weitere erfindungsgemäß besonders bevorzugte Stiftzusammensetzungen sind dadurch gekennzeichnet, dass sie mindestens ein färbendes, farbgebendes, mattierendes oder glanzgebendes Pigment in einer Gesamtmenge von 0,1 Gew.-% bis 30 Gew.-%, bevorzugt 0,5 bis 15 Gew.-%, besonders bevorzugt 1,0 bis 10 Gew.-% und außerordentlich bevorzugt 2 - 5 Gew.-%, enthalten, jeweils bezogen auf das Gesamtgewicht der erfindungsgemäßen Zusammensetzung.

Weitere erfindungsgemäß bevorzugte Stiftzusammensetzungen sind dadurch gekennzeichnet, dass (zusätzlich zu dem mindestens einen kosmetischen Wirkstoff g)) maximal 5 Gew.-%, bevorzugt maximal 4 Gew.-%, besonders bevorzugt maximal 3 Gew.-%, außerordentlich bevorzugt maximal 2 Gew.-% und weiterhin außerordentlich bevorzugt maximal 1 Gew.-% und weiterhin außerordentlich bevorzugt maximal 0,5 Gew.-% mindestens eines Aluminium- und/oder Zirconium- und/oder Zink-Salzes enthalten ist. Derartig geringe Konzentrationen eines an sich als Antitranspirant-Wirkstoff verwendeten Salzes haben in der erfindungsgemäßen O/W-Emulsionsbasis eine zu geringe schweißhemmende Wirkung, so dass sie sich nicht für ein kommerziell wirksames und erfolgreiches Antitranspirant-Produkt eignen. Allerdings hat sich für den Fachmann überraschend herausgestellt, dass die Gegenwart eines Aluminium- und/oder Zirconium- und/ oder Zink-Salzes in Mengen von maximal 5 Gew.-%, bevorzugt maximal 4 Gew.-%, besonders bevorzugt maximal 3 Gew.-%, außerordentlich bevorzugt maximal 2 Gew.-% und weiterhin außerordentlich bevorzugt maximal 1 Gew.-% und weiterhin außerordentlich bevorzugt maximal 0,5 Gew.-% die Stabilität und/oder die Festigkeit und/oder die Abriebeigenschaften der erfindungsgemäßen Stiftzusammensetzungen weiter optimieren kann. Besonders bevorzugte stabilisierende und/oder festigende Aluminium- und/oder Zirconium- und/oder Zink-Salze sind ausgewählt aus den Aluminiumchlorhydraten, insbesondere den Aluminiumchlorhydraten mit der allgemeinen Formel [Al₂(OH)₅Cl · 2-3 H₂O]ₙ, die in nicht-aktivierter oder in aktivierter (depolymerisierter) Form vorliegen können, weiterhin Aluminiumsesquichlorhydrat, Aluminiumchlorhydrex-Propylenglykol (PG) oder -Polyethylenglykol (PEG), Aluminiumsesquichlorhydrex-PG oder -PEG, Aluminium-PG-dichlorhydrex oder Aluminium-PEGdichlorhydrex, Aluminiumhydroxid, weiterhin ausgewählt aus den Aluminiumzirconiumchlorhydraten, wie Aluminiumzirconiumtrichlorhydrat, Aluminiumzirconiumtetrachlorhydrat, Aluminiumzirconiumpentachlorhydrat, Aluminiumzirconiumoctachlorhydrat, den Aluminium-Zirkonium-Chlorohydrat-Glycin-Komplexen wie Aluminiumzirconiumtrichlorhydrexglycin, Aluminiumzirconiumtetrachlorhydrexglycin, Aluminiumzirconiumpentachlorhydrexglycin, Aluminiumzirconiumoctachlorhydrexglycin, Kaliumaluminiumsulfat (KAl(SO₄)₂ · 12 H₂O, Alaun), Aluminiumundecylenoylkollagenaminosäure, Natriumaluminiumlactat + Aluminiumsulfat, Natriumaluminiumchlorhydroxylactat, Aluminiumbromhydrat, Aluminiumchlorid, den Komplexen von Zink- und Natriumsalzen, den Komplexen von Lanthan und Cer, den Aluminiumsalzen von Lipoaminosäuren, Aluminiumsulfat, Aluminiumlactat, Aluminiumchlorhydroxyallantoinat, Natrium-Aluminium-Chlorhydroxylactat, Zinkchlorid, Zinksulfocarbolat, Zinksulfat und Zirkoniumchlorohydrat.

Weitere erfindungsgemäß besonders bevorzugte Stiftzusammensetzungen sind dadurch gekennzeichnet, dass maximal 5 Gew.-%, bevorzugt maximal 4 Gew.-%, besonders bevorzugt maximal 3 Gew.-%, außerordentlich bevorzugt maximal 2 Gew.-% und weiterhin außerordentlich bevorzugt maximal 1 Gew.-% und weiterhin außerordentlich bevorzugt maximal 0,5 Gew.-% mindestens eines Aluminium- und/oder Zirconium- und/oder Zink-Salzes sowie als weitere Komponente mindestens ein anorganischer und/oder organischer polymerer Hydrogelbildner enthalten sind.

Weitere erfindungsgemäß besonders bevorzugte Stiftzusammensetzungen sind dadurch gekennzeichnet, dass kein Deodorant-Wirkstoff, ausgewählt aus Arylsulfatase-Inhibitoren, β-Glucuronidase-Inhibitoren, Aminoacylase-Inhibitoren, Esterase-Inhibitoren, Lipase-Inhibitoren und Lipoxigenase-Inhibitoren, α-Monoalkylglycerinethern mit einem verzweigten oder linearen gesättigten oder ungesättigten, gegebenenfalls hydroxylierten C₆ - C₂₂-Alkylrest, insbesondere α-(2-Ethylhexyl)-glycerinether, Phenoxyethanol, keimhemmend wirkenden Parfümölen, Deosafe-Parfümölen, präbiotisch wirksamen Komponenten, Trialkylcitronensäureestern, insbesondere Triethylcitrat, Wirkstoffen, die die Zahl der Hautkeime aus der Gruppe der Staphylokokken, Corynebakterien, Anaerokokken und Mikrokokken reduzieren bzw. deren Wachstum hemmen, Zinkverbindungen, insbesondere Zinkphenolsulfonat und Zinkricinoleat, Organohalogenverbindungen, insbesondere Triclosan, Chlorhexidin, Chlorhexidingluconat und Benzalkoniumhalogeniden, quartären Ammoniumverbindungen, insbesondere Cetylpyridiniumchlorid, Geruchsabsorbern, insbesondere Silikaten und Zeolithen, Natriumbicarbonat und Lantibiotika, enthalten ist.

Ein weiterer Gegenstand der vorliegenden Erfindung ist ein kosmetisches, nicht-therapeutisches Verfahren zur Verbesserung des Erscheinungsbildes der Haut, wobei eine Stiftzusammensetzung nach einem der Ansprüche 1 - 31 auf die Haut, insbesondere auf die Gesichtshaut, aufgetragen wird.

### Niedrigschmelzende Lipid- oder Wachskomponente

Besonders bevorzugte erfindungsgemäße kosmetische Stifte sind dadurch gekennzeichnet, dass mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist. Diese niedriger schmelzenden Lipid- oder Wachskomponenten ermöglichen eine Konsistenzoptimierung des Produktes und eine Minimierung der sichtbaren Rückstände auf der Haut. Besonders bevorzugt sind Handelsprodukte mit der INCI-Bezeichnung Cocoglycerides, insbesondere die Handelsprodukte Novata^{®} (ex Cognis), besonders bevorzugt Novata^{®} AB, ein Gemisch aus C₁₂-C₁₈-Mono-, Di- und Triglyceriden, das im Bereich von 30 - 32°C schmilzt, sowie die Produkte der Softisan-Reihe (Sasol Germany GmbH) mit der INCI-Bezeichnung Hydrogenated Cocoglycerides, insbesondere Softisan 100, 133, 134, 138, 142. Weitere bevorzugte Ester von gesättigten, einwertigen C₁₂-C₁₈-Alkoholen mit gesättigten C₁₂-C₁₈-Monocarbonsäuren sind Stearyllaurat, Cetearylstearat (z. B. Crodamol^{®} CSS), Cetylpalmitat (z. B. Cutina^{®} CP) und Myristylmyristat (z. B. Cetiol® MM).

Weitere besonders bevorzugte erfindungsgemäße kosmetische Stifte sind dadurch gekennzeichnet, dass die mindestens eine Lipid- oder Wachskomponente mit einem Schmelzpunkt im Bereich von 25 - < 50°C in Mengen von 0,01 bis 20 Gew.-%, bevorzugt 3 - 20 Gew.-%, besonders bevorzugt 5 - 18 Gew.-% und außerordentlich bevorzugt 6 - 15 Gew.-%, bezogen auf die Gesamtzusammensetzung, enthalten ist.

### Füllstoffe

Besonders bevorzugte erfindungsgemäße kosmetische Stifte sind dadurch gekennzeichnet, dass sie zur Verbesserung der Stiftkonsistenz und der sensorischen Eigenschaften weiterhin mindestens einen festen, wasserunlöslichen teilchenförmigen Füllstoff enthalten. In einer außerordentlich bevorzugten Ausführungsform ist dieser Füllstoff ausgewählt aus gegebenenfalls modifizierten Stärken (z. B. aus Mais, Reis, Kartoffeln) und Stärkederivaten, die gewünschtenfalls vorverkleistert sind, Cellulose, insbesondere mikrokristalliner Cellulose, und Cellulosederivaten, Siliciumdioxid, Kieselsäuren, z. B. Aerosil^{®}-Typen, sphärischen Polyalkylsesquisiloxan-Partikeln (insbesondere Aerosil^{®} R972 und Aerosil^{®} 200V von Degussa), Kieselgelen, Talkum, Kaolin, Tonen, z. B. Bentoniten, Magnesiumaluminiumsilikaten, Bornitrid, Lactoglobulinderivaten, z. B. Natrium-C₈₋₁₆-Isoalkylsuccinyllactoglobulinsulfonat, von Brooks Industries erhältlich als Handelsprodukt Biopol^{®} OE, Glaspulvern, Polymerpulvern, insbesondere aus Polyolefinen, Polycarbonaten, Polyurethanen, Polyamiden, z. B. Nylon, Polyestern, Polystyrolen, Polyacrylaten, (Meth)acrylat- oder (Meth)acrylat-Vinyliden-Copolymeren, die vernetzt sein können, oder Siliconen, sowie Mischungen dieser Substanzen.
Polymerpulver auf Basis eines Polymethacrylat-Copolymers sind z. B. als Handelsprodukt Polytrap^{®} 6603 (Dow Corning) erhältlich. Andere Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere Polymerpulver, die sich für den erfindungsgemäßen Zweck eignen, sind z. B. Polymethacrylate (Micropearl^{®} M von SEPPIC oder Plastic Powder A von NIKKOL), Styrol-Divinylbenzol-Copolymeren (Plastic Powder FP von NIKKOL), Polyethylen- und Polypropylen-Pulver (ACCUREL^{®} EP 400 von AKZO) oder auch Siliconpolymere (Silicone Powder X2-1605 von Dow Corning).
Besonders bevorzugte erfindungsgemäße kosmetische Stifte sind dadurch gekennzeichnet, dass sie mindestens einen festen, wasserunlöslichen teilchenförmigen Füllstoff in einer Gesamtmenge von 0,01 bis 30 Gew.-%, bevorzugt 5 - 20 Gew.-%, besonders bevorzugt 8 - 15 Gew.-%, jeweils bezogen auf die Gesamtzusammensetzung, enthalten.

### Duftstoffe

Besonders bevorzugte erfindungsgemäße kosmetische Stifte sind dadurch gekennzeichnet, dass weiterhin mindestens eine Duftstoffkomponente enthalten ist.
Als Duftstoffkomponente können Parfüme, Parfümöle oder Parfümölbestandteile eingesetzt werden. Parfümöle bzw. Duftstoffe können erfindungsgemäß einzelne Riechstoffverbindungen, z. B. die synthetischen Produkte vom Typ der Ester, Ether, Aldehyde, Ketone, Alkohole und Kohlenwasserstoffe sein. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmecyclat. Zu den Ethern zählen beispielsweise Benzylethylether und Ambroxan , zu den Aldehyden z.B. die linearen Alkanale mit 8 - 18 C-Atomen, Citral, Citronellal, Citronellyloxy-acetaldehyd, Cyclamenaldehyd, Lilial und Bourgeonal, zu den Ketonen z.B. die Jonone, alpha-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen.

Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller-Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliöl, Orangenschalenöl und Sandelholzöl.
Um wahrnehmbar zu sein, muss ein Riechstoff flüchtig sein, wobei neben der Natur der funktionellen Gruppen und der Struktur der chemischen Verbindung auch die Molmasse eine wichtige Rolle spielt. So besitzen die meisten Riechstoffe Molmassen bis etwa 200 Dalton, während Molmassen von 300 Dalton und darüber eher eine Ausnahme darstellen. Aufgrund der unterschiedlichen Flüchtigkeit von Riechstoffen verändert sich der Geruch eines aus mehreren Riechstoffen zusammengesetzten Parfüms bzw. Duftstoffs während des Verdampfens, wobei man die Geruchseindrücke in "Kopfnote" (top note), "Herz- bzw. Mittelnote" (middle note bzw. body) sowie "Basisnote" (end note bzw. dry out) unterteilt. Da die Geruchswahrnehmung zu einem großen Teil auch auf der Geruchsintensität beruht, besteht die Kopfnote eines Parfüms bzw. Duftstoffs nicht allein aus leichtflüchtigen Verbindungen, während die Basisnote zum größten Teil aus weniger flüchtigen, d.h. haftfesten Riechstoffen besteht. Bei der Komposition von Parfüms können leichter flüchtige Riechstoffe beispielsweise an bestimmte Fixative gebunden werden, wodurch ihr zu schnelles Verdampfen verhindert wird. Bei der nachfolgenden Einteilung der Riechstoffe in "leichter flüchtige" bzw. "haftfeste" Riechstoffe ist also über den Geruchseindruck und darüber, ob der entsprechende Riechstoff als Kopf- oder Herznote wahrgenommen wird, nichts ausgesagt.
Haftfeste Riechstoffe, die im Rahmen der vorliegenden Erfindung einsetzbar sind, sind beispielsweise die ätherischen Öle wie Angelikawurzelöl, Anisöl, Arnikablütenöl, Basilikumöl, Bayöl, Bergamottöl, Champacablütenöl, Edeltannenöl, Edeltannenzapfenöl, Elemiöl, Eukalyptusöl, Fenchelöl, Fichtennandelöl, Galbanumöl, Geraniumöl, Gingergrasöl, Guajakholzöl, Gurjunbalsamöl, Helichrysumöl, Ho-Öl, Ingweröl, Irisöl, Kajeputöl, Kalmusöl, Kamillenöl, Kampferöl, Kanagaöl, Kardamomenöl, Kassiaöl, Kiefernnadelöl, Kopaïvabalsamöl, Korianderöl, Krauseminzeöl, Kümmelöl, Kuminöl, Lavendelöl, Lemongrasöl, Limetteöl, Mandarinenöl, Melissenöl, Moschuskörneröl, Myrrhenöl, Nelkenöl, Neroliöl, Niaouliöl, Olibanumöl, Orangenöl, Origanumöl, Palmarosaöl, Patschuliöl, Perubalsamöl, Petitgrainöl, Pfefferöl, Pfefferminzöl, Pimentöl, Pine-Öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-Öl, Ysop-Öl, Zimtöl, Zimtblätteröl, Zitronellöl, Zitronenöl sowie Zypressenöl.
Aber auch die höhersiedenden bzw. festen Riechstoffe natürlichen oder synthetischen Ursprungs können im Rahmen der vorliegenden Erfindung als haftfeste Riechstoffe bzw. Riechstoffgemische, also Duftstoffe, eingesetzt werden. Zu diesen Verbindungen zählen die nachfolgend genannten Verbindungen sowie Mischungen aus diesen: Ambrettolid, α-Amylzimtaldehyd, Anethol, Anisaldehyd, Anisalkohol, Anisol, Anthranilsäuremethylester, Acetophenon, Benzylaceton, Benzaldehyd, Benzoesäureethylester, Benzophenon, Benzylakohol, Benzylacetat, Benzylbenzoat, Benzylformiat, Benzylvalerianat, Borneol, Bornylacetat, α-Bromstyrol, n-Decylaldehyd, n-Dodecylaldehyd, Eugenol, Eugenolmethylether, Eukalyptol, Farnesol, Fenchon, Fenchylacetat, Geranylacetat, Geranylformiat, Heliotropin, Heptincarbonsäuremethylester, Heptaldehyd, Hydrochinon-Dimethylether, Hydroxyzimtaldehyd, Hydroxyzimtalkohol, Indol, Iron, Isoeugenol, Isoeugenolmethylether, Isosafrol, Jasmon, Kampfer, Karvakrol, Karvon, p-Kresolmethylether, Cumarin, p-Methoxyacetophenon, Methyl-n-amylketon, Methylanthranilsäuremethylester, p-Methylacetophenon, Methylchavikol, p-Methylchinolin, Methyl-β-naphthylketon, Methyl-n-nonylacetaldehyd, Methyl-n-nonylketon, Muskon, β-Naphtholethylether, β-Naphtholmethylether, Nerol, Nitrobenzol, n-Nonylaldehyd, Nonylakohol, n-Octylaldehyd, p-Oxy-Acetophenon, Pentadekanolid, β-Phenylethylakohol, Phenylacetaldehyd-Dimethyacetal, Phenylessigsäure, Pulegon, Safrol, Salicylsäureisoamylester, Salicylsäuremethylester, Salicylsäurehexylester, Salicylsäurecyclohexylester, Santalol, Skatol, Terpineol, Thymen, Thymol, γ-Undelacton, Vanilin, Veratrumaldehyd, Zimtaldehyd, Zimatalkohol, Zimtsäure, Zimtsäureethylester, Zimtsäurebenzylester.
Zu den leichter flüchtigen Riechstoffen zählen insbesondere die niedriger siedenden Riechstoffe natürlichen oder synthetischen Usprung, die allein oder in Mischungen eingesetzt werrden können. Beispiele für leichter flüchtige Riechstoffe sind Alkylisothiocyanate (Alkylsenföle), Butandion, Limonen, Linalool, Linaylacetat und -propionat, Menthol, Menthon, Methyl-n-heptenon, Phellandren, Phenylacetaldehyd, Terpinylacetat, Zitral, Zitronellal.
Besonders bevorzugte erfindungsgemäße kosmetische Stifte sind dadurch gekennzeichnet, dass mindestens eine Duftstoffkomponente in einer Gesamtmenge von 0,00001 bis 4 Gew.-%, bevorzugt 0,5 - 2 Gew.-%, jeweils bezogen auf die Gesamtzusammensetzung, enthalten ist.

### Penetrationskraftwerte

In einer weiteren besonders bevorzugten Ausführungsform sind die erfindungsgemäßen Stiftzusammensetzungen gekennzeichnet durch einen Penetrationskraftwert im Bereich von 150 - 800 Gramm-Kraft (g-force), bevorzugt 200 - 750 Gramm-Kraft (g-force), besonders bevorzugt 350 - 600 Gramm-Kraft (g-force), bei einer Penetrationstiefe von 5,000 mm. Der Penetrationskraftwert stellt ein Maß für die Härte eines Stiftes (oder aber auch einer festen Cremezusammensetzung) dar und gibt an, mit welcher Maximalkraft eine definierte Messsonde, hier ein Edelstahlkonus mit 45° (Modell TA 15), bis zu einer für die Messung geeigneten Penetrationstiefe, hier bis zu einer Penetrationstiefe von 5,000 mm (fünf komma null null null mm), mit einer Vorschubgeschwindigkeit von 2 mm/Sekunde senkrecht (axial) in die zu vermessende Stiftmasse eingefahren wird. Die Bestimmung des Penetrationskraftwertes wird durchgeführt mit dem TA-XT2i Texture Analyzer der Firma Stable Micro Systems (Vienna Court, Lammas Road, Godalming, Surrey GU7 1YL, England). Die Maximalkraft wird in Gramm-Kraft (g-force) angegeben. Dabei bedeuten niedrigere Werte eine weichere Zusammensetzung, härtere Zusammensetzungen weisen einen höheren Penetrationskraftwert auf. Cremeartige Zusammensetzungen werden häufig mit einer Penetrationstiefe von 10,000 mm (zehn komma null null null mm) vermessen, um genauere Werte zu erhalten. Diese Eindringtiefe kann bei härteren Stiftmassen meist nicht vermessen werden, da hierbei oft schon die Stiftmasse zu brechen beginnt. Eine Verdoppelung der Penetrationstiefe bedeutet etwa eine Verdreifachung bis Vervierfachung des Messwertes der Maximalkraft. Die Messungen erfolgen bei Umgebungsbedingungen von 30°C und 50% relativer Luftfeuchte, die Probentemperatur beträgt 23°C. Die Messungen erfolgen bevorzugt 3 Tage und/oder 4 Wochen nach Herstellung der erfindungsgemäßen Stiftzusammensetzung.
Die in DE 199 62 878 A1 und DE 199 62 881 A1 offenbarten Antitranspirant-Cremes weisen unter den hier genannten Messbedingungen Penetrationskraftwerte von 9 - 15 Gramm-Kraft (g-force) auf.

### Elektrischer Widerstand

Die im Stand der Technik offenbarten wasserhaltigen Stifte liegen nahezu ausschließlich in Form von Wasser-in-Öl-Emulsionen oder Emulsionen mit der wässrigen Phase als dispergierter Phase vor. Um die erfindungsgemäßen Stifte klar und eindeutig vom Stand der Technik abzugrenzen, dient als schnell und verlässlich durchzuführender Test, wie allgemein bei der Untersuchungen von Emulsionen üblich, die Messung des elektrischen Widerstandes. Ein ÖI-in-Wasser-System weist wegen der kontinuierlichen Wasserphase eine höhere elektrische Leitfähigkeit und dementsprechend einen niedrigeren elektrischen Widerstand auf als ein Wasser-in-Öl-System. In einer weiteren besonders bevorzugten Ausführungsform sind die erfindungsgemäßen Stiftzusammensetzungen gekennzeichnet durch einen elektrischen Widerstand von maximal 300 kΩ. Bevorzugt ist ein elektrischer Widerstand von maximal 100 kΩ, besonders bevorzugt von maximal 80 kΩ. Der Widerstand wird mit einem Multimeter Voltcraft Modell VC820 mit automatischer Messbereichsumschaltung (0-400 Ω / 40MΩ (± 1 % + 2dgt)) und zwei Mikrotipp Messfühlern 1,0 mm aus Edelstahl gemessen. Der Elektrodenabstand wird mit einer Millimeterlehre fixiert. Die Messung wird bei Raumtemperatur (22°C) durchgeführt. Dazu werden die Mikrotipp-Elektroden im Abstand von 27,0 mm parallel auf der Millimeterlehre fixiert und mit dem Widerstandsmessgerät verbunden. Die Messung des elektrischen Widerstandes erfolgt direkt an den wasserhaltigen Stiften. Dazu wird die üblicherweise gewölbte Oberfläche der Stifte mit einem Messer soweit abgetragen, dass sich eine ebene Schnittfläche ergibt. Unmittelbar danach werden die Messelektroden ca. 5 mm senkrecht in die Stiftmasse eingesteckt. Der Messwert des elektrischen Widerstandes wird nach 30 Sekunden abgelesen. Die Reinigung der Messelektroden erfolgt mit einem alkoholgetränkten Zellstofftuch. Unter den genannten Messbedingungen weist Leitungswasser einen elektrischen Widerstand von 250 kΩ, eine 20 Gew.-%ige wässrige Aluminiumchlorohydrat-Lösung 3 kΩ und vollentsalztes Wasser 1,7 MΩ auf.

### Produktstabilisierung

Besonders bevorzugte erfindungsgemäße kosmetische Stifte sind dadurch gekennzeichnet, dass sie zur Produktstabilisierung mindestens eine Radikalfängersubstanz enthalten, besonders bevorzugt eine Substanz mit der INCI-Bezeichnung Tris(tetramethylhydroxypiperidinol)citrate, die z. B. unter dem Handelsnamen Tinogard Q von der Firma Ciba erhältlich ist. Tris(tetramethylhydroxypiperidinol)citrat ist bevorzugt in Mengen von 0,01 - 0,1, besonders bevorzugt 0,025 - 0,05 Gew.-%, bezogen auf das Gesamtgewicht der erfindungsgemäßen Zusammensetzung, enthalten.

Weitere besonders bevorzugte erfindungsgemäße kosmetische Stifte sind dadurch gekennzeichnet, dass sie mindestens einen UV-Filter enthalten. Dabei sind die UV-Filter bevorzugt ausgewählt aus Benzotriazolderivaten, insbesondere 2-(5-Chloro-2H-benzotriazol-2-yl)-6-(1,1-dimethylethyl)-4-methyl-phenol (INCI-Bezeichnung: Bumetrizole, erhältlich z. B. unter dem Handelsnamen Tinogard AS von der Firma Ciba), 2,2'-Methylen-bis-(6-(2H-benzotriazol-2-yl)-4-(1,1,3,3-tetramethylbutyl)-phenol) [Tinosorb M (Ciba)], 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amylphenyl)benzotriazol (CAS- Nr.: 025973-551), 2-(2'- Hydroxy-5'-octylphenyl)-benzotriazol (CAS-Nr. 003147-75-9), 2-(2'-Hydroxy-5'-methylphenyl)benzotriazol (CAS-Nr. 2440-22-4), 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane, 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazin oder auch Aniso Triazin, erhältlich als Tinosorb^{®} S von CIBA), 2,4-Bis-{[4-(3-sulfonato)-2-hydroxy-propyloxy)-2-hydroxy]-phenyl}-6-(4-methoxyphenyl)-1,3,5-triazin-Natriumsalz, 2,4-Bis-{[4-(3-(2-propyloxy)-2-hydroxy-propyloxy)-2-hydroxy]-phenyl}-6-(4-methoxyphenyl)-1,3,5-triazin, 2,4-Bis-{[4-(2-ethyl-hexyloxy)-2-hydroxy]-phenyl}-6-[4-(2-methoxyethylcarboxyl)-phenylamino]-1,3,5-triazin, 2,4-Bis-{[4-(3-(2-propyloxy)-2-hydroxy-propyloxy)-2-hydroxy]-phenyl}-6-[4-(ethylcarboxyl)-phenylamino]-1,3,5-triazin, 2,4-Bis-{[4-(2-ethyl-hexyloxy)-2-hydroxy]-phenyl}-6-(1-methyl-pyrrol-2-yl)-1,3,5-triazin, 2,4-Bis- {[4-tris(trimethylsiloxysilylpropyloxy)-2-hydroxy]-phenyl}-6-(4-methoxyphenyl)-1,3,5-triazin, 2,4-Bis-{[4-(2-methylpropenyloxy)-2-hydroxy]-phenyl}-6-(4-methoxyphenyl)-1,3,5-triazin, 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin sowie Mischungen der genannten Komponenten. Weiterhin ist der Zusatz wasserlöslicher UV-Filter bevorzugt. Bevorzugte wasserlösliche UV-Filter sind 2-Phenylbenzimidazol-5-sulfonsäure, Phenylen-1,4-bis-(2-benzimidazyl)-3,3'-5,5'-tetrasulfonsäure und deren Alkali-, Erdalkali-, Ammonium-, Alkylammonium-, Alkanolammonium- und Glucammoniumsalze, insbesondere die Sulfonsäure selbst mit der INCI Bezeichnung Phenylbenzimidazole Sulfonic Acid (CAS.-Nr. 27503-81-7), die beispielsweise unter dem Handelsnamen Eusolex 232 bei Merck oder unter Neo Heliopan Hydro bei Symrise erhältlich ist, und das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonate (CAS-Nr.: 180898-37-7), das beispielsweise unter dem Handelsnamen Neo Heliopan AP bei Symrise erhältlich ist, Sulfonsäurederivate von Benzophenonen, vorzugsweise 2-Hydroxy-4-methoxybenzophenon-5-sulfonsäure und ihre Salze und Sulfonsäurederivate des 3-Benzylidencamphers, wie z. B. 4-(2-Oxo-3-bornylidenmethyl)benzolsulfonsäure.

Weitere besonders bevorzugte erfindungsgemäße kosmetische Stifte sind dadurch gekennzeichnet, dass sie zur Produktstabilisierung den Radikalfänger Tris(tetramethylhydroxypiperidinol)citrat und den UV-Filter Bumetrizole enthalten. Bumetrizole ist bevorzugt in Mengen von 0,01 - 0,1, besonders bevorzugt 0,025 - 0,05 Gew.-%, bezogen auf das Gesamtgewicht der erfindungsgemäßen Zusammensetzung, enthalten.

Weitere besonders bevorzugte erfindungsgemäße kosmetische Stifte sind dadurch gekennzeichnet, dass sie zur Produktstabilisierung mindestens eine komplexbildende Substanz enthalten. Als komplexbildende Substanz besonders bevorzugt ist Ethylendiamintetraessigsäure (EDTA) und ihre Natriumsalze, wie sie beispielsweise unter dem Handelsnamen Trilon B von der Firma BASF erhältlich ist, weiterhin Nitrilotriessigsäure (NTA) und ihre Natriumsalze, β-Alanindiessigsäure und ihre Salze und Phosphonsäuren und deren Salze. Die mindestens eine komplexbildende Substanz ist bevorzugt in einer Gesamtmenge von 0,01 - 0,5 Gew.-%, besonders bevorzugt 0,08 - 0,2 Gew.-%, bezogen auf das Gesamtgewicht der erfindungsgemäßen Zusammensetzung, enthalten.

Weitere außerordentlich bevorzugte erfindungsgemäße kosmetische Stifte sind dadurch gekennzeichnet, dass sie mindestens eine Radikalfängersubstanz und mindestens eine Substanz, ausgewählt aus UV-Filtern und komplexbildenden Substanzen, enthalten.
Weitere außerordentlich bevorzugte erfindungsgemäße kosmetische Stifte sind dadurch gekennzeichnet, dass sie mindestens eine Radikalfängersubstanz, mindestens einen UV-Filter und mindestens eine komplexbildende Substanz enthalten.
Weitere außerordentlich bevorzugte erfindungsgemäße kosmetische Stifte sind dadurch gekennzeichnet, dass sie Tris(tetramethylhydroxypiperidinol)citrate, Bumetrizole und Ethylendiamintetraessigsäure, letztere gegebenenfalls als Natriumsalz, enthalten.

Weitere besonders bevorzugte erfindungsgemäße kosmetische Stifte sind dadurch gekennzeichnet, dass sie mindestens eine den Haarwuchs inhibierende Substanz enthalten. Bevorzugte Substanzen, die den Haarwuchs inhibieren, sind insbesondere ausgewählt aus Eflornithin, Wirkstoffkombinationen aus Sojaproteinhydrolysat, Harnstoff, Menthol, Salicylsäure und Extrakten aus Hypericum Perforatum, Hamamelis Virginiana, Arnica Montana und der Rinde von Salix Alba, wie sie beispielsweise und bevorzugt in dem Rohstoff Pilinhib^{®} Veg LS 9109 von Laboratoires Sérobiologiques mit der INCI-Deklaration "Propylene glycol, Hydrolyzed Soy Protein, Hypericum Perforatum Extract, Hamamelis Virginiana Extract, Arnica Montana Flower Extract, Urea, Salix Alba Bark Extract, Menthol, Salicylic acid" enthalten ist, weiterhin Wirkstoffkombinationen aus Extrakten aus Epilobium Angustifolium, den Samen von Cucurbita pepo (pumpkin, Zucchini) und den Früchten von Serenoa serrulata, wie sie beispielsweise und bevorzugt in den Rohstoffen ARP 100 von Greentech S.A./Rahn mit der INCI-Deklaration "Water, Alcohol, Serenoa Serrulata Fruit Extract, Epilobium Angustifolium Extract, Cucurbita Pepo (Pumpkin) Seed Extract" und ARP 100 Huileux (ex Greentech, INCI: Caprylic/Capric Triglyceride, Serenoa Serrulata Fruit Extract, Epilobium Angustifolium Flower/Leaf/Stem Extract, Cucurbita Pepo (Pumpkin) Seed Extract), enthalten sind, weiterhin Wirkstoffkombinationen aus Xylitol und Extrakten von Citrus Medica Limonum (Lemon) Fruit, Carica Papaya (Papaya) und Olivenblättern, wie sie beispielsweise und bevorzugt in dem Rohstoff Xyleine von Impag / Seporga mit der INCI-Deklaration "Xylitol and Citrus Medica Limonum (Lemon) Fruit Extract and Carica Papaya (Papaya) Fruit Extract and Olea europaea (olive) leaf extract" enthalten sind, weiterhin Wirkstoffkombinationen aus Humulus Lupulus, Viscum Album, Salvia Officinalis, Carica Papaya und Thuya Occidentalis, wie sie beispielsweise und bevorzugt in dem Rohstoff Plantafluid Komplex AH der Firma Plantapharm mit der INCI-Deklaration "Aqua, Propylene Glycol, Humulus Lupulus, Viscum Album, Salvia Officinalis, Carica Papaya, Thuya Occidentalis" enthalten sind, sowie Extrakten aus Larrea divaricata, wie sie beispielsweise und bevorzugt in dem Rohstoff Capislow von Sederma, der Lecithinvesikel mit einem hydroglycolischen Extrakt aus Larrea divaricata enthält, enthalten sind.
Weitere bevorzugte Haarwuchs-inhibierende Wirkstoffe sind ausgewählt aus Substanzen, die die Proteinkinase C hemmen, insbesondere aus dem racemischen Gemisch von (±)-5-[*N*-(3,4-Di-methoxyphenethyl)-*N*-methylamino]-2-(3,4-dimethoxyphenyl)-2-isopropylvaleronitril (internationaler Freiname: Verapamil), dem racemischen Gemisch von (±)-10-[2-(1-Methyl-2-piperidyl)ethyl]-2-(methylthio)-phenothiazin (internationaler Freiname: Thioridazin), Curcumin, Trifluoperizin, 1-(5-isoquinolinylsulfonyl)2-methylpiperazin, L-Ascorbylpalmitat, Glycyrrhetinsäureglycosiden, 18β-Glycyrrhetinsäure, 5-(3-Dimethylaminopropyl)-10,11-dihydro-5*H*-dibenz[*b*,*f*]azepin (internationaler Freiname: Imipramin), 3-[*N*-(2-Imidazolin-2-ylmethyl)-*p-*toluidino]phenol (internationaler Freiname: Phentolamin) Isochinolinsulfonamid, Bisindolylmaleimid, Lysosphingolipide, Staurosporin, Sangivamycin, Doxorubicin-Fe (III), Balanol (Azepinostatin), 2-(Aminomethyl)piperidin, 4-Propyl-5(4-pyridinyl)-2-(3H)-oxazolon und 1,10-Decamethylenbis(4-amino-2-methylchinolinium)-dichlorid (Dequaliniumchlorid).
Weitere bevorzugte Haarwuchs-inhibierende Wirkstoffe sind ausgewählt aus Substanzen, die die Protein-Tyrosinkinase hemmen, insbesondere aus Lavendustin-A, Erbstatin, Tyrphostin, Piceatannol, 4-Hydroxybenxylidenmalononitril, 3,5-Di-*tert*-butyl-4- hydroxybenzylidenmalononitril, α-Cyano-(3,4-dihydroxy)-cinnamonitril, α-Cyano-(3,4,5-trihydroxy)cinnamonitril, α-Cyano-(3,4-dihydroxy)cinnamid, α-Cyano-(3,4-dihydroxy)thiocinnamid, 2- Amino-4-(4'-hydroxyphenyl)-1,1,3-tricyanobuta-1,3-dien, 2-Amino-4-(3,4,5'- trihydroxyphenyl)-1,1,3-tricyanobuta-1,3-dien, 2-Amino-4-(1 H-alpha-indol-5-yl)-1,1,3-tricyanobuta-1,3-dien, 4-Hydroxy-3-methoxy-5-(benzothiazolylthiomethyl)benzylidencyanoacetamid, 4-Amino-N-(2,5-dihydroxybenzyl)methylbenzoat, α-Cyano-(3,4-dihydroxy)-cinnamonitril, 4-(3-Chloranilino)-6,7-dimethoxychinazolin, α-Cyano-(3,4-dihydroxy)-N-benzylcinnamid, (-)-R-N-(a-Methylbenzyl)-3,4-dihydroxybenzylidencyanoacetamid, α-Cyano-(3,4-dihydroxy)-N-(3-phenylpropyl)-cinnamid, α-Cyano-(3,4-dihydroxy)-N-phenylcinnamid, α-Cyano-(+)-(S)-N-(alpha-phenethyl)-(3,4-dihydroxy)cinnamid, α-Cyano-(3,4-dihydroxy)-N-(phenylbutyl)cinnamid, Herbimycin A, Thiazolidindion, Phenazocin, 2,3-Dihydro-2-thioxo-1 H-indol-3-Alkansäuren, 2,2'-Dithiobis-(1 H-indol-3-Alkansäuren), Sulfonylbenzoylnitrostyrol, Methylcaffeat, HNMPA(AM)₃(hydroxy-2-naphthalenylmethylphosphonsäure-tris-acetoxymethylester) und N-Acetyl-Asp-Tyr-(2-malonyl)-Val-Pro-Met-Leu-NH₂.
Weitere bevorzugte Haarwuchs-inhibierende Wirkstoffe sind ausgewählt aus den Substanzen, die in WO 2006/130330 A2 offenbart sind, nämlich Agonisten des Farnesoid X-Rezeptors, bevorzugt ausgewählt aus Gallensäuren, wie insbesondere Lithocholsäure, Cholsäure, Deoxycholsäure, Chenodeoxycholsäure, Ursodeoxycholsäure und 6-alpha-Ethylchenodeoxycholsäure, weiterhin aus Farnesoiden, insbesondere Farnesol (3,7,11-Trimethyl-2,6,10-dodecatrien-1-ol), Farnesal, Farnesylacetat, 3,7,11-Trimethyl-2,6,10-dodecatrien-1-carbonsäure, Methylfarnesylether, Methylfarnesoat, Ethylfarnesylether, Ethylfarnesoat, weiterhin aus 7-Methyl-9-(3,3-dimethyloxivanyl)-3-methyl-2,6-nonadiensäuremethylester (Juvenilhormon III), 7-Methyl-9-(3,3-dimethyloxivanyl)-3-methyl-2,6-nonadiensäureethylester, 3-alpha,7-alpha-Dihydroxy-6-alpha-ethyl-5p-cholan-24-säure, 7-alpha-Dihydroxy-6-alpha-propyl-5p-cholan-24-säure, 7-alpha-Dihydroxy-6-alpha-allyl-5p-cholan-24-säure, N-(2,2,2-trifluoroethyl)-N-[4-[2,2,2-trifluoro-1-hydroxy-1-(trifluoromethyl)-ethyl]-phenyl]-benzolsulfonanilid, 3-[2-[2-Chloro-4-[3-(2,6-dichlorophenyl)-5-(1-methylethyl)-4-isoxazolyl]methoxy]-phenylethenyl]-benzoesäure, [3,5-bis(1,1-dimethylethyl)-4-hydroxyphenyl]ethenyliden]bisphosphonsäuretetraethylester, [2-[3,5-bis(1,1-dimethylethyl)-4-hydroxyphenyl]ethyliden]-bisphosphonsäuretetrakis(1-methylethyl)ester, [2-[3,5-bis(1,1-dimethylethyl)-4-hydroxyphenyl]ethyliden]bisphosphonsäuretetraethylester und [3,5-bis(1,1-dimethylethyl-4-hydroxyphenyl]ethenyliden]bisphosphonsäuretetrakis(1-methylethyl)ester.
Bevorzugte erfindungsgemäße Zusammensetzungen enthalten mindestens eine den Haarwuchs inhibierende Substanz in einer Gesamtmenge von 0,0001 - 5 Gew.-%, bevorzugt 0,001 - 2 Gew.-%, besonders bevorzugt 0,01 - 1 Gew.-%, und außerordentlich bevorzugt 0,1 - 0,5 Gew.-%, jeweils bezogen auf das Gewicht an Aktivsubstanz des/der Haarwuchs inhibierenden Wirkstoffs/e und das Gesamtgewicht der erfindungsgemäßen Stiftzusammensetzung.

### Konservierungsstoffe

Den erfindungsgemäßen Zusammensetzungen können auch die üblichen Konservierungsstoffe zugesetzt werden, um den Verderb des Produktes durch mikrobielles Wachstum zu verhindern. Zahlreiche Konservierungsstoffe haben zwangsläufig auch desodorierende Eigenschaften, so dass einige Stoffe beiden Gruppen angehören. Für Kosmetika als Konservierungsstoffe bevorzugt geeignet sind beispielsweise Benzoesäure und deren Derivate (z. B. Propyl-, Phenyl- und Butylbenzoat, Ammonium-, Natrium, Kalium- und Magnesiumbenzoat), Propionsäure und deren Derivate (z. B. Ammonium-, Natrium-, Kalium-, und Magnesiumpropionat), Salicylsäure und deren Derivate (z. B. Natrium-, Kalium- und Magnesiumsalicylat), 4-Hydroxybenzoesäure und deren Ester und Alkalimetallsalze (z. B. Methyl-, Ethyl-, Propyl-, Isopropyl-, Butyl-, Isobutyl-, Isodecyl-, Phenyl-, Phenoxyethyl- und Benzylparaben, Hexamidinparaben und -diparaben, Natrium- und Kaliumparaben, Natrium- und Kaliummethylparaben, Kaliumbutylparaben, Natrium- und Kaliumpropylparaben), Alkohole und deren Salze (z.B. Ethanol, Propanol, Isopropanol, Benzylalkohol, Phenethylalkohol, Phenol, Kaliumphenolat, Phenoxyethanol, Phenoxyisopropanol, o-Phenylphenol), Guajacol und dessen Derivate, Chlorhexidin und dessen Derivate (z. B. Chlorhexidindiacetat, -digluconat, und -dihydrochlorid), Hydantoin und dessen Derivate (z. B. DEDM- und DMDM-Hydantoin, DEDM-Hydantoindilaurat), Harnstoff und Harnstoffderivate (z. B. Diazolidinylharnstoff, Imidazolidinylharnstoff), Ferulasäure und deren Derivate (z. B. Ethylferulat), Sorbinsäure und deren Derivate (z. B. Isopropylsorbat, TEA-Sorbat, Natrium-, Kalium-, und Magnesiumsorbat), Isothiazol- und Oxazolderivate (z. B. Methylisothiazolinon, Methylchloroisothiazolinon, Dimethyloxazolidin), quartäre Ammoniumverbindungen (z. B. Polyquaternium-42, Quaternium-8, Quaternium-14, Quaternium-15), Carbamate (z. B. lodopropynylbutylcarbamat), Formaldehyd und Natriumformat, Glutaraldehyd, Glyoxal, Hexamidin, Dehydracetsäure, 2-Bromo-2-nitropropan-1,3-diol, Isopropylkresol, Methyldibromoglutaronitril, Polyaminopropylbiguanid, Natriumhydroxymethylglycinat, Natriumphenolsulfonat, Triclocarban, Triclosan, Zinkpyrithion sowie diverse Peptid-Antibiotika (z. B. Nisin).

Erfindungsgemäß bevorzugte Konservierungsmittel sind Phenoxyethanol, die Ester der 4-Hydroxybenzoesäure, insbesondere Methyl-, Ethyl-, Propyl-, Isopropyl-, Butyl- und Isobutylparaben sowie lodopropynylbutylcarbamat.
Die Menge der Konservierungsmittel in den bevorzugten erfindungsgemäßen Zusammensetzungen beträgt 0,001 - 10 Gew.-%, vorzugsweise 0,01 - 5 Gew.-% und insbesondere 0,1 - 3 Gew.-%, bezogen auf das Gesamtgewicht der Zusammensetzung.

Ein weiterer Gegenstand der vorliegenden Erfindung ist ein Verfahren zur Herstellung eines kosmetischen Stiftes gemäß einem der Ansprüche 1 - 31, wobei die Wachs- und Ölkomponenten zusammen mit dem/den Öl-in-Wasser- und dem/den Wasser-in-Öl-Emulgator/en auf 90 - 95°C erhitzt und aufgeschmolzen werden, danach das ebenfalls auf 90 - 95°C erhitzte Wasser mit den wasserlöslichen Wirk- und Inhaltsstoffen unter kräftigem Rühren zugegeben wird, gegebenenfalls weitere Inhaltsstoffe beigemischt werden, die Mischung bis auf eine geeignete Abfülltemperatur abgekühlt, in geeignete Spenderformen abgefüllt und durch statisches Abkühlen (ohne weiteres Rühren) auf Raumtemperatur verfestigt wird.

Die nachfolgenden Beispiele sollen den Gegenstand der Erfindung verdeutlichen, ohne ihn hierauf zu beschränken.

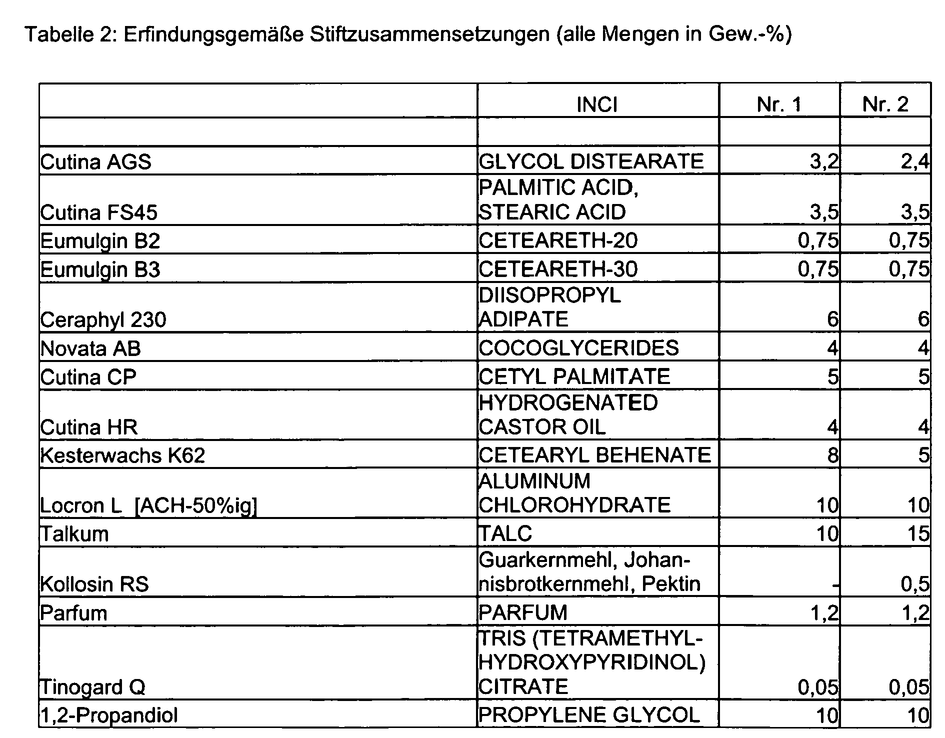

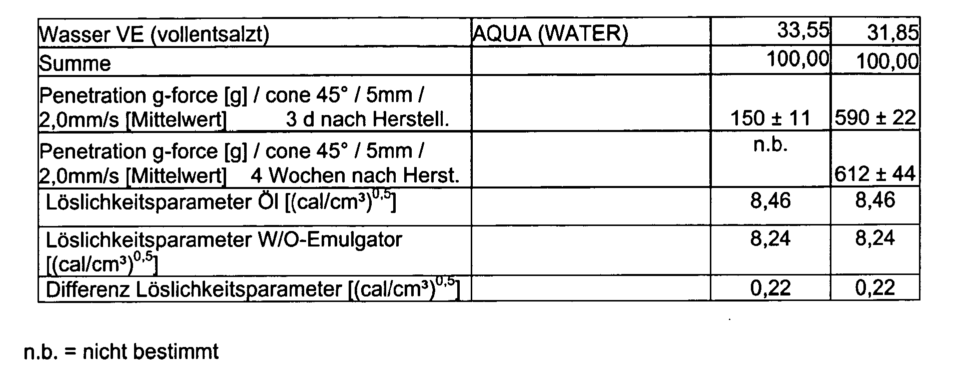

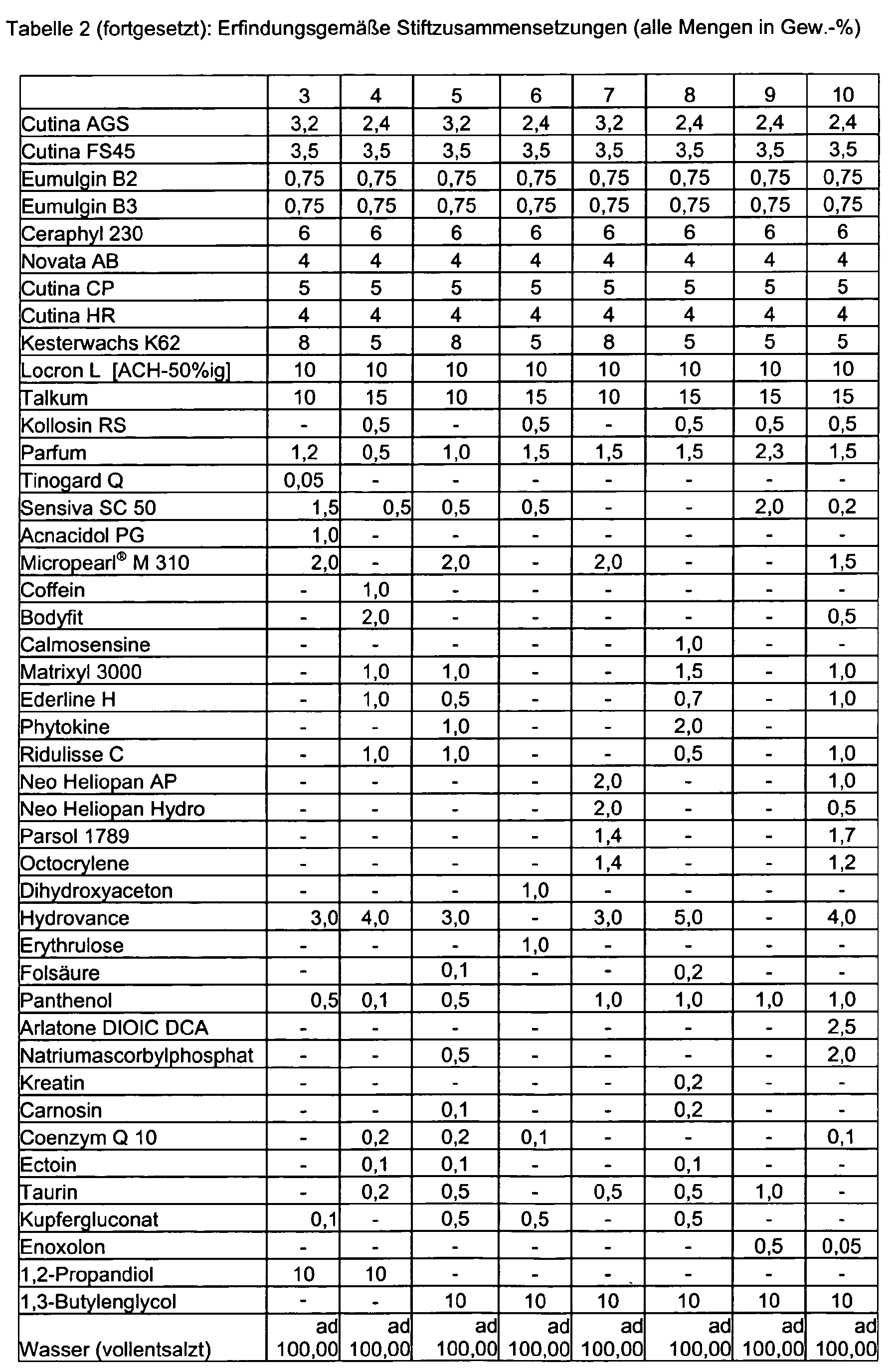

Die Beispiele 1 und 2 sind Beispiele für erfindungsgemäß bevorzugte Stift-Basen zur Behandlung und Pflege der Haut.
Beispiel 3 ist ein Beispiel für einen erfindungsgemäß bevorzugten kosmetischen Stift zur Behandlung von Pickeln, Akne und Mitessern.
Beispiel 4 ist ein Beispiel für einen erfindungsgemäß bevorzugten kosmetischen Stift zur Behandlung von Cellulite und zur Hautstraffung.
Beispiel 5 ist ein Beispiel für einen erfindungsgemäß bevorzugten kosmetischen Stift zur Behandlung von Falten, Fältchenund feinen Linien.
Beispiel 6 ist ein Beispiel für einen erfindungsgemäß bevorzugten kosmetischen Stift mit hautbräunender Wirkung (Selbstbräuner-Stift).
Beispiel 7 ist ein Beispiel für einen erfindungsgemäß bevorzugten kosmetischen Stift mit UV-Schutz und hautmattierender Wirkung.
Beispiel 8 ist ein Beispiel für einen erfindungsgemäß bevorzugten kosmetischen Stift mit feuchtigkeitsspendender Wirkung.
Beispiel 9 ist ein Beispiel für einen erfindungsgemäß bevorzugten Deodorant-Stift mit hautberuhigender Wirkung, speziell für die Haut nach der Rasur, und einem Haarwuchsinhibierenden Wirkstoff.
Beispiel 10 ist ein Beispiel für einen erfindungsgemäß bevorzugten kosmetischen Stift zur aufhellenden Behandlung der Haut.

### Anwendung der erfindungsgemäßen Stifte

Auf Haut mit Anzeichen der Akne, Pickeln und/oder Mitessern wurde morgens und abends nach der Reinigung der Stift aus Beispiel 3 aufgetragen (Gesicht und/oder Dekolleté und/oder Rücken). Je nach Bedarf wurden die betroffenen Hautpartien einmal oder auch mehrmals mit dem erfindungsgemäßen Stift bestrichen. Nach 7 Tagen zeigte sich eine deutliche Verbesserung des Hautbildes, Akne und Pickel traten nur noch in abgeschwächtem bzw. verringertem Maße auf.

Auf Haut mit Anzeichen der Cellulite wurde alle 2 Tage der Stift aus Beispiel 4 aufgetragen (Oberschenkel, Gesäß). Dazu wurden die betroffenen Hautpartien mehrmals mit dem erfindungsgemäßen Stift in kreisenden, massierenden Bewegungen bestrichen. Nach 28 Tagen zeigte sich eine deutliche Verbesserung des Hautbildes, die behandelte Haut wirkte straffer als vor der Behandlung.

Auf Haut mit Fältchen und feinen Linien wurde morgens und abends nach der Reinigung der Stift aus Beispiel 5 aufgetragen (Gesicht und/oder Hals). Je nach Bedarf wurden die betroffenen Hautpartien einmal oder auch mehrmals mit dem erfindungsgemäßen Stift, besonders bevorzugt in kreisenden, massierenden Bewegungen, bestrichen. Nach 7 Tagen zeigte sich eine deutliche Verbesserung des Hautbildes, Fältchen und feinen Linien waren nur noch in abgeschwächtem bzw. verringertem Maße sichtbar.
Die Beispiel-Stifte 6, 7, 8, 9 und 10 wurden in ähnlicher Weise angewendet.

**Liste der verwendeten Rohstoffe**

| Komponente | INCI | Lieferant/Hersteller |
|---|---|---|
| Acnacidol PG | Propylene Glycol, Aqua (Water), Sebacic acid, 10-hydroxydecanoic acid, 1,10-decanediol, PHENOXYETHANOL, METHYLPARABEN, ETHYLPARABEN,PROPYLPARABEN, BUTYLPARABEN | Vincience |
| Arlatone DIOIC DCA | OCTADECENEDIOIC ACID, BHT | Uniqema |
| Bodyfit | GLYCERIN, AQUA (WATER), COCOGLUCOSIDE, CAPRYLYL GLYCOL, ALCOHOL, GLAUCINE (0,1 - 1 Gew.-% Glaucin) | Sederma |
| Calmosensine | BUTYLENE GLYCOL, WATER, LAURETH-3, HYDROXYETHYLCELLULOSE, Acetyl Dipeptide-1 Cetylester | Sederma |
| Ceraphyl 230 | Diisopropyl Adipate | ISP |
| Cetiol^{®} B | Dibutyl Adipate | Cognis |
| Cetiol^{®} SB 45 | Butyrospermum Parkii (Shea Butter) | Cognis |
| Cutina^{®} AGS | Glycol Distearate | Cognis |
| Cutina^{®} CP | Cetyl Palmitate | Cognis |
| Cutina^{®} FS45 | Palmitic Acid, Stearic Acid | Cognis |
| Cutina^{®} HR | Hydrogenated Castor Oil | Cognis |
| Cutina^{®} MD | Glyceryl Stearate | Cognis |
| Ederline H | PEG-40 Hydrogenated Castor Oil, PPG-2-Ceteareth-9, Pyrus Malus (Apple) Fruit Extract | Seporga |
| Enoxolon | 18β-Glycyrrhetinsäure (98 %) | Polichimica |
| Eumulgin^{®} B1 | Ceteareth-12 | Cognis |
| Eumulgin^{®} B2 | Ceteareth-20 | Cognis |
| Eumulgin^{®} B3 | Ceteareth-30 | Cognis |
| Hydrovance | Wasser, Bis-N,N'-(2-Hydroxyethyl)harnstoff, Harnstoff, Ammoniumlactat | National Starch |
| Kesterwachs K 62 | Cetearyl behenate | Koster Keunen |
| Kesterwachs K80 H | C20-40 Alkyl Stearate | Koster Keunen |
| Kollosin RS | Guar-Gum, Locust Bean Gum, Pectin | Kramer und Söhne Kollosin GmbH |
| Locron L | Aluminum Chlorohydrate (50 % by weight), Water | Clariant |
| Matrixyl 3000 | GLYCERIN, AQUA (WATER), BUTYLENE GLYCOL, CARBOMER, POLYSORBATE-20, PALMITOYL OLIGOPEPTIDE, PALMITOYL TETRAPEPTIDE-3 | Sederma |
| Micropearl^{®} M 310 | METHYL METHACRYLATE CROSSPOLYMER | Seppic |
| Neo Heliopan AP | Disodium Phenyl Dibenzimidazole Tetrasulfonate | Symrise |
| Neo Heliopan Hydro | Phenylbenzimidazole sulfonic acid (98 %) | Symrise |
| Novata^{®} AB | Cocoglycerides | Cognis |
| Parsol 1789 | BUTYL METHOXYDIBENZOYLMETHANE | DSM |
| Phytokine | AQUA (WATER), HYDROLYZED SOY PROTEIN, METHYLPARABEN, ETHYLPARABEN, PROPYLPARABEN | Coletica |
| Ridulisse C | AQUA (WATER), HYDROLYZED SOY PROTEIN, BUTYLENE GLYCOL, METHYLPARABEN, ETHYLPARABEN, PROPYLPARABEN, BUTYLPARABEN | Silab |
| Sensiva^{®} SC 50 | 2-Ethylhexylglycerinether | Schülke & Mayr |
| Tinogard Q | Tris(tetramethylhydroxypiperidinol)citrate | Ciba |

## Patentansprüche

1. Kosmetische Stiftzusammensetzung in Form einer Öl-in-Wasser-Dispersion/Emulsion, enthaltend
a) mindestens eine Lipid- oder Wachskomponente mit einem Schmelzpunkt > 50°C, die nicht den Komponenten b) oder c) zuzurechnen ist,
b) mindestens einen nichtionischen Öl-in-Wasser-Emulgator mit einem HLB-Wert von mehr als 7 innerhalb eines Öl-in-Wasser-Emulgatorgemisches mit einem mittleren HLB-Wert im Bereich von 10 - 19,
c) mindestens einen nichtionischen Wasser-in-Öl-Emulgator mit einem HLB-Wert größer 1,0 und kleiner/gleich 7,0, der mit Wasser allein oder mit Wasser in Gegenwart eines hydrophilen Emulgators flüssigkristalline Strukturen ausbilden kann, als Konsistenzgeber und/oder Wasserbinder,
d) mindestens ein unter Normalbedingungen flüssiges Öl, das keine Duftstoffkomponente und kein etherisches Öl darstellt, wobei der (mittlere) Löslichkeitsparameter der Gesamtheit der enthaltenen Öle in Gegenwart von linearen gesättigten Fettalkoholen mit einer Kettenlänge von mindestens 8 Kohlenstoffatomen um maximal -0,7 (cal/cm³)^{0,5} bzw. maximal +0,7 (cal/cm³)^{0,5}, und in Gegenwart von Wasser-in-Öl-Emulgatoren, die von linearen gesättigten Fettalkoholen mit einer Kettenlänge von mindestens 8 Kohlenstoffatomen verschieden sind, in Abwesenheit von linearen gesättigten Fettalkoholen mit einer Kettenlänge von mindestens 8 Kohlenstoffatomen um maximal -0,4 (cal/cm³)^{0,5} bzw. maximal +0,7 (cal/cm³)^{0,5} vom (mittleren) Löslichkeitsparameter des Wasser-in-ÖI-Emulgators/der Wasser-in-ÖI-Emulgatoren abweicht,
e) mindestens ein wasserlösliches mehrwertiges C₂ - C₉-Alkanol mit 2 - 6 Hydroxylgruppen und/oder mindestens ein wasserlösliches Polyethylenglycol mit 3 - 20 Ethylenoxid-Einheiten,
f) 5 bis weniger als 50 Gew.-% Wasser, bezogen auf die Gesamtzusammensetzung,
g) mindestens einen kosmetischen Wirkstoff, ausgewählt aus
- Monomeren, Oligomeren und Polymeren von Aminosäuren, N-C₂-C₂₄-Acylaminosäuren, den Estern und/oder den physiologisch verträglichen Metallsalzen dieser Substanzen,
- DNA- oder RNA-Oligonucleotiden,
- feuchtigkeitsspendenden Wirkstoffen,
- Vitaminen, Provitaminen und Vitaminvorstufen der Gruppen A, B, C, E und H und den Estern der vorgenannten Substanzen,
- α-Hydroxycarbonsäuren, α-Ketocarbonsäuren, β-Hydroxycarbonsäuren und deren Ester-, Lacton- oder Salzform,
- Flavonoiden und Flavonoid-reichen Pflanzenextrakten,
- Isoflavonoiden und Isoflavonoid-reichen Pflanzenextrakten,
- Polyphenolen und Polyphenol-reichen Pflanzenextrakten,
- Ubichinon und Ubichinol sowie deren Derivaten,
- Silymarin,
- Purin und Purin-Derivaten, insbesondere den natürlich vorkommenden Xanthin-Derivaten, ausgewählt aus Coffein, Theophyllin, Theobromin und Aminophyllin,
- Aporphin-Alkaloiden,
- Ectoin,
- anorganischen und organischen UV-Filtersubstanzen,
- selbstbräunenden Wirkstoffen,
- hautaufhellenden Wirkstoffen,
- hautberuhigenden Wirkstoffen,
- sebumregulierenden Wirkstoffen,
- hyperämisierenden Wirkstoffen,
- antimikrobiellen Wirkstoffen,
- präbiotischen Wirkstoffen,
- färbenden, farbgebenden, mattierenden oder glanzgebenden Pigmenten,
h) einen Gesamtgehalt an nichtionischen und ionischen Emulgatoren und/oder Tensiden mit einem HLB-Wert über 8 von maximal 20 Gew.-%, bezogen auf die gesamte Stiftzusammensetzung.

2. Stiftzusammensetzung gemäß Anspruch 1, **dadurch gekennzeichnet, dass** mindestens ein anorganischer und/oder organischer polymerer Hydrogelbildner enthalten ist.

3. Stiftzusammensetzung gemäß Anspruch 1 oder 2, **dadurch gekennzeichnet, dass** mindestens ein anorganischer und/oder organischer polymerer Hydrogelbildner in einer Gesamtmenge von 0,1 - 3,0 Gew.-%, bevorzugt 0,3 - 2, 0 Gew.-%, besonders bevorzugt 0,5 - 1,5 Gew.-% und außerordentlich bevorzugt 0,7 - 1,0 Gew.-%, jeweils bezogen auf das Gewicht der gesamten erfindungsgemäßen Zusammensetzung, enthalten ist.

4. Stiftzusammensetzung gemäß einem der vorstehenden Ansprüche, **dadurch gekennzeichnet, dass** maximal 5 Gew.-%, bevorzugt maximal 4 Gew.-%, besonders bevorzugt maximal 3 Gew.-%, außerordentlich bevorzugt maximal 2 Gew.-% und weiterhin außerordentlich bevorzugt maximal 1 Gew.-% und weiterhin außerordentlich bevorzugt maximal 0,5 Gew.-% mindestens eines Aluminium- und/oder Zirconium- und/oder Zink-Salzes enthalten ist.

5. Stiftzusammensetzung gemäß einem der vorstehenden Ansprüche, **dadurch gekennzeichnet, dass** maximal 5 Gew.-%, bevorzugt maximal 4 Gew.-%, besonders bevorzugt maximal 3 Gew.-%, außerordentlich bevorzugt maximal 2 Gew.-% und weiterhin außerordentlich bevorzugt maximal 1 Gew.-% und weiterhin außerordentlich bevorzugt maximal 0,5 Gew.-% mindestens eines Aluminium- und/oder Zirconium- und/oder Zink-Salzes sowie weiterhin mindestens ein anorganischer und/oder organischer polymerer Hydrogelbildner enthalten sind.

6. Stiftzusammensetzung gemäß einem der vorstehenden Ansprüche, **dadurch gekennzeichnet, dass** die Lipid- oder Wachskomponente a) ausgewählt ist aus Estern aus einem gesättigten, einwertigen C₁₆-C₆₀-Alkanol und einer gesättigten C₈-C₃₆-Monocarbonsäure, insbesondere Cetylbehenat, Stearylbehenat und C₂₀-C₄₀-Alkylstearat, Glycerintriestern von gesättigten linearen C₁₂ - C₃₀-Carbonsäuren, die hydroxyliert sein können, Candelillawachs, Carnaubawachs, Bienenwachs, gesättigten linearen C₁₄ - C₃₆-Carbonsäuren sowie Mischungen der vorgenannten Substanzen.

7. Stiftzusammensetzung gemäß einem der vorstehenden Ansprüche, **dadurch gekennzeichnet, dass** die Lipid- oder Wachskomponente a) insgesamt in Mengen von 4-20 Gew.-%, bevorzugt 8-15 Gew.-%, bezogen auf die Gesamtzusammensetzung, enthalten ist.

8. Stiftzusammensetzung gemäß Anspruch 2, **dadurch gekennzeichnet, dass** der/die Ester aus einem gesättigten, einwertigen C₁₆-C₆₀-Alkanol und einer gesättigten C₈-C₃₆-Monocarbonsäure in Mengen von 2 - 10 Gew.-%, bevorzugt 2 - 6 Gew.-%, bezogen auf die Gesamtzusammensetzung, enthalten ist/sind.

9. Stiftzusammensetzung gemäß einem der vorstehenden Ansprüche, **dadurch gekennzeichnet, dass** der nichtionische Öl-in-Wasser-Emulgator b) mit einem HLB-Wert von mehr als 7 ausgewählt ist aus
- ethoxylierten C₈-C₂₄-Alkanolen mit durchschnittlich 10 - 100 Mol Ethylenoxid pro Mol,
- ethoxylierten C₈-C₂₄-Carbonsäuren mit durchschnittlich 10 - 100 Mol Ethylenoxid pro Mol,
- Silicon-Copolyolen mit Ethylenoxid-Einheiten oder mit Ethylenoxid- und Propylenoxid-Einheiten,
- Alkylmono- und -oligoglycosiden mit 8 bis 22 Kohlenstoffatomen im Alkylrest und deren ethoxylierten Analoga,
- ethoxylierten Sterinen,
- Partialestern von Polyglycerinen mit n = 2 bis 10 Glycerineinheiten und mit 1 - 4 gesättigten oder ungesättigten, linearen oder verzweigten, gegebenenfalls hydroxylierten C₈ - C₃₀-Fettsäureresten verestert, sofern sie einen HLB-Wert von mehr als 7 aufweisen,
- sowie Mischungen der vorgenannten Substanzen.

10. Stiftzusammensetzung gemäß einem der vorstehenden Ansprüche, **dadurch gekennzeichnet, dass** der Öl-in-Wasser-Emulgator b) in einer Gesamtmenge von 0,5-10 Gew.-%, bevorzugt 1-4 Gew.-%, bezogen auf die Gesamtzusammensetzung, enthalten ist.

11. Stiftzusammensetzung gemäß einem der vorstehenden Ansprüche, **dadurch gekennzeichnet, dass** der nichtionische Wasser-in-Öl-Emulgator c) ausgewählt ist aus
- linearen, gesättigten C₁₂ - C₃₀-Alkanolen,
- Ethylenglycolmono- und diestern von linearen, gesättigten und ungesättigten C₁₂-C₃₀-Carbonsäuren, die hydroxyliert sein können,
- Glycerinmono- und diestern von linearen, gesättigten und ungesättigten C₁₂- C₃₀-Carbonsäuren, die hydroxyliert sein können,
- Propylenglycolmono- und -diestern von linearen, gesättigten und ungesättig-ten C₁₂-C₃₀-Carbonsäuren, die hydroxyliert sein können,
- Sorbitanmono-, -di- und -triestern von linearen, gesättigten und ungesättigten C₁₂-C₃₀-Carbonsäuren, die hydroxyliert sein können,
- Pentaerythritylmono-, -di-, -tri- und -tetraestern von linearen, gesättigten und ungesättigten C₁₂ - C₃₀-Carbonsäuren, die hydroxyliert sein können,
- Methylglucosemono- und -diestern von linearen, gesättigten und ungesättigten C₁₂ - C₃₀-Carbonsäuren, die hydroxyliert sein können,
- Sterinen,
- Alkanolen und Carbonsäuren mit jeweils 8 - 24 C-Atomen, insbesondere mit 16 - 22 C-Atomen, in der Alkylgruppe und 1 - 4 Ethylenoxid-Einheiten pro Molekül, die einen HLB-Wert größer 1,0 und kleiner/gleich 7,0 aufweisen,
- Glycerinmonoethern gesättigter und/oder ungesättigter, verzweigter und/oder unverzweigter Alkohole einer Kettenlänge von 8 - 30, insbesondere 12 - 18 Kohlenstoffatomen,
- Partialestern von Polyglycerinen mit n = 2 bis 10 Glycerineinheiten und mit 1 bis 5 gesättigten oder ungesättigten, linearen oder verzweigten, gegebenenfalls hydroxylierten C₈ - C₃₀-Fettsäureresten verestert, sofern sie einen HLB-Wert von kleiner/gleich 7 aufweisen,
- sowie Mischungen der vorgenannten Substanzen.

12. Stiftzusammensetzung gemäß einem der vorstehenden Ansprüche, **dadurch gekennzeichnet, dass** der Wasser-in-Öl-Emulgator c) in einer Gesamtmenge von 0,1 - 15 Gew.-%, bevorzugt 0,5 - 8 Gew.-%, und besonders bevorzugt 1 - 4 Gew.-%, bezogen auf das Gesamtgewicht der Zusammensetzung, enthalten ist.

13. Stiftzusammensetzung gemäß einem der vorstehenden Ansprüche, **dadurch gekennzeichnet, dass** das unter Normalbedingungen flüssige Öl d) ausgewählt ist aus
- Benzoesäureestern von linearen oder verzweigten C₈₋₂₂-Alkanolen,
- verzweigten gesättigten oder ungesättigten Fettalkoholen mit 6 - 30 Kohlenstoffatomen,
- Triglyceriden von linearen oder verzweigten, gesättigten oder ungesättigten, gegebenenfalls hydroxylierten C₈-₃₀-Fettsäuren,
- Dicarbonsäureestern von linearen oder verzweigten C₂-C₁₀-Alkanolen,
- Estern von verzweigten gesättigten oder ungesättigten Fettalkoholen mit 2 - 30 Kohlenstoffatomen mit linearen oder verzweigten gesättigten oder ungesättigten Fettsäuren mit 2-30 Kohlenstoffatomen, die hydroxyliert sein können,
- Anlagerungsprodukten von 1 bis 5 Propylenoxid-Einheiten an ein- oder mehrwertige C₈₋₂₂-Alkanole,
- Anlagerungsprodukten von mindestens 6 Ethylenoxid- und/oder Propylenoxid-Einheiten an ein- oder mehrwertige C₃₋₂₂-Alkanole,
- C₈-C₂₂-Fettaikoholestern einwertiger oder mehrwertiger C₂-C₇-Hydroxycarbonsäuren,
- symmetrischen, unsymmetrischen oder cyclischen Estern der Kohlensäure mit Fettalkoholen,
- den Estern von Dimeren ungesättigter C₁₂-C₂₂-Fettsäuren (Dimerfettsäuren) mit einwertigen linearen, verzweigten oder cyclischen C₂-C₁₈-Alkanolen oder mit mehrwertigen linearen oder verzweigten C₂-C₆-Alkanolen,
- sowie Mischungen der vorgenannten Substanzen.

14. Stiftzusammensetzung gemäß einem der vorstehenden Ansprüche, **dadurch gekennzeichnet, dass** das unter Normalbedingungen flüssige Öl d) in einer Gesamtmenge von 3-20 Gew.-%, bevorzugt 5-14 Gew.-%, besonders bevorzugt 6-12 Gew.-%, jeweils bezogen auf das Gesamtgewicht der Zusammensetzung, enthalten ist.

15. Stiftzusammensetzung gemäß einem der vorstehenden Ansprüche, **dadurch gekennzeichnet, dass** die unter Normalbedingungen flüssigen Öle d) maximal 20 Gew.-% Öl/e, bezogen auf das Gesamtgewicht an unter Normalbedingungen flüssigen Ölen, enthalten, deren Löslichkeitsparameter um mehr als -0,4 bzw. -0,7 (cal/cm³)^{0,5} oder um mehr als +0,7 (cal/cm³)^{0,5} vom (mittleren) Löslichkeitsparameter des Wasser-in-ÖI-Emulgators/der Wasser-in-ÖI-Emulgatoren c) abweicht.

16. Stiftzusammensetzung gemäß einem der vorstehenden Ansprüche, **dadurch gekennzeichnet, dass** keine unter Normalbedingungen flüssigen Öle enthalten sind, deren Löslichkeitsparameter um mehr als ± 1,0 (cal/cm³)^{0,5} vom (mittleren) Löslichkeitsparameter des Wasser-in-Öl-Emulgators/der Wasser-in-Öl-Emulgatoren c) abweicht.

17. Stiftzusammensetzung gemäß einem der vorstehenden Ansprüche, **dadurch gekennzeichnet, dass** das wasserlösliche mehrwertige C₂ - C₉-Alkanol e) mit 2 - 6 Hydroxylgruppen ausgewählt ist aus 1,2-Propylenglycol, 2-Methyl-1,3-propandiol, Glycerin, Butylenglycolen wie 1,2-Butylenglycol, 1,3-Butylenglycol und 1,4-Butylenglycol, Pentylenglycolen wie 1,2-Pentandiol und 1,5-Pentandiol, Hexandiolen wie 1,2-Hexandiol und 1,6-Hexandiol, Hexantriolen wie 1,2,6-Hexantriol, 1,2-Octandiol, 1,8-Octandiol, Dipropylenglycol, Tripropylenglycol, Diglycerin, Triglycerin, Erythrit, Sorbit sowie Mischungen der vorgenannten Substanzen.

18. Stiftzusammensetzung gemäß einem der vorstehenden Ansprüche, **dadurch gekennzeichnet, dass** das wasserlösliche mehrwertige C₂- C₉-Alkanol mit 2- 6 Hydroxylgruppen und/oder das wasserlösliche Polyethylenglycol mit 3-20 Ethylenoxid-Einheiten insgesamt in Mengen von 3-25 Gew.-%, bevorzugt 8-18 Gew.-%, bezogen auf die Gesamtzusammensetzung, enthalten ist.

19. Stiftzusammensetzung gemäß einem der vorstehenden Ansprüche, **dadurch gekennzeichnet, dass** weiterhin mindestens eine Lipid- oder Wachskomponente mit einem Schmelzpunkt im Bereich von 25 - < 50°C, ausgewählt aus Kokosfettsäureglycerinmono-, -di- und -triestern, Butyrospermum Parkii (Shea Butter) und Estern von gesättigten, einwertigen C₈-C₁₈-Alkoholen mit gesättigten C₁₂-C₁₈-Monocarbonsäuren sowie Mischungen dieser Substanzen, enthalten ist.

20. Stiftzusammensetzung gemäß Anspruch 19, **dadurch gekennzeichnet, dass** die Lipid- oder Wachskomponente mit einem Schmelzpunkt im Bereich von 25 - < 50°C in Mengen von 0,01 - 20 Gew.-%, bevorzugt 3-20 Gew.-%, besonders bevorzugt 5-18 Gew.-% und außerordentlich bevorzugt 6-15 Gew.-%, bezogen auf die Gesamtzusammensetzung, enthalten ist.

21. Stiftzusammensetzung gemäß einem der vorstehenden Ansprüche, **dadurch gekennzeichnet, dass** weiterhin mindestens ein fester, wasserunlöslicher teilchenförmiger Füllstoff enthalten ist.

22. Stiftzusammensetzung gemäß Anspruch 21, **dadurch gekennzeichnet, dass** der feste, wasserunlösliche teilchenförmige Füllstoff ausgewählt ist aus gegebenenfalls modifizierten Stärken und Stärkederivaten, die gewünschtenfalls vorverkleistert sind, Cellulose und Cellulosederivaten, Siliciumdioxid, Kieselsäuren, sphärischen Polyalkylsesquisiloxan-Partikeln, Kieselgelen, Talkum, Kaolin, Tonen, z. B. Bentoniten, Magnesiumaluminiumsilikaten, Bornitrid, Lactoglobulinderivaten, Glaspulver, Polymerpulvern sowie Mischungen der vorgenannten Substanzen.

23. Stiftzusammensetzung gemäß einem der vorstehenden Ansprüche, **dadurch gekennzeichnet, dass** der feste, wasserunlösliche, teilchenförmige Füllstoff in einer Gesamtmenge von 0,01 bis 30 Gew.-%, bevorzugt 5 - 20 Gew.-%, besonders bevorzugt 8-15 Gew.-%, jeweils bezogen auf die Gesamtzusammensetzung, enthalten ist.

24. Stiftzusammensetzung gemäß einem der vorstehenden Ansprüche, **dadurch gekennzeichnet, dass** 10 bis 45 Gew.-% Wasser, bevorzugt 20 bis 40 Gew.-%, besonders bevorzugt 30 - 38 Gew.-%, jeweils bezogen auf die Gesamtzusammensetzung, enthalten sind.

25. Stiftzusammensetzung gemäß einem der vorstehenden Ansprüche, **dadurch gekennzeichnet, dass** weiterhin mindestens eine Duftstoffkomponente enthalten ist.

26. Stiftzusammensetzung gemäß einem der vorstehenden Ansprüche, **gekennzeichnet durch** einen Penetrationskraftwert im Bereich von 150 - 800 Gramm-Kraft (g-force), 200 - 750 Gramm-Kraft (g-force), besonders bevorzugt 350 - 600 Gramm-Kraft (g-force) bei einer Penetrationstiefe von 5,000 mm.

27. Stiftzusammensetzung gemäß einem der vorstehenden Ansprüche, **gekennzeichnet durch** einen elektrischen Widerstand von maximal 300 kΩ, bevorzugt maximal 100 kΩ und besonders bevorzugt maximal 80 kΩ.

28. Stiftzusammensetzung gemäß einem der vorstehenden Ansprüche, **dadurch gekennzeichnet, dass** mindestens eine Radikalfängersubstanz enthalten ist.

29. Stiftzusammensetzung gemäß einem der vorstehenden Ansprüche, **dadurch gekennzeichnet, dass** mindestens ein UV-Filter enthalten ist.

30. Stiftzusammensetzung gemäß einem der vorstehenden Ansprüche, **dadurch gekennzeichnet, dass** mindestens eine komplexbildende Substanz enthalten ist.

31. Stiftzusammensetzung gemäß einem der vorstehenden Ansprüche, **dadurch gekennzeichnet, dass** mindestens eine den Haarwuchs inhibierende Substanz enthalten ist.

32. Kosmetisches, nicht-therapeutisches Verfahren zur Verbesserung des Erscheinungsbildes der Haut, **dadurch gekennzeichnet, dass** eine Stiftzusammensetzung nach einem der Ansprüche 1 - 31 auf die Haut, insbesondere auf die Gesichtshaut, aufgetragen wird.

33. Verfahren zur Herstellung eines kosmetischen Stiftes gemäß einem der Ansprüche 1 - 31, wobei die Wachs- und Ölkomponenten zusammen mit dem/den Öl-in-Wasser- und dem/den Wasser-in-Öl-Emulgator/en auf 90 - 95°C erhitzt und aufgeschmolzen werden, danach das ebenfalls auf 90 - 95°C erhitzte Wasser mit den wasserlöslichen Wirk- und Inhaltsstoffen unter kräftigem Rühren zugegeben wird, gegebenenfalls weitere Inhaltsstoffe beigemischt werden, die Mischung bis auf eine geeignete Abfülltemperatur abgekühlt, in geeignete Spenderformen abgefüllt und durch statisches Abkühlen (ohne weiteres Rühren) auf Raumtemperatur verfestigt wird.
